# EUROPÄISCHE PATENTSCHRIFT

(11) **EP 1 560 810 B1**
(45) Veröffentlichungstag und Bekanntmachung des Hinweises auf die Patenterteilung: **01.08.2012**
(21) Anmeldenummer: 03779818.8
(22) Anmeldetag: 29.10.2003
(51) Int. Cl.: C07C 331/28, C07D 207/20, C07D 211/70, C07D 211/18, C07D 213/76, C07D 307/52, C07D 319/06, C07D 333/20, C07D 295/26, C07C 303/34, C07C 307/06

(54) **BIFUNKTIONELLE PHENYLISO(THIO)CYANATE; VERFAHREN UND ZWISCHENPRODUKTE ZU IHRER HERSTELLUNG**
BIFUNCTIONAL PHENYLISO(THIO)CYANATES; METHOD AND INTERMEDIATE PRODUCTS FOR THE PRODUCTION THEREOF
PHENYLISO(THIO)CYANATES BIFONCTIONNELS ET PRODUITS INTERMEDIAIRES PERMETTANT DE LES PRODUIRE

(30) Priorität: 30.10.2002 DE 10250614
(43) Veröffentlichungstag der Anmeldung: 10.08.2005
(73) Patentinhaber: BASF SE, 67056 Ludwigshafen (DE); Hamprecht, Gerhard, 69469 Weinheim (DE); Puhl, Michael, 68623 Lampertheim (DE); Reinhard, Robert, 67065 Ludwigshafen (DE); Seitz, Werner, 68723 Plankstadt (DE)
(72) Erfinder: HAMPRECHT, Gerhard, 69469 Weinheim (DE); PUHL, Michael, 68623 Lampertheim (DE); WOLF, Bernd, 67136 Fussgönheim (DE); GÖTZ, Norbert, 67547 Worms (DE); KEIL, Michael, 67251 Freinsheim (DE); REINHARD, Robert, 67065 Ludwigshafen (DE); SAGASSER, Ingo, 67125 Dannstadt-Schauernheim (DE); SEITZ, Werner, 68723 Plankstadt (DE)
(86) Internationale Anmeldenummer: PCT/EP2003/012013
(87) Internationale Veröffentlichungsnummer: WO 2004/039768

(56) Entgegenhaltungen:
- WO-A-01/83459
- DATABASE CA [Online] CHEMICAL ABSTRACTS SERVICE, COLUMBUS, OHIO, US; BODRIKOV, I. V. ET AL: "Adducts of nitriles with sulfur trioxide and their reactions" retrieved from STN Database accession no. 84:59407 CA XP002270931 & ZHURNAL ORGANICHESKOI KHIMII (1975), 11(10), 2217 ,
- DATABASE CROSSFIRE BEILSTEIN [Online] Beilstein Institut zur Förderung der Chemischen Wissenschaften, Frankfurt am Main, DE; retrieved from XFIRE Database accession no. brn 2309607 XP002270932 & J ORG CHEM USSR, Bd. 13, 1977, Seiten 390-394,

## Beschreibung

Die Erfindung betrifft ein Verfahren zur Herstellung bifunktioneller Phenyliso(thio)cyanate der allgemeinen Formel I mit einer Acylsulfonamid-Gruppe, 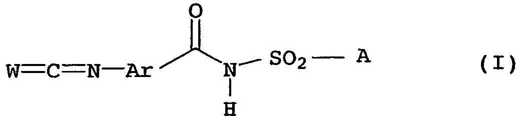 worin die Variablen folgende Bedeutung haben:
- W: Sauerstoff oder Schwefel,
- Ar: Phenyl, das durch folgende Gruppen ein- oder mehrfach substituiert sein kann: Wasserstoff, Halogen, C₁-C₄-Halogenalkyl, oder Cyano;
- A: ein von einem primären oder sekundären Amin abgeleiteter Rest oder NH₂,
durch Umsetzung von Anilinen oder deren Hydrochloriden mit Phosgenderivaten. Die Erfindung betrifft auch bifunktionelle Phenyl-iso(thio)cyanate.

Iso(thio)cyanatobenzoylsulfamidsäureamide sind potentielle Vorstufen für die Herstellung von Pflanzenschutzmitteln mit Triazol-3,5-dion-4-ylgruppe, Pyrimidin-2,6-dion-1-ylgruppe oder 1,3,5-Triazin-2,4,6-trion-1-ylgruppe oder deren S-Analoga wie sie z. B. in der WO 01/83459 beschrieben sind. Aufgrund ihrer Reaktionsfähigkeit sollte sich die Iso(thio)cyanato-Struktureinheit leicht in andere Gruppen wie (Thio)Harnstoff- oder Urethangruppen überführen lassen. Ihre Herstellung wurde jedoch aus im nachfolgenden genannten Gründen für nicht möglich erachtet.

Grundsätzlich kann man Phenyliso(thio)cyanate durch Umsetzung von primären aromatischen Aminen mit Phosgen beziehungsweise mit Thiophosgen herstellen (siehe z. B. Houben-Weyl, Methoden der organischen Chemie, 4. Auflage, Bd. IX, S. 869, 875-877 und Bd. VIII, S. 120-124). Weitere allgemeine Verfahren sind beispielsweise aus EP 70389, EP 75267 und EP 409 025 bekannt.

Allen beschriebenen Verfahren ist gemeinsam, dass die eingesetzten Phenyliso(thio)cyanate keine Acylsulfonamidgruppe tragen. Es ist nämlich bekannt, dass eine Iso(thio)cyanatogruppe mit einer Sulfonamid-Gruppe unter Bildung von Sulfonylharnstoffen reagiert. So beschreiben beispielsweise J. Cervello und T. Sastre in Synthesis 1990, 221-222, die Umsetzung eines Sulfonamides mit Isocyanaten gemäß der folgenden Reaktionsfolge:

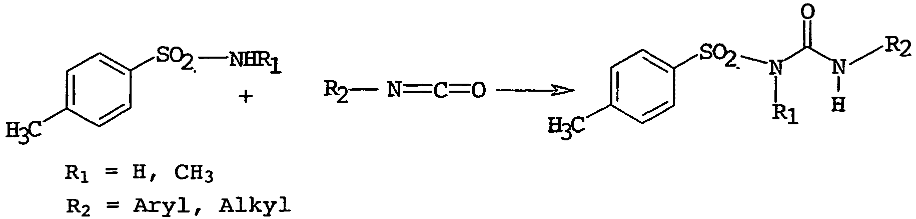

Aus der US 4,309,209 ist bekannt, dass Phenylisocyanate mit Chlormethan-(N-methyl)sulfonamid (= ClCH₂SO₂NHCH₃) unter Bildung eines 1,2,4-Thiadiazolidin-1,1,3-trions reagiert. P. Schwenkkraus und H.-H. Otto beschreiben in Arch. Pharm (Weinheim) 326, 437 - 441 (1993) die Umsetzung von 3-Halogenalkyl-β-sultamen mit Phenylisocyanat unter Bildung von Carbamoylverbindungen.

Aus der DE 3433391 ist die Umsetzung von Saccharin mit Acylisocyanaten zu N-acylierten Saccharin-Derivaten bekannt.

B. A. Arbuzov, N. N. Zobova und N. R. Fedotava beschreiben in JZV Akad Nauk SSSR, Ser Khim 1990, 2874 (engl. Übersetzung: Bulletin of the Academy of Sciences of the USSR, Division of Chemical Sciences, Bd. 39, (1990) S. 2610) die N- und O-Acylierung von Saccharin durch Umsetzung mit einem Trifluoracetylisocyanat.

Vor diesem Hintergrund wurde daher sowohl die Herstellung von Phenyliso(thio)cyanaten, die im gleichen Molekül noch eine reaktive Acylsulfonamidfunktion tragen, als auch deren Isolierung - ohne intermolekulare Folgereaktionen - für nicht möglich gehalten. Ein Fachmann musste annehmen, dass Sulfonamide aufgrund ihres aciden Protons mit Phenyliso(thio)cyanaten zu Sulfonylharnstoff-Derivaten reagieren. Bislang wurde daher kein Verfahren zur Herstellung von Phenyliso(thio)cyanaten, die als weitere funktionelle Gruppe eine Acylsulfonamidgruppe tragen, beschrieben.

Der Erfindung liegt die Aufgabe zugrunde, Iso(thio)cyanatobenzoylsulfamidsäureamide der Formel I bereit zu stellen.

Es wurde überraschenderweise gefunden, dass diese Aufgabe durch ein Verfahren gelöst wird, bei dem man ein Aminobenzoylsulfamidsäureamid der allgemeinen Formel II, 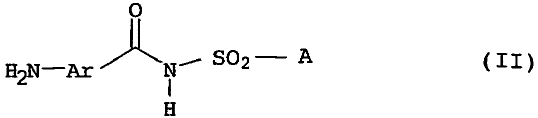 worin die Variablen Ar und A die zuvor genannten Bedeutungen aufweisen, mit Phosgen, Diphosgen bzw. Thiophosgen umsetzt.

Demgemäß betrifft die vorliegende Erfindung ein Verfahren zur Herstellung von Phenyliso(thio)cyanaten der allgemeinen Formel I, das dadurch gekennzeichnet ist, dass man eine Verbindung der allgemeinen Formel II oder deren HCl-Addukt mit Phosgen, Thiophosgen oder Diphosgen umsetzt (siehe Schema 1). In Schema 1 haben die Variablen Ar, A und W die zuvor genannten Bedeutungen.

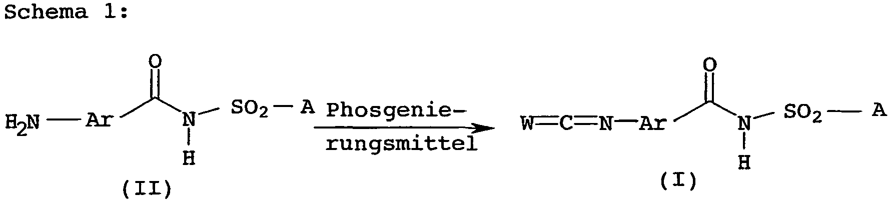

Die nach dem erfindungsgemäßen Verfahren in hoher Ausbeute erhältlichen Phenyliso(thio)cyanate I sind wertvolle Zwischenprodukte für die Herstellung von Pflanzenschutzmitteln, insbesondere von 3-(Triazolindion) substituierten Phenylsulfamoylcarboxamiden. Daher ist ein weiterer Gegenstand der vorliegenden Erfindung die Bereitstellung eines Verfahrens zur Herstellung von 3-Heterocyclyl substituierten Phenylsulfamoylcarboxamiden ausgehend von Phenyliso(thio)cyanaten I. Die erfindungsgemäßen Verbindungen I sind wider Erwarten stabile, auch im technischen Maßstab gut herstellbare Verbindungen. Die Erfindung betrifft daher auch die Phenyl-iso(thio)cyanate der allgemeinen Formel I. Die Stabilität der erfindungsgemäßen Verbindungen I ist insofern überraschend, da der Fachmann eine intermolekulare Reaktion zwischen der Iso(thio)cyanato-Struktureinheit und der Sulfamid-Gruppierung erwartet hätte.

Die bei der Definition der Substituenten genannten organischen Molekülteile stellen - wie die Bedeutung Halogen - Sammelbegriffe für individuelle Aufzählungen der einzelnen Gruppenmitglieder dar, wobei der Ausdruck Cₙ-Cₘ die mögliche Anzahl der Kohlenstoffatome im Molekülteil angibt. Sämtliche Kohlenstoffketten, also alle Alkyl-, Alkenyl- und Alkinylteile können geradkettig oder verzweigt sein. Soweit nicht anders angegeben, tragen halogenierte Substituenten vorzugsweise ein bis sechs gleiche oder verschiedene Halogenatome. Die Bedeutung Halogen steht jeweils für Fluor, Chlor, Brom oder Iod.

Ferner stehen beispielsweise:
- C₁-C₄-Alkyl für: z. B. Methyl, Ethyl, Propyl, 1-Methylethyl, Butyl, 1-Methylpropyl, 2-Methylpropyl oder 1,1-Dimethylethyl;
- C₁-C₁₀-Alkyl: ein gesättigter aliphatischer Kohlenwasserstoffrest mit 1 bis 10 C-Atomen, z. B. C₁-C₄-Alkyl, wie voranstehend genannt, sowie z. B. n-Pentyl, 1-Methylbutyl, 2-ethylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl, 1-Ethyl-3-methylpropyl, n-Heptyl, n-Nonyl, n-Decyl, 1-Methylhexyl, 1-Ethylhexyl, 1-Methylheptyl, 1-Methyloctyl, 1-Methylnonyl;
- C₂-C₁₀-Alkenyl: ein einfach ungesättigter olefinischer Kohlenwasserstoffrest mit 2 bis 10 C-Atomen, vorzugsweise 3 bis 6 C-Atomen, z. B. Ethenyl, Prop-2-en-1-yl (= Allyl), Prop-1-en-1-yl, But-1-en-4-yl, But-2-en-1-yl, But-3-en-1-yl, 1-Methylprop-2-en-1-yl, 2-Methylprop-2-en-1-yl, 1-Penten-3-yl, 1-Penten-4-yl, 2-Penten-4-yl, 1-Methylbut-2-en-1-yl, 2-Methylbut-2-en-1-yl, 3-Methyl-but-2-en-1-yl, 1-Methylbut-3-en-1-yl, 2-Methylbut-3-en-1-yl, 3-Methylbut-3-en-1-yl, 1,1-Dimethylprop-2-en-1-yl, 1,2-Dimethylprop-2-en-1-yl, 1-Ethylprop-2-en-1-yl, 1-Ethylprop-1-en-2-yl, n-Hex-1-en-1-yl, n-Hex-2-en-1-yl, Hex-3-en-1-yl, Hex-4-en-1-yl, Hex-5-en-1-yl, 1-Methylpent-l-en-1-yl, 2-Methylpent-1-en-1-yl, 3-Methylpent-1-en-1-yl, 4-Methylpent-1-en-1-yl, 1-Methylpent-2-en-1-yl, 2-Methylpent-2-en-1-yl, 3-Methylpent-2-en-1-yl, 4-Methylpent-2-en-1-yl, 1-Methylpent-3-en-1-yl, 2-Methylpent-3-en-1-yl, 3-Methylpent-3-en-1-yl, 4-Methylpent-3-en-1-yl, 1-Methylpent-4-en-1-yl, 2-Methylpent-4-en-1-yl, 3-Methylpent-4-en-1-yl, 4-Methylpent-4-en-1-yl, 1,1-Dimethylbut-2-en-1-yl, 1,1-Dimethylbut-3-en-1-yl, 1,2-Dimethyl-but-2-en-1-yl, 1,2-Dimethylbut-3-en-1-yl, 1,3-Dimethylbut-2-en-1-yl, 1,3-Dimethylbut 3-en-1-yl, 2,2-Dimethylbut-3-en-1-yl, 2,3-Dimethylbut-2-en-1-yl, 2,3-Dimethylbut-3-en-1-yl, 3,3-Dimethylbut-2-en-1-yl, 1-Ethylbut-2-en-1-yl, 1-Ethylbut-3-en-1-yl, 2-Ethylbut-2-en-1-yl, 2-Ethyl-but-3-en-1-yl, 1,1,2-Trimethylprop-2-en-1-yl, 1-Ethyl-1-methylprop-2-en-1-yl, 1-Ethyl-2-methylprop-2-en-1-yl, Hept-2-en-1-yl, Oct-2-en-1-yl, Non-2-en-1-yl, Dec-2-en-1-yl;
- C₂-C₁₀-Alkinyl: ein Kohlenwasserstoffrest mit 2 bis 10 C-Atomen, vorzugsweise 3 bis 6 C-Atomen und einer Dreifachbindung, z. B. Ethinyl, Prop-2-in-1-yl (= Propargyl), Prop-1-in-1-yl, But-1-in-1-yl, But-1-in-3-yl, But-1-in-4 yl, But-2-in-1-yl, Pent-1-in-1-yl, Pent-1-in-3-yl, Pent-1-in-4-yl, Pent-1-in-5-yl, Pent-2-in-1-yl, Pent-2-in-4-yl, Pent-2-in-5-yl, 3-Methylbut-1-in-3-yl, 3-Methylbut-1-in-4-yl, Hex-1-in-3 yl, Hex-1-in-4-yl, Hex-1-in-5-yl, Hex-1-in-6-yl, Hex-2-in-1-yl, Hex-2-in-4-yl, Hex-2-in-5-yl, Hex-2-in-6-yl, Hex-3-in-1-yl, Hex-3-in-2-yl, 3-Methylpent-1-in-3-yl, 3-Methylpent-1-in-4-yl, 3-Methylpent-1-in-5-yl, 4-Methyl-pent-2-in-4-yl, 4-Methylpent-2-in-5-yl, Hept-2-in-1-yl, Oct-2-in-1-yl, Non-2-in-1-yl, Dec-2-in-1-yl;
- C₁-C₄-Halogenalkyl für: einen C₁-C₄-Alkylrest, wie vorstehend genannt, der partiell oder vollständig durch Fluor, Chlor, Brom und/oder Iod substituiert ist, also z. B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl;
- C₁-C₁₀-Halogenalkyl: C₁-C₁₀-Alkyl wie vorstehend genannt, worin 1 bis 6 Wasserstoffatome durch Halogenatome, vorzugsweise durch Fluor und/oder Chlor substituiert sind, z. B.: C₁-C₄-Halogenalkyl, wie vorstehend genannt, sowie 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, oder 6-Iodhexyl;
- C₂-C₁₀-Halogenalkenyl: C₂-C₁₀-Alkenyl wie vorstehend genannt, worin 1 bis 6 Wasserstoffatome durch Halogenatome, vorzugsweise durch Fluor und/oder Chlor substituiert sind: z. B. 2-Chlorallyl, 3-Chlorallyl, 2,3-Dichlorallyl, 3,3-Dichlorallyl, 2,3,3-Trichlorallyl, 2,3-Dichlor-but-2-en-1-yl, 2-Bromallyl, 3-Bromallyl, 2,3-Dibromallyl, 3,3-Dibromallyl, 2,3,3-Tribromallyl oder 2,3-Dibrombut-2-en-1-yl;
- C₂-C₁₀-Halogenalkinyl: C₂-C₁₀-Alkinyl wie vorstehend genannt, worin 1 bis 6 Wasserstoffatome durch Halogenatome, vorzugsweise durch Fluor und/oder Chlor substituiert sind: z. B. 1,1-Difluorprop-2-in-1-yl, 1,1-Difluorbut-2-in-1-yl, 4-Fluorbut-2-in-1-yl, 4-Chlorbut-2-in-1-yl, 5-Fluorpent-3-in-1-yl oder 6-Fluorhex-4-in-1-yl;
- C₁-C₁₀-Cyanoalkyl: durch eine CN-Gruppe substituiertes C₁-C₁₀-Alkyl, z. B Cyanomethyl, 1-Cyanoethyl, 2-Cyanoethyl, 1-Cyanopropyl, 2-Cyanopropyl, 3-Cyanopropyl, 1-Cyanoprop-2-yl, 2-Cyanoprop-2-yl, 1-Cyanobutyl, 2-Cyanobutyl, 3-Cyanobutyl, 4-Cyanobutyl, 1-Cyanobut-2-yl, 2-Cyanobut-2-yl, 1-Cyanobut-3-yl, 2-Cyanobut-3-yl, 1-Cyano-2-methylprop-3-yl, 2-Cyano-2-methylprop-3-yl, 3-Cyano-2-methylprop-3-yl, 3-Cyano-2,2-dimethylpropyl, 6-Cyanohex-1-yl, 7-Cyanohept-1-yl, 8-Cyanooct-1-yl, 9-Cyanonon-1-yl, 10-Cyanodec-1-yl;
- C₃-C₁₀-Cycloalkyl: für einen cycloaliphatischen Rest mit 3 bis 10 C-Atomen: z B. Cyclopropyl, Cyclobutyl, Cyclopentyl, Cyclohexyl, Cycloheptyl, Cyclooctyl, Cyclononyl oder Cyclodecyl;
- C₃-C₁₀-Cycloalkenyl: für einen cycloaliphatischen Rest mit 3 bis 10 C-Atomen und einer Doppelbindung: z. B. Cyclopropen-1-yl, Cyclobuten-1-yl, Cyclopenten-1-yl, Cyclohexen-1-yl, Cyclohepten-1-yl, Cycloocten-1-yl, Cyclononen-1-yl, Cyclodecen-1-yl, Cyclopent-2-en-1-yl, Cyclohex-2-en-1-yl, Cyclohept-2-en-1-yl, Cyclooct-2-en-1-yl, Cyclonon-2-en-1-yl, Cyclodec-2-en-1-yl, Cyclohex-3-en-1-yl, Cyclohept-3-en-1-yl, Cyclooct-3-en-1-yl, Cyclooct-4-en-1-yl, Cyclonon-3-en-1-yl, Cyclonon-4-en-1-yl, Cyclodec-4-en-1-yl oder Cyclodec-3-en-1-yl;
- C₁-C₄-Alkylcarbonyl: für einen über eine Carbonylgruppe gebundenen Alkylrest mit 1 bis 4 C-Atomen, z. B. für Acetyl, Propionyl, Butyryl oder Isobutyryl;
- (C₁-C₄-Alkylamino)carbonyl: z. B. Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl oder 1,1-Dimethylethylaminocarbonyl;
- Di-(C₁-C₄-alkyl)-aminocarbonyl: z. B. N,N-Dimethylaminocarbonyl, N,N-Diethylaminocarbonyl, N,N-Di-(1-methylethyl)aminocarbonyl, N,N-Dipropylaminocarbonyl, N,N-Dibutylaminocarbonyl, N,N-Di-(1-methylpropyl)-aminocarbonyl, N,N-Di-(2-methylpropyl)-aminocarbonyl, N,N-Di-(1,1-dimethylethyl)-aminocarbonyl, N-Ethyl-N-methylaminocarbonyl, N-Methyl-N-propylaminocarbonyl, N-Methyl-N-(1-methylethyl)-aminocarbonyl, N-Butyl-N-methylaminocarbonyl, N-Methyl-N-(1-methylpropyl)-aminocarbonyl, N-Methyl-N-(2-methylpropyl)-aminocarbonyl, N-(1,1-Dimethylethyl)-N-methylaminocarbonyl, N-Ethyl-N-propylaminocarbonyl, N-Ethyl-N-(1-methylethyl)-aminocarbonyl, N-Butyl-N-ethylaminocarbonyl, N-Ethyl-N-(1-methylpropyl)-aminocarbonyl, N-Ethyl-N-(2-methylpropyl)-aminocarbonyl, N-Ethyl-N-(1,1-dimethylethyl)-aminocarbonyl, N-(1-Methylethyl)-N-propylaminocarbonyl, N-Butyl-N-propylaminocarbonyl, N-(1-Methylpropyl)-N-propylaminocarbonyl, N-(2-Methylpropyl)-N-propylaminocarbonyl, N-(1,1-Dimethylethyl)-N-propylaminocarbonyl, N-Butyl-N-(1-methylethyl)-aminocarbonyl, N-(1-Methylethyl)-N-(1-methylpropyl)-aminocarbonyl, N-(1-Methylethyl)-N-(2-methylpropyl)-aminocarbonyl, N-(1,1-Dimethylethyl)-N-(1-methylethyl)-aminocarbonyl, N-Butyl-N-(1-methylpropyl)-aminocarbonyl, N-Butyl-N-(2-methylpropyl)-aminocarbonyl, N-Butyl-N-(1,1-dimethylethyl)-aminocarbonyl, N-(1-Methylpropyl)-N-(2-methylpropyl)-aminocarbonyl, N-(1,1-Dimethylethyl)-N-(1-methylpropyl)-aminocarbonyl oder N-(1,1-Dimethylethyl)-N-(2-methylpropyl)-aminocarbonyl; - C₁-C₄-Alkoxy: für einen über ein Sauerstoffatom gebundenen Alkylrest mit 1 bis 4 C-Atomen, z. B. Methoxy, Ethoxy, Propoxy, 1-Methylethoxy, Butoxy, 1-Methylpropoxy, 2-Methylpropoxy oder 1,1-Dimethylethoxy;
- C₁-C₄-Alkoxycarbonyl: für einen über eine Carbonylgruppe gebundenen Alkoxyrest mit 1 bis 4 C-Atomen, z. B. Methoxycarbonyl, Ethoxycarbonyl, Propoxycarbonyl, 1-Methylethoxycarbonyl, Butoxycarbonyl, 1-Methylpropoxycarbonyl, 2-Methylpropoxycarbonyl oder 1,1-Dimethylethoxycarbonyl;
- C₁-C₄-Alkylthio (C₁-C₄-Alkylsulfanyl: C₁-C₄-Alkyl-S-): für einen über ein Schwefelatom gebundenen Alkylrest mit 1 bis 4 C-Atomen, z.B. Methylthio, Ethylthio, Propylthio, 1-Methylethylthio, Butylthio, 1-Methylpropylthio, 2-Methylpropylthio oder 1,1-Dimethylethylthio;
- C₁-C₄-Alkylsulfinyl (C₁-C₄-Alkyl-S(=O)-): z.B. für Methylsulfinyl, Ethylsulfinyl, Propylsulfinyl, 1-Methylethylsulfinyl, Butylsulfinyl, 1-Methylpropylsulfinyl, 2-Methylpropylsulfinyl oder 1,1-Dimethylethylsulfinyl;
- C₁-C₄-Alkylsulfonyl (C₁-C₄ Alkyl-S(=O)₂-): z. B. für Methylsulfonyl, Ethylsulfonyl, Propylsulfonyl, 1-Methylethylsulfonyl, Butylsulfonyl, 1-Methylpropylsulfonyl, 2-Methylpropylsulfonyl oder 1,1-Dimethylethylsulfonyl;
- 3- bis 8-gliedriges Heterocyclyl: ein heterocyclischer Rest, der 3, 4, 5, 6, 7 oder 8 Ringglieder aufweist, wobei 1, 2 oder 3 der Ringglieder Heteroatome sind, die ausgewählt sind unter Sauerstoff, Schwefel, Stickstoff und einer Gruppe NR⁶ (worin R⁶ für Wasserstoff, C₁-C₆-Alkyl, C₃-C₆-Alkenyl oder C₃-C₆-Alkinyl steht). Außerdem kann der Heterocyclus gegebenenfalls ein oder zwei Carbonylgruppen oder Thiocarbonylgrupen als Ringglieder aufweisen. Der Heterocyclus kann aromatisch (Heteroaryl) oder teilweise oder vollständig gesättigt sein.

Beispiele für gesättigte Heterocyclen sind:
Oxiran-1-yl, Aziridin-1-yl, Oxetan-2-yl, Oxetan-3-yl, Thietan-2-yl, Thietan-3-yl, Azetidin-1-yl, Azetidin-2-yl, Azetidin-3-yl, Tetrahydrofuran-2-yl, Tetrahydrofuran-3-yl, Tetra-hydrothiophen-2-yl, Tetrahydrothiophen-3-yl, Pyrrolidin-1-yl, Pyrrolidin-2-yl, Pyrrolidin-3-yl, 1,3-Dioxolan-2-yl, 1,3-Dioxolan-4-yl, 1,3-Oxathiolan-2-yl, 1,3-Oxathiolan-4-yl, 1,3-Oxathiolan-5-yl, 1,3-Oxazolidin-2-yl, 1,3-Oxazolidin-3-yl, 1,3-Oxazolidin-4-yl, 1,3-Oxazolidin-5-yl, 1,2-Oxa-zolidin-2-yl, 1,2-Oxazolidin-3-yl, 1,2-Oxazolidin-4-yl, 1,2-Oxazolidin-5-yl, 1,3-Dithiolan-2-yl, 1,3-Dithiolan-4-yl, Pyrrolidin-1-yl, Pyrrolidin-2-yl, Pyrrolidin-5-yl, Tetrahydropyrazol-1-yl, Tetrahydropyrazol-3-yl, Tetrahydropyrazol-4-yl, Tetrahydropyran-2-yl, Tetrahydropyran-3-yl, Tetrahydropyran-4-yl, Tetrahydrothiopyran-2-yl, Tetrahydrothiopyran-3-yl, Tetrahydropyran-4-yl, Piperidin-1-yl, Piperidin-2-yl, Piperidin-3-yl, Piperidin-4-yl, 1,3-Dioxan-2-yl, 1,3-Dioxan-4-yl, 1,3-Dioxan-5-yl, 1,4-Dioxan-2-yl, 1,3-Oxa-thian-2-yl, 1,3-Oxathian-4-yl, 1,3-Oxathian-5-yl, 1,3-Oxa-thian-6-yl, 1,4-Oxathian-2-yl, 1,4-Oxathian-3-yl, Morpholin-2-yl, Morpholin-3-yl, Morpholin-4-yl, Hexahydropyridazin-1-yl, Hexahydropyridazin-3-yl, Hexahydropyridazin-4-yl, Hexahydropyrimidin-1-yl, Hexahydropyrimidin-2-yl, Hexahydropyrimidin-4-yl, Hexahydropyrimidin-5-yl, Piperazin-1-yl, Piperazin-2-yl, Piperazin-3-yl, Hexahydro-1,3,5-triazin-1-yl, Hexahydro-1,3,5-triazin-2-yl, Oxepan-2-yl, Oxepan-3-yl, Oxepan-4-yl, Thiepan-2-yl, Thiepan-3-yl, Thiepan-4-yl, 1,3-Dioxepan-2-yl, 1,3-Dioxepan-4-yl, 1,3-Dioxepan-5-yl, 1,3-Dioxepan-6-yl, 1,3-Dithiepan-2-yl, 1,3-Dithiepan-4-yl, 1,3-Dithiepan-5-yl, 1,3-Dithiepan-2-yl, 1,4-Dioxepan-2-yl, 1,4-Dioxepan-7-yl, Hexahydroazepin-1-yl, Hexahydroazepin-2-yl, Hexahydroazepin-3-yl, Hexahydroazepin-4-yl, Hexahydro-1,3-diazepin-1-yl, Hexahydro-1,3-diazepin-2-yl, Hexahydro-1,3-diazepin-4-yl, Hexahydro-1,4-diazepin-1-yl und Hexahydro-1,4-diazepin-2-yl;

Beispiele für ungesättigte Heterocyclen sind: Dihydrofuran-2-yl, 1,2-Oxazolin-3-yl, 1,2-Oxazolin-5-yl, 1,3-Oxazolin-2-yl;

Beispiele für aromatisches Heterocyclyl sind die 5- und 6-gliedrigen aromatischen, heterocyclischen Reste, z.B. Furyl wie 2-Furyl und 3-Furyl, Thienyl wie 2-Thienyl und 3-Thienyl, Pyrrolyl wie 2-Pyrrolyl und 3-Pyrrolyl, Isoxazolyl wie 3-Isoxazolyl, 4-Isoxazolyl und 5-Isoxazolyl, Isothiazolyl wie 3-Isothiazolyl, 4-Isothiazolyl und 5-Isothiazolyl, Pyrazolyl wie 3-Pyrazolyl, 4-Pyrazolyl und 5-Pyrazolyl, Oxazolyl wie 2-Oxazolyl, 4-Oxazolyl und 5-Oxazolyl, Thiazolyl wie 2-Thiazolyl, 4-Thiazolyl und 5-Thiazolyl, Imidazolyl wie 2-Imidazolyl und 4-Imidazolyl, Oxadiazolyl wie 1,2,4-Oxadiazol-3-yl, 1,2,4-Oxadiazol-5-yl und 1,3,4-Oxadiazol-2-yl, Thiadiazolyl wie 1,2,4-Thiadiazol-3-yl, 1,2,4-Thiadiazol-5-yl und 1,3,4-Thiadiazol-2-yl, Triazolyl wie 1,2,4-Triazol-1-yl, 1,2,4-Triazol-3-yl und 1,2,4-Triazol-4-yl, Pyridinyl wie 2-Pyridinyl, 3-Pyridinyl und 4-Pyridinyl, Pyridazinyl wie 3-Pyridazinyl und 4-Pyridazinyl, Pyrimidinyl wie 2-Pyrimidinyl, 4-Pyrimidinyl und 5-Pyrimidinyl, des weiteren 2-Pyrazinyl, 1,3,5-Triazin-2-yl und 1,2,4-Triazin-3-yl, insbesondere Pyridyl, Furanyl und Thienyl.

Der von einem primären oder sekundären Amin abgeleitete Rest A steht in der Regel für eine Gruppe der Formel -NR¹R²,
worin die Variablen R¹ und R² die folgenden Bedeutungen aufweisen:
- R¹ und R²: stehen unabhängig voneinander für Wasserstoff, C₁-C₁₀-Alkyl, C₂-C₁₀-Alkenyl oder C₂-C₁₀-Alkinyl, die unsubstituiert oder durch einen der folgenden Reste substituiert sein können: C₁-C₄-Alkoxy, C₁-C₄-Alkylthio, CN, NO₂, Formyl, C₁-C₄-Alkylcarbonyl, C₁-C₄-Alkoxycarbonyl, C₁-C₄-Alkylaminocarbonyl, C₁-C₄-Dialkylaminocarbonyl, C₁-C₄-Alkylsulfinyl, C₁-C₄-Alkylsulfonyl, C₃-C₁₀-Cycloalkyl, 3- bis 8-gliedriges Heterocyclyl mit ein, zwei oder drei unter O, S, N und einer Gruppe NR⁶ (worin R⁶ für Wasserstoff, C₁-C₆-Alkyl, C₃-C₆-Alkenyl oder C₃-C₆-Alkinyl steht) ausgewählten Heteroatomen, Phenyl, das seinerseits 1, 2, 3 oder 4 Substituenten, ausgewählt unter Halogen, C₁-C₄-Alkyl, C₁-C₄-Alkoxy, C₁-C₄-Fluoralkyl, C₁-C₄-Alkyloxycarbonyl, Trifluormethylsulfonyl, C₁-C₃-Alkylamino, C₁-C₃-Dialkylamino, Formyl, Nitro oder Cyano, aufweisen kann, C₁-C₁₀-Halogenalkyl, C₂-C₁₀-Halogenalkenyl, C₂-C₁₀-Halogen-alkinyl, C₃-C₈-Cycloalkyl, C₃-C₁₀-Cycloalkenyl, 3- bis 8-gliedriges Heterocyclyl mit ein bis drei Heteroatomen, ausgewählt unter O, S, N und einer Gruppe NR⁶ (worin R⁶ für Wasserstoff, C₁-C₆-Alkyl, C₃-C₆-Alkenyl oder C₃-C₆-Alkinyl steht), Phenyl oder Naphthyl, wobei C₃-C₈-Cycloalkyl, C₃-C₁₀-Cycloalkenyl, 3-bis 8-gliedriges Heterocyclyl, Phenyl oder Naphthyl, ihrerseits 1, 2, 3 oder 4 Substituenten, ausgewählt unter Halogen, C₁-C₄-Alkyl, C₁-C₄-Alkoxy, C₁-C₄-Fluoralkyl, C₁-C₄-Alkyloxycarbonyl, Trifluormethylsulfonyl, Formyl, C₁-C₃-Alkylamino, C₁-C₃-Dialkylamino, Phenoxy, Nitro oder Cyano, aufweisen können, oder
- R¹ und R²: bilden gemeinsam einen gesättigten oder teilweise ungesättigten 5- bis 8-gliedrigen Stickstoffheterocyclus, der seinerseits durch C₁-C₄-Alkyl, C₁-C₄-Alkoxy und/oder C₁-C₄-Halogenalkyl, substituiert sein kann, ein oder zwei Carbonylgruppen, Thiocarbonylgruppen und/oder ein oder zwei weitere Heteroatome, ausgewählt unter O, S, N und einer Gruppe NR⁶ (worin R⁶ die zuvor genannten Bedeutungen aufweist), als Ringglieder aufweisen kann.
Bevorzugte Substituenten R¹ und R² sind unabhängig voneinander ausgewählt unter Wasserstoff, C₁-C₆-Alkyl, das gegebenenfalls durch einen Substituenten ausgewählt unter Halogen, Cyano, C₁-C₄-Alkoxy, C₁-C₄-Alkoxycarbonyl, C₁-C₄-Alkylthio, C₃-C₈-Cycloalkyl, Phenyl, das seinerseits gegebenenfalls durch Halogen oder C₁-C₄-Alkoxy substituiert ist, Furyl, Thienyl, 1,3-Dioxolanyl substituiert ist. Bevorzugt sind weiterhin C₂-C₆-Alkenyl, C₂-C₆-Alkinyl, C₃-C₈-Cycloalkyl oder Phenyl, das gegebenenfalls durch 1 oder 2 Substituenten, ausgewählt unter Halogen, C₁-C₄-Alkyl, C₁-C₄-Fluoralkyl, C₁-C₄-Alkoxy, C₁-C₄-Alkoxycarbonyl, Nitro oder C₁-C₃-Dialkylamino substituiert ist, Naphthtyl oder Pyridyl. In einer weiteren bevorzugten Ausführungsform bilden R¹ und R² zusammen einen fünf-, sechs- oder siebengliedrigen gesättigten oder ungesättigten Stickstoffheterocyclus, der ein weiteres Heteroatom, ausgewählt unter N, einer Gruppe NR⁶ (worin R⁶ die zuvor genannten Bedeutungen aufweist) und 0, als Ringglied enthalten kann, und/oder durch ein, zwei oder drei Substituenten, ausgewählt unter C₁-C₄-Alkyl und C₁-C₄-Halogenalkyl, substituiert sein kann.

In einer besonders bevorzugten Ausführungsform der Erfindung steht einer der Reste R¹ oder R² für Wasserstoff, C₁-C₆-Alkyl, C₂-C₆-Alkenyl oder C₂-C₆-Alkinyl und der andere Rest R¹ oder R² für C₁-C₆-Alkyl, C₃-C₈-Cycloalkyl oder Phenyl.

Die Gruppe Ar steht insbesondere für eine Gruppe der allgemeinen Formel Ar-1 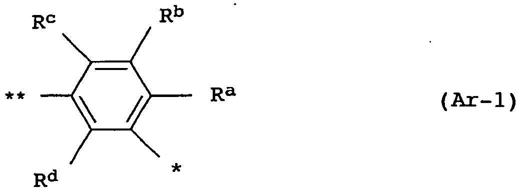 worin
- R^{a}, R^{b}, R^{c} und R^{d}: unabhängig voneinander für Wasserstoff, Halogen, C₁-C₄-Halogenalkyl oder Cyano stehen;
- *: die Verknüpfung von Ar mit der C(O)-Gruppe kennzeichnet und
- **: die Verknüpfung von Ar mit dem Stickstoffatom der Amino-, Nitro- beziehungsweise Iso(thio)cyanato-Gruppe kennzeichnet.

In einer besonders bevorzugten Ausführungsform der Erfindung weisen die Variablen R^{a}, R^{b}, R^{c} und R^{d} die folgenden Bedeutungen, und zwar jeweils für sich allein oder in Kombination auf:
- R^{a}: Halogen oder Cyano, insbesondere Fluor, Chlor oder Cyano;
- R^{b}: Wasserstoff;
- R^{c}: Halogen oder Wasserstoff, insbesondere Fluor, Chlor oder Wasserstoff;
- R^{d}: Wasserstoff.

Demnach betrifft die vorliegende Erfindung besonders die Herstellung der Verbindungen IA, 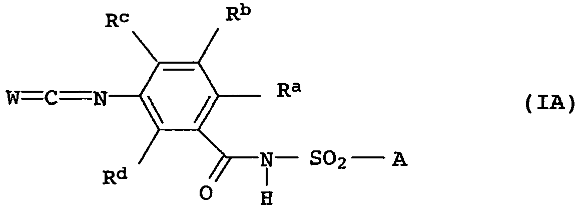 worin die Variablen R^{a}, R^{b}, R^{c}, R^{d}, A und W die zuvor genannten Bedeutungen aufweisen.

Insbesondere betrifft die vorliegende Erfindung die Herstellung der Verbindungen IA.1, worin A für NR¹R² steht. Diese Verbindungen werden im Folgenden als Verbindungen IA.1 bezeichnet.

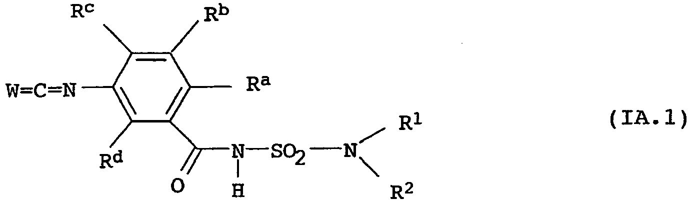

Die Umsetzung von Verbindung II mit Phosgen, Thiophosgen oder Diphosgen, im Folgenden auch als Phosgenierungsmittel bezeichnet, erfolgt üblicherweise in einem inerten organischen Lösungsmittel. Als Lösungsmittel verwendet man für diese Umsetzungen - je nach Temperaturbereich - Kohlenwasserstoffe wie Pentan, Hexan, Cyclopentan, Cyclohexan, Toluol, Xylol, chlorierte Kohlenwasserstoffe wie Methylenchlorid, Chloroform, 1,2-Dichlorethan, 1,1,2,2-Tetrachlorethan, Chlorbenzol, 1,2-, 1,3- oder 1,4-Dichlorbenzol, Ether wie 1,4-Dioxan, Anisol, Glykolether wie Dimethylglykolether, Diethylglykolether, Diethylenglykoldimethylether, Ester wie Ethylacetat, Propylacetat, Methylisobutyrat, Isobutylacetat, Carbonsäureamide wie N,N-Dimethylformamid, N-Methylpyrrolidon, Nitrokohlenwasserstoffe wie Nitrobenzol, Tetraalkylharnstoffe wie Tetraethylharnstoff, Tetrabutylharnstoff, Dimethylethylenharnstoff, Dimethylpropylenharnstoff, Nitrile wie Acetonitril, Propionitril, Butyronitril oder Isobutyronitril oder auch Gemische einzelner Lösungsmittel.

Bei Verwendung von Phosgen wird man vorzugsweise ein Lösungsmittel einsetzen, das weitgehend von protischen Verunreinigungen wie Wasser und Alkoholen befreit ist. Bei der Herstellung der Isothiocyanate kann man jedoch auch in Anlehnung an Houben-Weyl, Methoden der organischen Chemie, 4. Auflage, Bd. IX, S. 875, die Umsetzung von II mit Thiophosgen in einem Zweiphasensystem aus Wasser und einem damit nicht mischbaren organischen Lösungsmittel oder auch in Wasser durchführen.

In der Regel wird man die Verbindung II in einem Reaktionsgefäß, vorzugsweise als Lösung oder Suspension in einem der vorgenannten Lösungsmittel vorlegen und dann das Phosgenierungsmittel zugeben. Vorzugsweise erfolgt die Zugabe des Phosgenierungsmittels unter Rühren. Die Zugabe erfolgt vorzugsweise über einen Zeitraum von 10 bis 60 Minuten. Das Phosgenierungsmittel kann als solches oder als Lösung in einem der vorgenannten Lösungsmittel zugegeben werden. Im Falle des Phosgens wird man dieses in der Regel in die Lösung beziehungsweise in die Suspension einleiten.

Die Reaktionstemperatur wird in der Regel 180 °C, vorzugsweise 120 °C und insbesondere 100 °C nicht überschreiten und wird in der Regel wenigstens 40 °C und vorzugsweise wenigstens 50 °C betragen. Häufig wird man so vorgehen, dass man zumindest die Hauptmenge des Phosgenierungsmittels bei einer niedrigen Temperatur, z. B. im Bereich von 0 bis 40 °C, insbesondere 10 bis 40 °C und speziell 20 bis 30°C zugibt und während oder nach beendeter Zugabe auf eine Temperatur im Bereich von 40 bis 180 °C, insbesondere 50 bis 120 °C und speziell 70 bis 100 °C erwärmt bis der Umsatz vollständig ist.

In der Regel setzt man 0,9 bis 2, vorzugsweise 0,95 bis 1,5, besonders bevorzugt 0,98 bis 1,09 Moläquivalente Phosgenierungsmittel pro Mol der Verbindung II ein.

Gegebenenfalls führt man die Umsetzung von II in Gegenwart einer Base durch. Als Basen kommen beispielsweise basische anorganische Verbindungen in Betracht, z. B. Alkali- oder Erdalkalihydroxide, -hydrogencarbonate oder -carbonate. Man kann die Reaktion jedoch auch in Gegenwart einer organischen Base, beispielsweise eines tertiären Amins wie Triethylamin, Tri-n-propylamin, N-Ethyldiisopropylamin, Pyridin, α-, β-,γ-Picolin, 2,4-, 2,6-Lutidin, N-Methylpyrrolidin, Dimethylanilin, N,N-Dimethylcyclohexylamin, Chinolin oder Acridin durchführen. Die Base (berechnet als Basenäquivalent) kann substöchiometrisch, überstöchiometrisch oder äquimolar, bezogen auf die Verbindung II, eingesetzt werden. Pro Mol der Verbindung II setzt man im allgemeinen 0,01 bis 6 Mol, vorzugsweise 0,1 bis 3 Mol Base ein.

In einer anderen Ausführungsform des erfindungsgemäßen Verfahrens führt man die Umsetzung in Gegenwart von Chlorwasserstoff durch. Die Menge an Chlorwasserstoff beträgt dann üblicherweise 0,9 bis 5,0 mol, vorzugsweise 1,0 bis 2,5 mol und insbesondere 1,0 bis 1,2 mol Chlorwasserstoff pro Mol der Verbindung II. Hierbei wird man in der Regel so vorgehen, dass man zunächst in eine Lösung oder Suspension die Verbindung II in einem der vorgenannten Lösungsmittel die vorgenannte Menge an gasförmigen Chlorwasserstoff einleitet oder eine Lösung von Chlorwasserstoff in einem Lösungsmittel zugibt, dann das Phosgenierungsmittel in der zuvor beschriebenen Weise zugibt und die Reaktion dann in der oben beschriebenen Weise fortführt. Das Einleiten von Chlorwasserstoff erfolgt üblicherweise bei Temperaturen zwischen 10 °C und 60 °C, vorzugsweise bei 20 bis 30°C.

Sofern man das Verfahren in Gegenwart von Chlorwasserstoff durchführt, kann Aktivkohle als Katalysator verwendet werden. Zweckmäßigerweise beträgt die Menge an Aktivkohle 1 bis 10 Gew.-%, vorzugsweise 1 bis 3 Gew.-%, bezogen auf das Gewicht der Verbindung II.

Die Reaktion kann drucklos oder unter Druck, kontinuierlich oder diskontinuierlich durchgeführt werden. In der Regel wird man die Umsetzung der Verbindung II mit einem Phosgenierungsmittel unter Ausschluss von Wasser durchführen. Gegebenenfalls kann es von Vorteil sein, die Umsetzung unter einer Schutzgasatmosphäre durchzuführen.

Die Aufarbeitung zur Gewinnung des Zielproduktes kann nach den hierfür üblichen Verfahren erfolgen. Bei Verwendung von Phosgen als Phosgenierungsmittel wird man in der Regel zunächst unumgesetztes Phosgen entfernen, beispielsweise indem man einen Stickstoffstrom in das Reaktionsgemisch einleitet. Anschließend wird man das Lösungsmittel nach üblichen Verfahren, beispielsweise destillativ, entfernen. Zur weiteren Reinigung kann man übliche Verfahren wie Kristallisation, Chromatographie, beispielsweise an Kieselgel, anwenden. Gegebenenfalls kann man den Rückstand auch durch Verrühren mit einem Lösungsmittel, beispielsweise aromatische Kohlenwasserstoffe wie Benzol, Toluol oder Xylol oder aliphatische Kohlenwasserstoffe wie Petrolether, Hexan, Cyclohexan, Pentan, Ether wie Diethylether usw. und Gemischen davon reinigen.

Die zur Durchführung des erfindungsgemäßen Verfahrens als Edukte benötigten Verbindungen der Formel II sind ebenfalls neu und als interessante Vorstufen für das erfindungsgemäße Verfahren von Bedeutung. In der Formel II stehen die Variablen Ar und A vorzugsweise für diejenigen Reste, die bereits im Zusammenhang mit der Beschreibung der erfindungsgemäßen Verbindungen I als bevorzugt für diese Substituenten genannt wurden.

Die Verbindungen der Formel II können in Analogie zu bekannten Verfahren zur Herstellung von Anilinen gewonnen werden. Die Anilinverbindungen der Formel II kann man beispielsweise gemäß Schema 2 herstellen, indem man zunächst eine Aroylverbindung der Formel III mit einem Sulfamidsäureamid IV im Sinne einer Kondensationsreaktion zu einem N-Aroylsulfamidsäureamid der allgemeinen Formel V umsetzt und anschließend das erhaltene N-Aroylsulfamidsäureamid V zur Verbindung II reduziert.

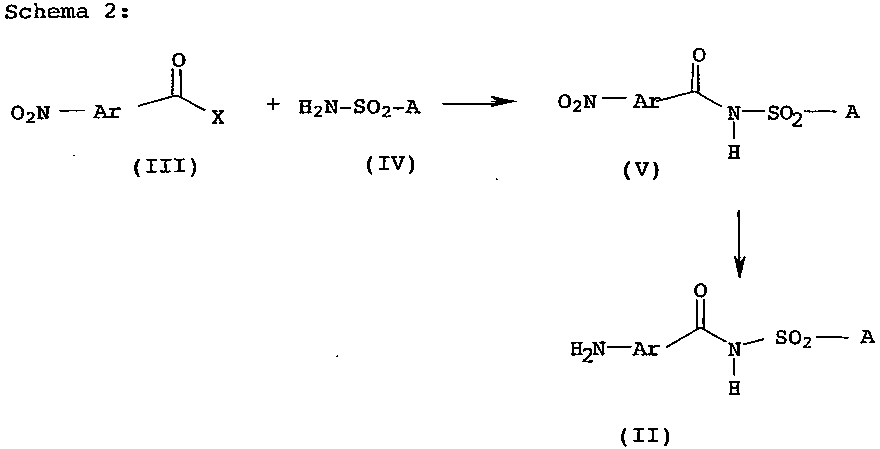

In Schema 2 haben die Variablen A und Ar die vorgenannten Bedeutungen, insbesondere die als bevorzugt angegebenen Bedeutungen. X steht für Halogen, vorzugsweise Chlor, Hydroxy oder eine C₁-C₄-Alkoxygruppe. Die Kondensation von Aroylverbindungen der allgemeinen Formel III mit Sulfamidsäureamiden der allgemeinen Formel IV zu den entsprechenden Benzoylsulfamiden der allgemeinen Formel V erfolgt in Anlehnung an bekannte Verfahren, beispielsweise wie in der WO 01/83459, S. 31-35, in der unveröffentlichten deutschen Patentanmeldung DE 102 21 910.0 beschrieben .

Im Folgenden wird der erste Reaktionsschritt näher erläutert:

Sofern X in Formel III für Hydroxy steht, aktiviert man vorzugsweise zunächst die Carbonsäure III, indem man sie mit einem Kupplungsmittel umsetzt. Anschließend setzt man die aktivierte Carbonsäure III in der Regel ohne vorherige Isolierung mit dem Sulfamidsäureamid IV um. Als Kupplungsmittel kommen beispielsweise N,N'-Carbonyldiimidazol oder Carbodiimide wie Dicyclohexylcarbodiimid in Betracht. Diese werden in der Regel wenigstens in äquimolarer Menge und bis zu einem vierfachen Überschuss, bezogen auf die Carbonsäure III, eingesetzt. Gegebenenfalls erwärmt man das erhaltene Reaktionsgemisch aus Carbonsäure III und Kupplungsmittel und lässt dann auf Raumtemperatur abkühlen. Üblicherweise führt man die Umsetzung in einem Lösungsmittel durch. Als Lösungsmittel kommen z. B. chlorierte Kohlenwasserstoffe wie Methylenchlorid, 1,2-Dichlorethan, Ether z. B. Dialkylether wie Diethylether, Methyl-tert.-butylether oder cyclische Ether wie Tetrahydrofuran oder Dioxan, Carbonsäureamide wie Dimethylformamid, N-Methyllactame wie N-Methylpyrrolidon, Nitrile wie Acetonitril, aromatische Kohlenwasserstoffe wie Toluol, aromatische Amine wie Pyridin oder Gemische hiervon in Betracht. Anschließend versetzt man mit dem Sulfamidsäureamid IV. In der Regel löst man das Sulfamid IV in dem Lösungsmittel, das auch zur Aktivierung der Carbonsäure verwendet wurde.

Sofern X in Formel III für C₁-C₄-Alkoxy steht, kann man zunächst die Ester nach bekannten Verfahren durch Hydrolyse im sauren Milieu unter Verwendung von starken Mineralsäuren wie konzentrierte Salzsäure oder Schwefelsäure oder organischen Säuren wie Eisessig oder Gemischen davon in die entsprechenden Carbonsäuren III überführen. Alternativ lassen sich Ester auch im alkalischen Milieu unter Verwendung von Basen wie Alkalihydroxid, beispielsweise Natriumhydroxid oder Kaliumhydroxid in Gegenwart von Wasser hydrolysieren.

Anschließend kann man die Carbonsäuren III (X = OH) in der oben beschriebenen Weise umsetzen oder mit einem Chlorierungsagens wie Thionylchlorid oder Phosgen zunächst in die Säurechloride (X = Cl) überführen und diese in der nachfolgend beschriebenen Weise mit IV umsetzen. Die Darstellung der Säurechloride erfolgt in Anlehnung an bekannte Verfahren, beispielsweise wie in der EP 1 176 133 und WO 01/087872 beschrieben.

Man kann jedoch auch direkt den Carbonsäureester der Formel III, worin X für C₁-C₄-Alkoxy steht, mit einem Sulfamidsäureamid IV oder dessen Metallsalz im Sinne einer Amidierungsreaktion unter Abspaltung des Esterrestes umsetzen. Die Umsetzung wird man in Anlehnung an die in Houben-Weyl, 4. Auflage, Bd. VIII, S. 658 - 659 beschriebene Arbeitsweise durchführen.

Sofern X in Formel III für Halogen steht, wird man in der Regel so vorgehen, dass man die Aroylverbindung III, vorzugsweise verdünnt in einem inerten Lösungsmittel, zu dem Sulfamidsäureamid der Formel IV, vorzugsweise ebenfalls verdünnt in einem inerten Lösungsmittel, gibt. Selbstverständlich kann man auch die Aroylverbindung III vorlegen und hierzu das Sulfamidsäureamid IV geben.

Die molaren Verhältnisse, in denen die Ausgangsverbindungen III und IV miteinander umgesetzt werden, betragen im Allgemeinen 0,9 bis 1,2, vorzugsweise 0,95 bis 1,1, besonders bevorzugt 0,98 bis 1,04 für das Verhältnis von Aroylverbindung III zu Sulfamidsäureamid IV.

Üblicherweise führt man die Umsetzung bei Temperaturen im Bereich von -30 bis 100 °C, vorzugsweise -10 bis 80 °C, besonders bevorzugt 0 bis 60 °C durch.

Vorteilhaft arbeitet man im ersten Reaktionsschritt unter neutralen Bedingungen. Sofern bei der Reaktion ein saures Reaktionsprodukt, z. B. Halogenwasserstoff (wenn X in Formel III für Halogen steht) entsteht, so entfernt man diesen durch Zugabe einer basischen Verbindung. Zu den geeigneten basischen Verbindungen zählen anorganische und organische Basen. Geeignete anorganische basische Verbindungen sind z. B. Alkali- oder Erdalkalihydroxide bzw. -hydrogencarbonate oder -carbonate. Man kann die Reaktion jedoch auch in Gegenwart einer organischen Base, z. B. Triethylamin, Tri-n-propylamin, N-Ethyldiisopropylamin, Pyridin, α-, β-, γ-Picolin, 2,4-, 2,6-Lutidin, N-Methylpyrrolidin, Dimethylanilin, N,N-Dimethylcyclohexylamin, Chinolin oder Acridin durchführen. In der Regel setzt man die Base im Überschuss, bezogen auf die Verbindung III, ein. Die molare Menge an Base beträgt 1,0 bis 2 Mol, vorzugsweise 1,02 bis 1,3 Mol Base (berechnet als Basenäquivalent) pro Mol der Verbindung III. Gegebenenfalls enthält das Reaktionsgemisch Pyridin oder eine Pyridinverbindung, beispielsweise ein 4-Dialkylaminopyridin wie 4-Dimethylaminopyridin als Katalysator. Der Zusatz an Katalysator beträgt etwa 0,1 - 10 %, bezogen auf die Verbindung III.

Die Umsetzung der Aroylverbindungen III mit den Verbindungen der Formel IV wird vorteilhaft in Gegenwart eines Lösungsmittels durchgeführt. Als Lösungsmittel verwendet man für diese Umsetzungen - je nach Temperaturbereich - Kohlenwasserstoffe wie Pentan, Hexan, Cyclopentan, Cyclohexan, Toluol, Xylol, chlorierte Kohlenwasserstoffe wie Methylenchlorid, Chloroform, 1,2-Dichlorethan, 1,1,2,2-Tetrachlorethan, Chlorbenzol, 1,2-, 1,3- oder 1,4-Dichlorbenzol, Ether wie 1,4-Dioxan, Anisol, Glykolether wie Dimethylglykolether, Diethylglykolether, Diethylenglykoldimethylether, Ester wie Ethylacetat, Propylacetat, Methylisobutyrat, Isobutylacetat, Carbonsäureamide wie N,N-Dimethylformamid, N-Methylpyrrolidon, Nitrokohlenwasserstoffe wie Nitrobenzol, Tetraalkylharnstoffe wie Tetraethylharnstoff, Tetrabutylharnstoff, Dimethylethylenharnstoff, Dimethylpropylenharnstoff, Sulfoxide wie Dimethylsulfoxid, Sulfone wie Dimethylsulfon, Diethylsulfon, Tetramethylensulfon, Nitrile wie Acetonitril, Propionitril, Butyronitril oder Isobutyronitril; Wasser oder auch Gemische einzelner Lösungsmittel.

Ferner kann man die Reaktion auch in einem wässrigen Zweiphasensystem durchführen, vorzugsweise in Gegenwart von Phasentransferkatalysatoren wie quartären Ammonium- oder Phosphoniumsalzen. Für die Zweiphasen-Reaktion sind die in der EP-A 556737 beschriebenen Reaktionsbedingungen geeignet.

Als Phasentransferkatalysatoren können quartäre Ammonium- oder Phosphoniumsalze verwendet werden. An geeigneten Verbindungen seien folgende genannt: Tetraalkyl-(C₁-C₁₈)-ammoniumchloride, -bromide oder -fluoride, N-Benzyltrialkyl-(C₁-C₁₈)-ammoniumchloride, -bromide oder -fluoride, Tetraalkyl-(C₁-C₁₈)-phosphonium-chloride oder -bromide, Tetraphenylphosponiumchlorid oder -bromid, (Phenyl)ₒ(C₁-C,₈-alkyl)ₚ-phosponiumchloride oder -bromide, wobei o = 1 bis 3, p = 3 bis 1 und o + p = 4 ist. Besonders bevorzugt sind Tetraethylammoniumchlorid und N-Benzyltriethylammoniumchlorid. Die Menge an Phasentransferkatalysator beträgt im Allgemeinen bis zu 20 Gew.-%, bevorzugt zwischen 1 und 15 Gew.-% und besonders bevorzugt zwischen 2 bis 8 Gew.-%, bezogen auf die Ausgangsverbindung IV.

vorteilhaft gibt man die Aroylverbindung III innerhalb eines Zeitraums von 0,25 bis 2 Stunden zu einer Mischung des Sulfamidsäureamids IV und gegebenenfalls der Base in einem der vorgenannten Lösungsmittel und rührt zur Vervollständigung der Reaktion noch 0,5 bis 16 Stunden, vorzugsweise 2 bis 8 Stunden nach. Die Reaktionstemperatur liegt in der Regel zwischen 0 °C und 60 °C.

Bei Verwendung eines wässrigen Zweiphasensystems kann man in beliebiger Reihenfolge die Ausgangsstoffe III und IV zu einer Mischung des Phasentransferkatalysators in den beiden Phasen unter Rühren zugeben und dann im genannten Temperaturbereich unter Zugabe von Base die Umsetzung zu Ende bringen.

Die Reaktion kann kontinuierlich oder diskontinuierlich, drucklos oder unter Druck durchgeführt werden.

Zur Aufarbeitung extrahiert man die organische Phase mit verdünnter Mineralsäure wie Salzsäure, trocknet die organische Phase und entfernt das Lösungsmittel im Vakuum. Gegebenenfalls kann man den Rückstand auch durch Verrühren mit einem Lösungsmittel oder Lösungsmittelgemisch, beispielsweise aromatische Kohlenwasserstoffe wie Benzol, Xylol oder Toluol oder aliphatische oder cycloaliphatische Kohlenwasserstoffe wie Petrolether, Pentan,Hexan oder Cyclohexan, Ether wie Diethylether usw. und Gemischen davon, Absaugen und Trocknen noch weiter reinigen.

Im Folgenden wird der 2. Reaktionsschritt, die Reduktion der Nitroverbindung V zu der Verbindung II, näher erläutert.

Die Reduktion der Verbindung V zur Verbindung II gelingt beispielsweise mit nascierendem Wasserstoff. Hierzu setzt man die Nitroverbindung mit einer Säure in Gegenwart eines unedlen Metalls um. Unedle Metalle sind naturgemäß solche, die von einer Brönstedsäure unter Wasserstoffentwicklung gelöst werden. Derartige Metalle weisen in der Regel ein Normalpotential < 0 V und insbesondere kleiner gleich -0,1 V, z. B. im Bereich von -0,1 bis -1,0 V (in saurer wässriger Lösung bei 15°C und 1 bar) auf. Beispiele für geeignete Metalle sind Zn, Fe und Sn, insbesondere Fe. Als Säuren kommen für diesen Zweck sowohl anorganische Mineralsäuren, beispielsweise Salzsäure oder verdünnte Schwefelsäure, oder Mischungen aus anorganischer Säure und einem der vorgenannten Lösungsmittel, beispielsweise gasförmige HCl in einem Ether oder einem Alkohol oder in einer Mischung davon, oder organische Carbonsäuren, zweckmäßig Essigsäure, Propionsäure oder Buttersäure, in Betracht.

Die Reaktionsbedingungen entsprechen im Wesentlichen den für die Reduktion aliphatischer oder aromatischer Nitrogruppen zu aliphatischen oder aromatischen Aminogruppen mit nascierendem Wasserstoff angewendeten Reaktionsbedingungen (siehe beispielsweise H. Koopman, Rec. Trav. 80 (1961), 1075; siehe auch N. Kornblum, L. Fischbein, J. Am. Chem. Soc. 77, (1955) 6266).

Je nach Art des Metalls und Säure liegt die Reaktionstemperatur in der Regel im Bereich von -20 bis +120°C, wobei man bei Verwendung von Alkansäuren wie Essigsäure vorzugsweise Temperaturen im Bereich von 50 bis 100°C anwendet. Die Reaktionsdauer kann wenige Minuten bis mehrere Stunden, z. B. etwa 20 Minuten bis 5 Stunden, betragen. Vorzugsweise legt man die zu reduzierende Verbindung V im Reaktionsgefäß vor und gibt dann unter Durchmischen das jeweilige Metall, vorzugsweise in feinteiliger Form, insbesonder als Pulver zu der Reaktionsmischung. Vorzugsweise erfolgt die Zugabe über einen Zeitraum von 10 Minuten bis 2 Stunden. Selbstverständlich kann man auch das Metall und die Säure vorlegen und die Verbindung V, gegebenenfalls zusammen mit einem inerten Lösungsmittel, zugeben. Häufig lässt man das Reaktionsgemisch bei Reaktionstemperatur noch einen gewissen Zeitraum, z. B. 10 Minuten bis 4 Stunden nachreagieren.

Vorzugsweise führt man die Reduktion von V nach II mit Eisenpulver in verdünnter Säure durch. Geeignete Säuren sind Mineralsäuren wie Salzsäure oder organische Säuren wie Ameisensäure, Essigsäure, Propionsäure, Buttersäure. Vorzugsweise verwendet man Essigsäure. Die Menge an Eisenpulver beträgt vorzugsweise 2 bis 5 Mol, insbesondere 2,5 bis 4 Mol, pro Mol der Verbindung V. Die Menge an Säure ist in der Regel nicht kritisch. Zweckmäßigerweise verwendet man mindestens eine äquimolare Menge an Säure, bezogen auf die Nitroverbindung V, um die Ausgangsverbindung möglichst vollständig zu reduzieren. Die Reaktion kann kontinuierlich oder diskontinuierlich durchgeführt werden. Die Reaktionstemperaturen liegen dann im Bereich von 50 bis 100 °C, vorzugsweise 65 bis 75 °C. In einer Ausführungsform legt man beispielsweise das Eisenpulver in Essigsäure vor und gibt dann die Verbindung V in das Reaktionsgefäß. Vorzugsweise erfolgt die Zugabe innerhalb 20 bis 60 Minuten unter Durchmischen der Bestandteile, z. B. unter Rühren. Nach beendeter Zugabe lässt man noch 0,5 bis 2 Stunden, vorzugsweise etwa 1 Stunde bei Reaktionstemperatur nachreagieren. Man kann jedoch auch das Eisenpulver unter Rühren zu der Mischung der Verbindung V in Eisessig geben und die Reaktion wie zuvor beschrieben zu Ende führen.

Die Aufarbeitung zur Gewinnung des Zielproduktes kann nach den hierfür üblichen Verfahren erfolgen. In der Regel wird man zunächst das Lösungsmittel entfernen, beispielsweise durch Destillation. Zur weiteren Reinigung kann man übliche Verfahren wie Kristallisation, Chromatographie, beispielsweise an Kieselgel, Verühren mit einem Lösungsmittel, beispielsweise aromatische Kohlenwasserstoffe wie Benzol, Toluol oder Xylol oder aliphatische Kohlenwasserstoffe wie Petrolether, Hexan, Cyclohexan, Pentan, Carbonsäureester wie Essigsäureethylester usw. und Gemische davon anwenden.

Als Reduktionsmittel kommen weiterhin auch Metallhydride und Halbmetallhydride wie Aluminiumhydrid und davon abgeleitete Hydride wie Lithiumaluminiumhydrid, Diisobutylaluminiumhydrid, Borhydride wie Diboran und davon abgeleitete Boranate wie Natriumborhydrid oder Lithiumboranat in Betracht. Hierzu bringt man die Nitroverbindung V in einem inerten Lösungsmittel mit dem komplexen Metallhydrid bei 10 bis 65°C, vorteilhaft 20 bis 50 °C in Kontakt. Vorzugsweise beträgt die Reaktionszeit 2 bis 10 Stunden, vorteilhaft 3 bis 6 Stunden. Vorzugsweise führt man die Umsetzung in einem gegenüber dem Reduktionsmittel inerten organischen Lösungsmittel durch. Als Lösungsmittel kommen - in Abhängigkeit vom gewählten Reduktionsmittel- z. B. Alkohole beispielsweise C₁-C₄-Alkohole wie Methanol, Ethanol, n-Propanol, Isopropanol oder n-Butanol, und deren Mischungen mit Wasser oder Ether wie Diisopropylether, Methyl-tert.-butylether, Ethylenglykoldimethylether, Dioxan oder Tetrahydrofuran ein Betracht.

In der Regel setzt man 0,5 bis 3, vorteilhaft 0,75 bis 2,5 mol Metallhydrid, Metallhalbhydrid, Borhydrid beziehungsweise Boranat pro mol Nitroverbindung V ein. Das Verfahren folgt der in Organikum, VEB Deutscher Verlag der Wissenschaften, Berlin 1976, 15. Auflage, S. 612-616 beschriebenen Verfahrensweise.

Ein weiteres geeignetes Reduktionsmittel für die Umwandlung der Verbindung V in die Verbindung II ist Wasserstoff in Gegenwart katalytischer Mengen an Übergangsmetallen oder Übergangsmetallverbindungen, insbesondere der 8. Nebengruppe. Bevorzugte Übergangsmetalle sind beispielsweise Nickel, Palladium, Platin, Ruthenium oder Rhodium. Die Übergangsmetalle können als solche oder in geträgerter Form eingesetzt werden. Beispiele für Träger sind Aktivkohle, Aluminiumoxid, ZrO₂, TiO₂, SiO₂, Carbonate und dergleichen. Die Übergangsmetalle können auch in Form aktivierter Metalle wie Raney-Nickel eingesetzt werden. Die Übergangsmetalle können auch in Form von Verbindungen eingesetzt werden. Geeignete Übergangsmetallverbindungen sind z. B. Palladiumoxid und Platinoxid. Die Katalysatoren werden im Allgemeinen in einer Menge von 0,05 bis 10,0 Mol-% (gerechnet als Metall), bezogen auf die zu reduzierende Verbindung V, eingesetzt. Man arbeitet entweder lösungsmittelfrei oder in einem inerten Lösungs- oder Verdünnungsmittel. Geeignete Lösungsmittel oder Verdünnungsmittel für die Reduktion sind je nach Löslichkeit des zu hydrierenden Substrates und dem gewählten Reduktionsmittel, z. B. Carbonsäuren wie Essigsäure, oder wässrige Lösungen organischer Säuren wie Essigsäure und Wasser, Carbonsäureester wie Essigsäureethylester, C₁-C₄-Alkohole wie Methanol, Ethanol, n-Propanol, Isopropanol, n-Butanol oder aromatische Kohlenwasserstoffe wie Toluol. Nach Abtrennen des Katalysators kann die Reaktionslösung wie üblich auf das Produkt hin aufgearbeitet werden. Die Hydrierung kann bei Normalwasserstoffdruck oder unter erhöhtem Wasserstoffdruck, beispielsweise bei einem Wasserstoffdruck von 0,01 bis 50 bar, vorzugsweise 0,1 bis 40 bar, durchgeführt werden. Zur katalytischen Hydrierung aromatischer Nitroverbindungen siehe beispielsweise Rylander in "Catalytic Hydrogenation over Platinum Metalls", Academic Press, New York, 1967, 168-202; Furst et al. Chem. Rev. 1965, 65, 52; Tepko et al., J. Org. Chem. 1980, 45, 4992.

Bei chlorhaltigen Benzoylsulfamiden hydriert man je nach Empfindlichkeit des Substituenten bei 20 bis 170 °C, zweckmäßig 20 bis 140 °C, vorteilhaft 20 bis 80 °C. Bei reaktiven Halogensubstituenten empfiehlt es sich ferner in neutraler Lösung, eventuell bei nur leicht erhöhtem Druck mit geringen Mengen an Nickel-, Platin- oder auch Rhodiumkatalysatoren zu hydrieren; geeignet sind auch Edelmetallsulfide wie Platinsulfid. Das Verfahren ist im Houben-Weyl "Methoden der organischen Chemie", Bd. IV/lC, S. 520-526 eingehend beschrieben.

Die Reduktion der Verbindung V zur Verbindung II kann auch mit Natriumsulfid, vorteilhaft in wässrig ammoniakalischer Lösung, in Gegenwart von Ammoniumchlorid gemäß dem in Org. Syn., Coll. Vol., 3, 82 (1955) beschriebenen Verfahren erfolgen. Die Reaktionstemperatur liegt in der Regel zwischen 40 bis 90 °C, vorzugsweise zwischen 60 bis 80°C. Zweckmäßig setzt man 3 bis 4 Mol Natriumsulfid pro Mol Nitroverbindung V ein.

Die in Schema 2 eingesetzten Aroylverbindungen III sind nach an sich im Stand der Technik bekannten Verfahren erhältlich oder lassen sich in Anlehnung an bekannte Verfahren herstellen, beispielsweise gemäß US 6,251,829, EP 415 641, EP 908 457, EP 1176133 und WO 01/087872.

Die Sulfamidsäureamide IV sind im Stand der Technik bekannt oder lassen sich nach bekannten Verfahren, z. B nach der deutschen Patentanmeldung DE 102 21 910.0 durch Umsetzung von Ammoniak mit Sulfamidsäurehalogeniden herstellen.

Vorzugsweise stellt man die Sulfamidsäureamide IV nach dem in der unveröffentlichten deutschen Patentanmeldung DE 102 21 910.0 beschriebenen Verfahren her. Dieses Verfahren umfasst die folgenden Schritte: (i) Umsetzung eines primären oder sekundären Amins mit wenigstens einer äquimolaren Menge SO₃ oder einer SO₃-Quelle in Gegenwart von wenigstens äquimolaren Mengen eines tertiären Amins, jeweils bezogen auf das primäre oder sekundäre Amin, wobei man ein Amidosulfonsäureammoniumsalz erhält; (ii) Umsetzung des Amidosulfonsäureammoniumsalzes mit wenigstens einer stöchiometrischen Menge eines Phosphorhalogenids, wobei man ein Sulfamidsäurehalogenid erhält und (iii) Umsetzung des in Schritt ii) erhaltenen Sulfamindsäurehalogenids mit Ammoniak, wobei man das Sulfamidsäureamid V erhält.

Das erfindungsgemäße Verfahren erlaubt erstmalig die Herstellung von Iso(thio)cyanatobenzoylsulfamidsäureamiden der allgemeinen Formel I. Die Verbindungen I sind neu und ebenfalls Gegenstand der vorliegenden Erfindung.

Unter den Iso(thio)cyanatobenzoylsulfamidsäureamiden der allgemeinen Formel I sind solche der Formel IA bevorzugt, worin die Variablen R^{a}, R^{b}, R^{c}, R^{d} die zuvor genannten Bedeutungen aufweisen.

Ganz besonders bevorzugt sind die Verbindungen der Formel IA.1, 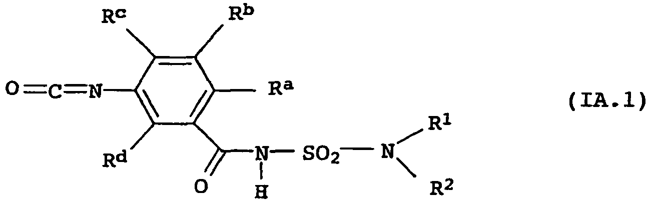 in denen die Variablen R¹, R², R^{a}, R^{b}, R^{c}, R^{d} die zuvor genannten Bedeutungen aufweisen.

Unter den Iso(thio)cyanatobenzoylsulfamidsäureamiden der allgemeinen Formel IA.1 sind insbesondere solche bevorzugt, in denen die Variablen R¹, R², R^{a}, R^{b}, R^{c}, R^{d} unabhängig voneinander, vorzugsweise jedoch in Kombination, die nachfolgend angegebenen Bedeutungen aufweisen:
- R^{a}: Cyano oder Halogen, insbesondere Cyano, Fluor oder Chlor;
- R^{b}: Wasserstoff;
- R^{c}: Wasserstoff oder Halogen, insbesondere Wasserstoff, Fluor oder Chlor;
- R^{d}: Wasserstoff;
- R¹ und R²: stehen unabhängig voneinander für Wasserstoff, C₁-C₆-Alkyl, das gegebenenfalls durch einen Substituenten ausgewählt unter Halogen, Cyano, C₁-C₄-Alkoxy, C₁-C₄-Alkoxycarbonyl, C₁-C₄-Alkylthio, C₃-C₈-Cycloalkyl, Furyl, Thienyl, 1,3-Dioxolanyl, Phenyl, das seinerseits gegebenenfalls durch Halogen oder C₁-C₄-Alkoxy substituiert ist, substituiert ist, C₂-C₆-Alkenyl, C₂-C₆-Alkinyl, C₃-C₈-Cycloalkyl oder Phenyl, das gegebenenfalls durch 1 oder 2 Substituenten, ausgewählt unter Halogen, C₁-C₄-Alkyl, C₁-C₄-Fluoralkyl, C₁-C₄-Alkoxy, C₁-C₄-Alkoxycarbonyl, Nitro oder C₁-C₃-Dialkylamino substituiert ist, Naphthtyl oder Pyridyl oder
- R¹ und R²: bilden zusammen einen fünf-, sechs- oder siebengliedrigen gesättigten oder ungesättigten Stickstoffheterocyclus, der gegebenenfalls ein weiteres Heteroatom, ausgewählt unter N, einer Gruppe NR⁶ (worin R⁶ die zuvor genannten Bedeutungen aufweist) und O, als Ringglied enthalten kann, und/oder durch ein, zwei oder drei Substituenten, ausgewählt unter C₁-C₄-Alkyl und C₁-C₄-Halogenalkyl, substituiert sein kann. Insbesondere steht einer der Reste R¹ oder R² für Wasserstoff, C₁-C₆-Alkyl, C₂-C₆-Alkenyl oder C₂-C₆-Alkinyl und der andere Rest R¹ oder R² für C₁-C₆-Alkyl, C₃-C₈-Cycloalkyl oder Phenyl.

Ganz besonders bevorzugt sind die Isocyanatobenzoylsulfamidsäureamide der Formel IA.1-a (≡ I mit W = Sauerstoff, Ar = Ar-1 mit R^{a} = Cl und R^{b} = R^{d} = Wasserstoff und R^{c} = F, A = NR¹R²), worin R¹, R² die oben genannten, insbesondere die als bevorzugt genannten Bedeutungen, aufweisen. Beispiele für derartige Verbindungen sind die Verbindungen IA.1-a.1 bis IA.1-a.495, in denen die Variablen R¹, R² gemeinsam die in einer Zeile der Tabelle 1 angegebenen Bedeutungen aufweisen.

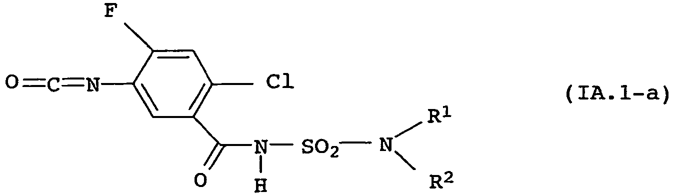

**Tabelle 1:**

| Nr. | R¹ | R² |
|---|---|---|
| 1 | H | CH3 |
| 2 | H | C₂H₅ |
| 3 | H | CH₂CH₂-Cl |
| 4 | H | CH₂CH₂-CN |
| 5 | H | CH₂-CO-OCH₃ |
| 6 | H | CH₂-CO-OC₂H₅ |
| 7 | H | CH(CH₃)-CO-OCH₃ |
| 8 | H | CH₂CH₂-OCH₃ |
| 9 | H | CH₂-C₂H₅ |
| 10 | H | CH₂CH₂-C₂H₅ |
| 11 | H | CH(CH₃)₂ |
| 12 | H | CH(CH₃)-C₂H₅ |
| 13 | H | CH₂-CH(CH₃)₂ |
| 14 | H | C(CH₃)₃ |
| 15 | H | CH(CH₃)-CH₂-C₂H₅ |
| 16 | H | CH₂-CH (CH₃)-C₂H₅ |
| 17 | H | CH₂CH₂-CH(CH₃)₂ |
| 18 | H | CH₂-CH=CH₂ |
| 19 | H | CH(CH₃)=CH₂ |
| 20 | H | CH₂=CH-CH₃ |
| 21 | H | CH₂-C≡CH |
| 22 | H | CH (CH₃)-C≡CH |
| 23 | H | Cyclopropyl |
| 24 | H | CH₂-Cyclopropyl |
| 25 | H | Cyclopentyl |
| 26 | H | CH₂-Cyclopentyl |
| 27 | H | CH₂-(1,3-Dioxolan-2-yl) |
| 28 | H | CH₂-(2-Furyl) |
| 29 | H | CH₂-(3-Furyl) |
| 30 | H | CH₂-(2-Thienyl) |
| 31 | H | CH₂-(3-Thienyl) |
| 32 | H | Phenyl |
| 33 | H | 2-Chlorphenyl |
| 34 | H | 3-Chlorphenyl |
| 35 | H | 4-Chlorphenyl |
| 36 | H | 2-Fluorphenyl |
| 37 | H | 3-Fluorphenyl |
| 38 | H | 4-Fluorphenyl |
| 39 | H | 2-Methylphenyl |
| 40 | H | 3-Methylphenyl |
| 41 | H | 4-Methylphenyl |
| 42 | H | 2-Methoxyphenyl |
| 43 | H | 3-Methoxyphenyl |
| 44 | H | 4-Methoxyphenyl |
| 45 | H. | 2-(Methoxycarbonyl)phenyl |
| 46 | H | 3-(Methoxycarbonyl)phenyl |
| 47 | H | 4-(Methoxycarbonyl)phenyl |
| 48 | H | 2-Nitrophenyl |
| 49 | H | 3-Nitrophenyl |
| 50 | H | 4-Nitrophenyl |
| 51 | H | 2-(Dimethylamino)phenyl |
| 52 | H | 3-(Dimethylamino)phenyl |
| 53 | H | 4-(Dimethylamino)phenyl |
| 54 | H | 2-(Trifluormethyl)phenyl |
| 55 | H | 3-(Trifluormethyl)phenyl |
| 56 | H | 4-(Trifluormethyl)phenyl |
| 57 | H | 3-(Phenoxy)phenyl |
| 58 | H | 4-(Phenoxy)phenyl |
| 59 | H | 2,4-Difluorphenyl |
| 60 | H | 2,4-Dichlorphenyl |
| 61 | H | 3,4-Difluorphenyl |
| 62 | H | 3,4-Dichlorphenyl |
| 63 | H | 3,5-Difluorphenyl |
| 64 | H | 3,5-Dichlorphenyl |
| 65 | H | 2-Pyridyl |
| 66 | H | 3-Pyridyl |
| 67 | H | 4-Pyridyl |
| 68 | H | α-Naphthyl |
| 69 | H | Benzyl |
| 70 | H | 2-Chlorbenzyl |
| 71 | H | 3-Chlorbenzyl |
| 72 | H | 4-Chlorbenzyl |
| 73 | H | 2-Methoxybenzyl |
| 74 | H | 3-Methoxybenzyl |
| 75 | H | 4-Methoxybenzyl |
| 76 | CH₃ | CH₃ |
| 77 | CH3 | C₂H₅ |
| 78 | CH₃ | CH₂CH₂-Cl |
| 79 | CH₃ | CH₂CH₂-CN |
| 80 | CH₃ | CH₂-CO-OCH₃ |
| 81 | CH₃ | CH₂-CO-0C₂H₅ |
| 82 | CH₃ | CH(CH₃)-CO-OCH₃ |
| 83 | CH₃ | CH₂CH₂-OCH₃ |
| 84 | CH₃ | CH₂-C₂H₅ |
| 85 | CH₃ | CH₂CH₂-C₂H₅ |
| 86 | CH₃ | CH(CH₃)₂ |
| 87 | CH₃ | CH(CH₃)-C₂H₅ |
| 88 | CH₃ | CH₂-CH(CH₃)₂ |
| 89 | CH₃ | C(CH₃)₃ |
| 90 | CH₃ | CH(CH₃)-CH₂-C₂H₅ |
| 91 | CH₃ | CH₂-CH(CH₃)-C₂H₅ |
| 92 | CH₃ | CH₂CH₂-CH(CH₃)₂ |
| 93 | CH₃ | CH₂-CH=CH₂ |
| 94 | CH₃ | CH(CH₃)=CH₂ |
| 95 | CH₃ | CH₂=CH-CH₃ |
| 96 | CH₃ | CH₂-C≡CH |
| 97 | CH₃ | CH(CH₃)-C≡CH |
| 98 | CH₃ | Cyclopropyl |
| 99 | CH₃ | CH₂-Cyclopropyl |
| 100 | CH₃ | Cyclopentyl |
| 101 | CH₃ | CH₂-Cyclopentyl |
| 102 | CH₃ | CH₂-(1,3-Dioxolan-2-yl) |
| 103 | CH₃ | CH₂-(2-Furyl) |
| 104 | CH₃ | CH₂-(3-Furyl) |
| 105 | CH₃ | CH₂-(2-Thienyl) |
| 106 | CH₃ | CH₂-(3-Thienyl) |
| 107 | CH₃ | Phenyl |
| 108 | CH₃ | 2-Chlorphenyl |
| 109 | CH₃ | 3-Chlorphenyl |
| 110 | CH₃ | 4-Chlorphenyl |
| 111 | CH₃ | 2-Fluorphenyl |
| 112 | CH₃ | 3-Fluorphenyl |
| 113 | CH₃ | 4-Fluorphenyl |
| 114 | CH₃ | 2-Methylphenyl |
| 115 | CH₃ | 3-Methylphenyl |
| 116 | CH₃ | 4-Methylphenyl |
| 117 | CH₃ | 2-Methoxyphenyl |
| 118 | CH₃ | 3-Methoxyphenyl |
| 119 | CH₃ | 4-Methoxyphenyl |
| 120 | CH₃ | 2-(Methoxycarbonyl)phenyl |
| 121 | CH₃ | 3-(Methoxycarbonyl)phenyl |
| 122 | CH₃ | 4-(Methoxycarbonyl)phenyl |
| 123 | CH₃ | 2-Nitrophenyl |
| 124 | CH₃ | 3-nitrophenyl |
| 125 | CH₃ | 4-Nitrophenyl |
| 126 | CH₃ | 2-(Dimethylamino)phenyl |
| 127 | CH₃ | 3-(Dimethylamino)phenyl |
| 128 | CH₃ | 4-(Dimethylamino)phenyl |
| 129 | CH₃ | 2-(Trifluormethyl)phenyl |
| 130 | CH₃ | 3-(Trifluormethyl)phenyl |
| 131 | CH₃ | 4-(Trifluormethyl)phenyl |
| 132 | CH₃ | 3-(Phenoxy)phenyl |
| 133 | CH₃ | 4-(Phenoxy)phenyl |
| 134 | CH₃ | 2,4-Difluorphenyl |
| 135 | CH₃ | 2,4-Dichlorphenyl |
| 136 | CH₃ | 2,4-Dichlorphenyl |
| 137 | CH₃ | 3,4-Dichlorphenyl |
| 138 | CH₃ | 3,5-Difluorphenyl |
| 139 | CH₃ | 3,5-Dichlorphenyl |
| 140 | CH₃ | 2-Pyridyl |
| 141 | CH₃ | 3-Pyridyl |
| 142 | CH₃ | 4-Pyridyl |
| 143 | CH₃ | α-Naphthyl |
| 144 | CH₃ | Benzyl |
| 145 | CH₃ | 2-Chlorbenzyl |
| 146 | CH₃ | 3-chlorbenzoyl |
| 147 | CH₃ | 4-Chlorbenzyl |
| 148 | CH₃ | 2-Methoxybenzyl |
| 149 | CH₃ | 3-Methoxybenzyl |
| 150 | CH₃ | 4-Methoxybenzyl |
| 151 | C₂H₅ | C₂H₅ |
| 152 | C₂H₅ | CH₂CH₂-Cl |
| 153 | C₂H₅ | CH₂CH₂-CN |
| 154 | C₂H₅ | CH₂-CO-OCH₃ |
| 155 | C₂H₅ | CH₂-CO-OC₂H₅. |
| 156 | C₂H₅ | CH(CH₃)-CO-OCH₃ |
| 157 | C₂H₅ | CH₂CH₂-OCH₃ |
| 158 | C₂H₅ | CH₂-C₂B₅ |
| 159 | C₂H₅ | CH₂CH₂-C₂H₅ |
| 160 | C₂H₅ | CH(CH₃)₂ |
| 161 | C₂H₅ | CH(CH₃)-C₂H₅ |
| 162 | C₂H₅ | CH₂-CH(CH₃)₂ |
| 163 | C₂H₅ | C(CH₃)₃ |
| 164 | C₂H₅ | CH(CH₃)-CH₂-C₂H₅ |
| 165 | C₂H₅ | CH₂-CH(CH₃)-C₂H₅ |
| 166 | C₂H₅ | CH₂CH₂-CH(CH₃)₂ |
| 167 | C₂H₅ | CH₂-CH=CH₂ |
| 168 | C₂H₅ | CH(CH₃)=CH₂ |
| 169 | C₂H₅ | CH₂=CH-CH₃ |
| 170 | C₂H₅ | CH₂-C≡CH |
| 171 | C₂H₅ | CH(CH₃)-C≡CH |
| 172 | C₂H₅ | Cyclopropyl |
| 173 | C₂H₅ | CH₂-Cyclopropyl |
| 174 | C₂H₅ | Cyclopentyl |
| 175 | C₂H₅ | CH₂-Cyclopentyl |
| 176 | C₂H₅ | CH₂-(1,3-Dioxolan-2-yl) |
| 177 | C₂H₅ | CH₂-(2-Furyl) |
| 178 | C₂H₅ | CH₂-(3-Furyl) |
| 179 | C₂H₅ | CH₂-(2-Thienyl) |
| 180 | C₂H₅ | CH₂-(3-Thienyl) |
| 181 | C₂H₅ | Phenyl |
| 182 | C₂H₅ | 2-Chlorphenyl |
| 183 | C₂H₅ | 3-Chlorphenyl |
| 184 | C₂H₅ | 4-Chlorphenyl |
| 185 | C₂H₅ | 2-Fluorphenyl |
| 186 | C₂H₅ | 3-Fluorphenyl |
| 187 | C₂H₅ | 4-Fluorphenyl |
| 188 | C₂H₅ | 2-Methylphenyl |
| 189 | C₂H₅ | 3-Methylphenyl |
| 190 | C₂H₅ | 4-Methylphenyl |
| 191 | C₂H₅ | 2-Methoxyphenyl |
| 192 | C₂H₅ | 3-Methoxyphenyl |
| 193 | C₂H₅ | 4-Methoxyphenyl |
| 194 | C₂H₅ | 2-(Methoxycarbonyl)phenyl |
| 195 | C₂H₅ | 3-(Methoxycarbonyl)phenyl |
| 196 | C₂H₅ | 4-(Methoxycarbonyl)phenyl |
| 197 | C₂H₅ | 2-Nitrophenyl |
| 198 | C₂H₅ | 3-Nitrophenyl |
| 199 | C₂H₅ | 4-Nitrophenyl |
| 200 | C₂H₅ | 2-(Dimethylamino)phenyl |
| 201 | C₂H₅ | 3-(Dimethylamino)phenyl |
| 202 | C₂H₅ | 4-(Dimethylamino)phenyl |
| 203 | C₂H₅ | 2-(Trifluormethyl)phenyl |
| 204 | C₂H₅ | 3-(Trifluormethyl)phenyl |
| 205 | C₂H₅ | 4-(Trifluormethyl)phenyl |
| 206 | C₂H₅ | 3-(Phenoxy)phenyl |
| 207 | C₂H₅ | 4-(Phenoxy)phenyl |
| 208 | C₂H₅ | 2,4-Difluorphenyl |
| 209 | C₂H₅ | 2,4-Dichlorphenyl |
| 210 | C₂H₅ | 3,4-Difluorphenyl |
| 211 | C₂H₅ | 3,4-Dichlorphenyl |
| 212 | C₂H₅ | 3,5-Difluorphenyl |
| 213 | C₂H₅ | 3,5-Dichlorphenyl |
| 214 | C₂H₅ | 2-Pyridyl |
| 215 | C₂H₅ | 3-Pyridyl |
| 216 | C₂H₅ | 4-Pyridyl |
| 217 | C₂H₅ | α-Naphthyl |
| 218 | C₂H₅ | Benzyl |
| 219 | C₂H₅ | 2-Chlorbenzyl |
| 220 | C₂H₅ | 3-Chlorbenzyl |
| 221 | C₂H₅ | 4-Chlorbenzyl |
| 222 | C₂H₅ | 2-Methoxybenzyl |
| 223 | C₂H₅ | 3-Methoxybenzyl |
| 224 | C₂H₅ | 4-Methoxybenzyl |
| 225 | CH₂-C₂H₅ | C₂H₅ |
| 226 | CH₂-C₂H₅ | CH₂CH₂-Cl |
| 227 | CH₂-C₂H₅ | CH₂CH₂-CN |
| 228 | CH₂-C₂H₅ | CH₂-CO-OCH₃ |
| 229 | CH₂-C₂H₅ | CH₂-CO-OC₂H₅ |
| 230 | CH₂-C₂H₅ | CH(CH₃)-CO-OCH₃ |
| 231 | CH₂-C₂H₅ | CH₂CH₂-OCH₃ |
| 232 | CH₂-C₂H₅ | CH₂-C₂H₅ |
| 233 | CH₂-C₂B₅ | CH₂CH₂-C₂H₅ |
| 234 | CH₂-C₂H₅ | CH(CH₃)₂ |
| 235 | CH₂-C₂H₅ | CH(CH₃)-C₂H₅ |
| 236 | CH₂-C₂H₅ | CH₂-CH(CH₃)₂ |
| 237 | CH₂-C₂H₅ | C(CH₃)₃ |
| 238 | CH₂-C₂H₅ | CH(CH₃)-CH₂-C₂H₅ |
| 239 | CH₂-C₂H₅ | CH₂-CH(CH₃)-C₂H₅ |
| 240 | CH₂-C₂H₅ | CH₂CH₂-CH(CH₃)₂ |
| 241 | CH₂-C₂H₅ | CH₂-CH=CH₂ |
| 242 | CH₂-C₂H₅ | CH(CH₃)=CH₂ |
| 243 | CH₂-C₂H₅ | CH₂=CH-CH₃ |
| 244 | CH₂-C₂H₅ | CH₂-C≡CH |
| 245 | CH₂-C₂H₅ | CH(CH₃)-C≡CH |
| 246 | CH₂-C₂H₅ | Cyclopropyl |
| 247 | CH₂-C₂H₅ | CH₂-Cyclopropyl |
| 248 | CH₂-C₂H₅ | Cyclopentyl |
| 249 | CH₂-C₂H₅ | CH₂-Cyclopentyl |
| 250 | CH₂-C₂H₅ | CH₂-(1,3-Dioxolan-2-yl) |
| 251 | CH₂-C₂H₅ | CH₂-(2-Furyl) |
| 252 | CH₂-C₂H₅ | CH₂-(3-Furyl) |
| 253 | -CH₂-C₂H₅ | CH₂-(2-Thienyl) |
| 254 | CH₂-C₂H₅ | CH₂-(3-Thienyl) |
| 255 | CH₂-C₂H₅ | Phenyl |
| 256 | CH₂-C₂H₅ | 2-chlorophenyl |
| 257 | CH₂-C₂H₅ | 3-Chlorphenyl |
| 258 | CH₂-C₂H₅ | 4-Chlorphenyl |
| 259 | CH₂-C₂H₅ | 2-Fluorphenyl |
| 260 | CH₂-C₂H₅ | 3-Fluorphenyl |
| 261 | CH₂-C₂H₅ | 4-Fluorphenyl |
| 262 | CH₂-C₂H₅ | 2-Methylphenyl |
| 263 | CH₂-C₂H₅ | 3-Methylphenyl |
| 264 | CH₂-C₂H₅ | 4-Methylphenyl |
| 265 | CH₂-C₂H₅ | 2-Methoxyphenyl |
| 266 | CH₂-C₂H₅ | 3-Methoxyphenyl |
| 267 | CH₂-C₂H₅ | 4-Methoxyphenyl |
| 268 | CH₂-C₂H₅ | 2-(Methoxycarbonyl)phenyl |
| 269 | CH₂-C₂H₅ | 3-(Methoxycarbonyl)phenyl |
| 270 | CH₂-C₂H₅ | 4-(Methoxycarbonyl)phenyl |
| 271 | CH₂-C₂H₅ | 2-Nitrophenyl |
| 272 | CH₂-C₂H₅ | 3-Nitrophenyl |
| 273 | CH₂-C₂H₅ | 4-Nitrophenyl |
| 274 | CH₂-C₂H₅ | 2-(Dimethylamino)phenyl |
| 275 | CH₂-C₂H₅ | 3-(Dimethylamino)phenyl |
| 276 | CH₂-C₂H₅ | 4-(Dimethylamino)phenyl |
| 277 | CH₂-C₂H₅ | 2-(Trifluormethyl)phenyl |
| 278 | CH₂-C₂H₅ | 3-(Trifluormethyl)phenyl |
| 279 | CH₂-C₂H₅ | 4-(Trifluormethyl)phenyl |
| 280 | CH₂-C₂H₅ | 3-(Phenoxy)phenyl |
| 281 | CH₂-C₂H₅ | 4-(Phenoxy)phenyl |
| 282 | CH₂-C₂H₅ | 2,4-Difluorphenyl |
| 283 | CH₂-C₂H₅ | 2,4-Dichlorphenyl |
| 284 | CH₂-C₂H₅ | 3,4-Difluorphenyl |
| 285 | CH₂-C₂H₅ | 3,4-Dichlorphenyl |
| 286 | CH₂-C₂H₅ | 3,5-Difluorphenyl |
| 287 | CH₂-C₂H₅ | 3,5-Dichlorphenyl |
| 288 | CH₂-C₂H₅ | 2-Pyridyl |
| 289 | CH₂-C₂H₅ | 3-Pyridyl |
| 290 | CH₂-C₂H₅ | 4-Pyridyl |
| 291 | CH₂-C₂H₅ | α-Naphthyl |
| 292 | CH₂-C₂H₅ | Benzyl |
| 293 | CH₂-C₂H₅ | 2-Chlorbenzyl |
| 294 | CH₂-C₂H₅ | 3-Chlorbenzyl |
| 295 | CH₂-C₂H₅ | 4-Chlorbenzyl |
| 296 | CH₂-C₂H₅ | 2-Methoxybenzyl |
| 297 | CH₂-C₂H₅ | 3-Methoxybenzyl |
| 298 | CH₂-C₂H₅ | 4-Methoxybenzyl |
| 299 | CH₂-CH₂-C₂H₅ | CH₂CH₂-Cl |
| 300 | CH₂-CH₂-C₂H₅ | CH₂CH₂-CN |
| 301 | CH₂-CH₂-C₂H₅ | CH₂-CO-OCH₃ |
| 302 | CH₂-CH₂-C₂H₅ | CH₂-CO-OC₂H₅ |
| 303 | CH₂-CH₂-C₂H₅ | CH(CH₃)-CO-OCH₃ |
| 304 | CH₂-CH₂-C₂H₅ | CH₂CH₂-OCH₃ |
| 305 | CH₂-CH₂-C₂H₅ | CH₂CH₂-C₂H₅ |
| 306 | CH₂-CH₂-C₂H₅ | CH(CH₃)₂ |
| 307 | CH₂-CH₂-C₂H₅ | CH(CH₃)-C₂H₅ |
| 308 | CH₂-CH₂-C₂H₅ | CH₂-CH(CH₃)₂ |
| 309 | CH₂-CH₂-C₂H₅ | C(CH₃)₃ |
| 310 | CH₂-CH₂-C₂H₅ | CH(CH₃)-CH₂-C₂H₅ |
| 311 | CH₂-CH₂-C₂H₅ | CH₂-CH(CH₃)-C₂H₅ |
| 312 | CH₂-CH₂-C₂H₅ | CH₂CH₂-CH(CH₃)₂ |
| 313 | CH₂-CH₂-C₂H₅ | CH₂-CH=CH₂ |
| 314 | CH₂-CH₂-C₂H₅ | CH(CH₃)=CH₂ |
| 315 | CH₂-CH₂-C₂H₅ | CH₂=CH-CH₃ |
| 316 | CH₂-CH₂-C₂H₅ | CH₂-C≡CH |
| 317 | CH₂-CH₂-C₂H₅ | CH(CH₃)-C≡CH |
| 318 | CH₂-CH₂-C₂H₅ | Cyclopropyl |
| 319 | CH₂-CH₂-C₂H₅ | CH₂-Cyclopropyl |
| 320 | CH₂-CH₂-C₂H₅ | Cyclopentyl |
| 321 | CH₂-CH₂-C₂H₅ | CH₂-Cyclopentyl |
| 322 | CH₂-CH₂-C₂H₅ | CH₂-(1,3-Dioxolan-2-yl) |
| 323 | CH₂-CH₂-C₂H₅ | CH₂-(2-Furyl) |
| 324 | CH₂-CH₂-C₂H₅ | CH₂-(3-Furyl) |
| 325 | CH₂-CH₂-C₂H₅ | CH₂-(2-Thienyl) |
| 326 | CH₂-CH₂-C₂H₅ | CH₂-(3-Thienyl) |
| 327 | CH₂-CH₂-C₂H₅ | Phenyl |
| 328 | CH₂-CH₂-C₂H₅ | 2-Chlorphenyl |
| 329 | CH₂-CH₂-C₂H₅ | 3-Chlorphenyl |
| 330 | CH₂-CH₂-C₂H₅ | 4-Chlorphenyl |
| 331 | CH₂-CH₂-C₂H₅ | 2-Fluorphenyl |
| 332 | CH₂-CH₂-C₂H₅ | 3-Fluorphenyl |
| 333 | CH₂-CH₂-C₂H₅ | 4-Fluorphenyl |
| 334 | CH₂-CH₂-C₂H₅ | 2-Methylphenyl |
| 335 | CH₂-CH₂-C₂H₅ | 3-Methylphenyl |
| 336 | CH₂-CH₂-C₂H₅ | 4-Methylphenyl |
| 337 | CH₂-CH₂-C₂H₅ | 2-Methoxyphenyl |
| 338 | CH₂-CH₂-C₂H₅ | 3-Methoxyphenyl |
| 339 | CH₂-CH₂-C₂H₅ | 4-Methoxyphenyl |
| 340 | CH₂-CH₂-C₂H₅ | 2-(Methoxycarbonyl)phenyl |
| 341 | CH₂-CH₂-C₂H₅ | 3-(Methoxycarbonyl)phenyl |
| 342 | CH₂-CH₂-C₂H₅ | 4-(Methoxycarbonyl)phenyl |
| 343 | CH₂-CH₂-C₂H₅ | 2-Nitrophenyl |
| 344 | CH₂-CH₂-C₂H₅ | 3-Nitrophenyl |
| 345 | CH₂-CH₂-C₂H₅ | 4-Nitrophenyl |
| 346 | CH₂-CH₂-C₂H₅ | 2-(Dimethylamino)phenyl |
| 347 | CH₂-CH₂-C₂H₅ | 3-(Dimethylamino)phenyl |
| 348 | CH₂-CH₂-C₂H₅ | 4-(Dimethylamino)phenyl |
| 349 | CH₂-CH₂-C₂H₅ | 2-(Trifluormethyl)phenyl |
| 350 | CH₂-CH₂-C₂H₅ | 3-(Trifluormethyl)phenyl |
| 351 | CH₂-CH₂-C₂H₅ | 4-(Trifluormethyl)phenyl |
| 352 | CH₂-CH₂-C₂H₅ | 3-(Phenoxy)phenyl |
| 353 | CH₂-CH₂-C₂H₅ | 4-(Phenoxy)phenyl |
| 354 | CH₂-CH₂-C₂H₅ | 2,4-Difluorphenyl |
| 355 | CH₂-CH₂-C₂H₅ | 2,4-Dichlorphenyl |
| 356 | CH₂-CH₂-C₂H₅ | 3,4-Difluorphenyl |
| 357 | CH₂-CH₂-C₂H₅ | 3,4-Dichlorphenyl |
| 358 | CH₂-CH₂-C₂H₅ | 3,5-Difluorphenyl |
| 359 | CH₂-CH₂-C₂H₅ | 3,5-Dichlorphenyl |
| 360 | CH₂-CH₂-C₂H₅ | 2-Pyridyl |
| 361 | CH₂-CH₂-C₂H₅ | 3-Pyridyl |
| 362 | CH₂-CH₂-C₂H₅ | 4-Pyridyl |
| 363 | CH₂-CH₂-C₂H₅ | α-Naphthyl |
| 364 | CH₂-CH₂-C₂H₅ | Benzyl |
| 365 | CH₂-CH₂-C₂H₅ | 2-Chlorbenzyl |
| 366 | CH₂-CH₂-C₂H₅ | 3-Chlorbenzyl |
| 367 | CH₂-CH₂-C₂H₅ | 4-Chlorbenzyl |
| 368 | CH₂-CH₂-C₂H₅ | 2-Methoxybenzyl |
| 369 | CH₂-CH₂-C₂H₅ | 3-Methoxybenzyl |
| 370 | CH₂-CH₂-C₂H₅ | 4-Methoxybenzyl |
| 371 | CH(CH₃)₂ | CH₂CH₂-Cl |
| 372 | CH(CH₃)₂ | CH₂CH₂-CN |
| 373 | CH(CH₃)₂ | CH₂-CO-OCH₃ |
| 374 | CH(CH₃)₂ | CH₂-CO-OC₂H₅ |
| 375 | CH(CH₃)₂ | CH(CH₃)-CO-OCH₃ |
| 376 | CH(CH₃)₂ | CH₂CH₂-OCH₃ |
| 377 | CH(CH₃)₂ | CH(CH₃)₂ |
| 378 | CH(CH₃)₂ | CH(CH₃)-C₂H₅ |
| 379 | CH(CH₃)₂ | CH₂-CH(CH₃)₂ |
| 380 | CH(CH₃)₂ | C(CH₃)₃ |
| 381 | CH(CH₃)₂ | CH(CH₃)-CH₂-C₂H₅ |
| 382 | CH(CH₃)₂ | CH₂-CH(CH₃)-C₂H₅ |
| 383 | CH(CH₃)₂ | CH₂CH₂-CH(CH₃)₂ |
| 384 | CH(CH₃)₂ | CH₂-CH=CH₂ |
| 385 | CH(CH₃)₂ | CH(CH₃)=CH₂ |
| 386 | CH(CH₃)₂ | CH₂=CH-CH₃ |
| 387 | CH(CH₃)₂ | CH₂-C≡CH |
| 388 | CH(CH₃)₂ | CH(CH₃)-C≡CH |
| 389 | CH(CH₃)₂ | Cyclopropyl |
| 390 | CH(CH₃)₂ | CH₂-Cyclopropyl |
| 391 | CH(CH₃)₂ | Cyclopentyl |
| 392 | CH(CH₃)₂ | CH₂-Cyclopentyl |
| 393 | CH(CH₃)₂ | CH₂-(1,3-Dioxolan-2-yl) |
| 394 | CH(CH₃)₂ | CH₂-(2-Furyl) |
| 395 | CH(CH₃)₂ | CH₂-(3-Furyl) |
| 396 | CH(CH₃)₂ | CH₂-(2-Thienyl) |
| 397 | CH(CH₃)₂ | CH₂-(3-Thienyl) |
| 398 | CH(CH₃)₂ | Phenyl |
| 399 | CH(CH₃)₂ | 2-Chlorphenyl |
| 400 | CH(CH₃)₂ | 3-Chlorphenyl |
| 401 | CH(CH₃)₂ | 4-Chlorphenyl |
| 402 | CH(CH₃)₂ | 2-Fluorphenyl |
| 403 | CH(CH₃)₂ | 3-Fluorphenyl |
| 404 | CH(CH₃)₂ | 4-Fluorphenyl |
| 405 | CH(CH₃)₂ | 2-Methylphenyl |
| 406 | CH(CH₃)₂ | 3-Methylphenyl |
| 407 | CH(CH₃)₂ | 4-Methylphenyl |
| 408 | CH(CH₃)₂ | 2-Methoxyphenyl |
| 409 | CH(CH₃)₂ | 3-Methoxyphenyl |
| 410 | CH(CH₃)₂ | 4-Methoxyphenyl |
| 411 | CH(CH₃)₂ | 2-(Methoxycarbonyl)phenyl |
| 412 | CH(CH₃)₂ | 3-(Methoxycarbonyl)phenyl |
| 413 | CH(CH₃)₂ | 4-(Methoxycarbonyl)phenyl |
| 414 | CH(CH₃)₂ | 2-Nitrophenyl |
| 415 | CH(CH₃)₂ | 3-Nitrophenyl |
| 416 | CH(CH₃)₂ | 4-Nitrophenyl |
| 417 | CH(CH₃)₂ | 2-(Dimethylamino)phenyl |
| 418 | CH(CH₃)₂ | 3-(Dimethylamino)phenyl |
| 419 | CH(CH₃)₂ | 4-(Dimethylamino)phenyl |
| 420 | CH(CH₃)₂ | 2-(Trifluormethyl)phenyl |
| 421 | CH(CH₃)₂ | 3-(Trifluormethyl)phenyl |
| 422 | CH(CH₃)₂ | 4-(Trifluormethyl)phenyl |
| 423 | CH(CH₃)₂ | 3-(Phenoxy)phenyl |
| 424 | CH(CH₃)₂ | 4-(Phenoxy)phenyl |
| 425 | CH(CH₃)₂ | 2,4-Difluorphenyl |
| 426 | CH(CH₃)₂ | 2,4-Dichlorphenyl |
| 427 | CH(CH₃)₂ | 3,4-Difluorphenyl |
| 428 | CH(CH₃)₂ | 3,4-Dichlorphenyl |
| 429 | CH(CH₃)₂ | 3,5-Difluorphenyl |
| 430 | CH(CH₃)₂ | 3,5-Dichlorphenyl |
| 431 | CH(CH₃)₂ | 2-Pyridyl |
| 432 | CH(CH₃)₂ | 3-Pyridyl |
| 433 | CH(CH₃)₂ | 4-Pyridyl |
| 434 | CH(CH₃)₂ | α-Naphthyl |
| 435 | CH(CH₃)₂ | Benzyl |
| 436 | CH(CH₃)₂ | 2-Chlorbenzyl |
| 437 | CH(CH₃)₂ | 3-Chlorbenzyl |
| 438 | CH(CH₃)₂ | 4-Chlorbenzyl |
| 439 | CH(CH₃)₂ | 2-Methoxybenzyl |
| 440 | CH(CH₃)₂ | 3-Methoxybenzyl |
| 441 | CH(CH₃)₂ | 4-Methoxybenzyl |
| 442 | -(CH₂)₄- | |
| 443 | -CH₂-CH=CH-CH₂- | |
| 444 | H | Cyclohexyl |
| 445 | CH3 | Cyclohexyl |
| 446 | C₂H₅ | Cyclohexyl |
| 447 | n-C₃H₇ | Cyclohexyl |
| 448 | i-C₃H₇ | Cyclohexyl |
| 449 | n-C₄H₉ | Cyclohexyl |
| 450 | i-C₄H₉ | Cyclohexyl |
| 451 | sek.-C₄H₉ | Cyclohexyl |
| 452 | tert.-C₄H₉ | Cyclohexyl |
| 453 | H | CH₂-CH=CH-CH₃ |
| 454 | CH₃ | CH₂-CH=CH-CH₃ |
| 455 | C₂H₅ | CH₂-CH=CH-CH₃ |
| 456 | n-C₃H₇ | CH₂-CH=CH-CH₃ |
| 457 | i-C₃H₇ | CH₂-CH=CH-CH₃ |
| 458 | n-C₄H₉ | CH₂-CH=CH-CH₃ |
| 459 | i-C₄H₉ | CH₂-CH=CH-CH₃ |
| 460 | sek.-C₄H₉ | CH₂-CH=CH-CH₃ |
| 461 | tert.-C₄H₉ | CH₂-CH=CH-CH₃ |
| 462 | H | CH₃S-CH₂CH₂ |
| 463 | CH₃ | CH₃S-CH₂CH₂ |
| 464 | C₂H₅ | CH₃S-CH₂CH₂ |
| 465 | n-C₃H₇ | CH₃S-CH₂CH₂ |
| 466 | i-C₃N₇ | CH₃S-CH₂CH₂ |
| 467 | n-C₄H₉ | CH₃S-CH₂CH₂ |
| 468 | i-C₄H₉ | CH₃S-CH₂CH₂ |
| 469 | sek.-C₄H₉ | CH₃S-CH₂CH₂ |
| 470 | tert.-C₄H₉ | CH₃S-CH₂CH₂ |
| 471 | H | C₂H₅-O-CH₂CH₂ |
| 472 | CH₃ | C₂H₅-O-CH₂CH₂ |
| 473 | C₂H₅ | C₂H₅-O-CH₂CH₂ |
| 474 | n-C₃H₇ | C₂H₅-O-CH₂CH₂ |
| 475 | i-C₃H₇ | C₂H₅-O-CH₂CH₂ |
| 476 | n-C₄H₉ | C₂H₅-O-CH₂CH₂ |
| 477 | i-C₄H₉ | C₂H₅-O-CH₂CH₂ |
| 478 | sek.-C₄H₉ | C₂H₅-O-CH₂CH₂ |
| 479 | tert.-C₄H₉ | C₂H₅-O-CH₂CH₂ |
| 480 | CH₂CH₂-O-CH₂CH₂ | |
| 481 | CH₂-CH=CH-CH₂ | |
| 482 | CH=CH-CH₂-CH₂ | |
| 483 | CH₂-CH₂-CH₂-CH₂-CH₂ | |
| 484 | CH₂-CH₂-O-CH(CH₃)-CH₂ | |
| 485 | CH₂-CH₂-O-CH₂-CH(CH₃) | |
| 486 | CH₂-CH₂-N(CH₃)-CH₂-CH₂ | |
| 487 | CH₂-CH(CH₃)-O-CH(CH₃)-CH₂ | |
| 488 | CH₂-CH=CH-CH₂-CH₂ | |
| 489 | CH=CH-CH₂-CH₂-CH₂ | |
| 490 | CH₂-CH₂-CH₂-CH₂-CH(CH₃) | |
| 491 | CH₂-CH₂-CH₂-CH(CH₃)-CH₂ | |
| 492 | CH₂-CH₂-CH(CH₃)-CH₂-CH₂ | |
| 493 | CH₂-CH₂-CH₂-CH₂-CH(CH₂CH₂Cl) | |
| 494 | CH₂-CH₂-CH₂-CH(CH₂CH₂Cl)-CH₂ | |
| 495 | CH₂-CH₂-CH(CH₂CH₂Cl)-CH₂-CH₂ | |

Ganz besonders bevorzugt sind die Isocyanatobenzoylsulfamidsäureamide der Formel IA.1-b (≡ I mit W = Sauerstoff, Ar = Ar-1 mit R^{a} = Cl und R^{b} = R^{d} = Wasserstoff und R^{c} = H, A = NR¹R²), worin R¹, R² die oben genannten, insbesondere die als bevorzugt genannten Bedeutungen, aufweisen. Beispiele für derartige Verbindungen sind die Verbindungen IA.1-b.1 bis IA.1-b.495, in denen die Variablen R¹, R² gemeinsam die in einer Zeile der Tabelle 1 angegebenen Bedeutungen aufweisen.

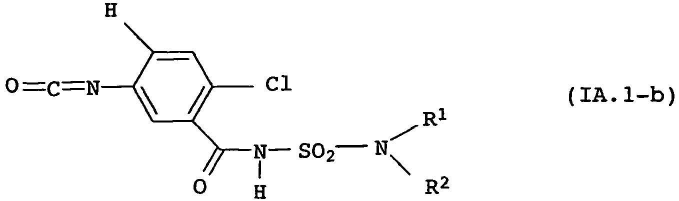

Ganz besonders bevorzugt sind die Isocyanatobenzoylsulfamidsäureamide der Formel IA.1-c (≡ I mit W = Sauerstoff, Ar = Ar-1 mit R^{a} = Cl und R^{b} = R^{d} = Wasserstoff und R^{c} = Cl, A = NR¹R²), worin R¹, R² die oben genannten, insbesondere die als bevorzugt genannten Bedeutungen, aufweisen. Beispiele für derartige Verbindungen sind die Verbindungen IA.1-c.1 bis IA.1-c.495, in denen die Variablen R¹, R² gemeinsam die in einer Zeile der Tabelle 1 angegebenen Bedeutungen aufweisen.

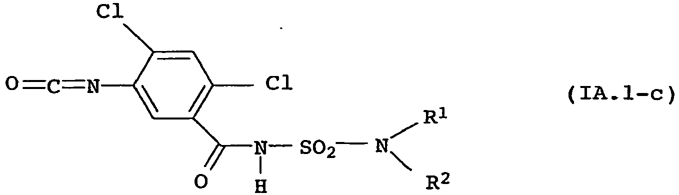

Ganz besonders bevorzugt sind die Isocyanatobenzoylsulfamidsäureamide der Formel IA.1-d (≡ I mit W = Sauerstoff, Ar = Ar-1 mit R^{a} = F und R^{b} = R^{d} = Wasserstoff und R^{c} = F, A = NR¹R²), worin R¹, R² die oben genannten, insbesondere die als bevorzugt genannten Bedeutungen, aufweisen. Beispiele für derartige Verbindungen sind die Verbindungen IA.1-d.1 bis IA.1-d.495, in denen die Variablen R¹, R² gemeinsam die in einer Zeile der Tabelle 1 angegebenen Bedeutungen aufweisen.

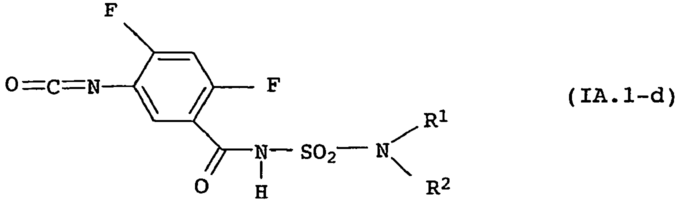

Ganz besonders bevorzugt sind die Isocyanatobenzoylsulfamidsäureamide der Formel IA.1-e (≡ I mit W = Sauerstoff, Ar = Ar-1 mit R^{a} = CN und R^{b} = R^{d} = Wasserstoff und R^{c} = F, A = NR¹R²), worin R¹, R² die oben genannten, insbesondere die als bevorzugt genannten Bedeutungen, aufweisen. Beispiele für derartige Verbindungen sind die Verbindungen IA.1-e.1 bis IA.1-e.495, in denen die Variablen R¹, R² gemeinsam die in einer Zeile der Tabelle 1 angegebenen Bedeutungen aufweisen.

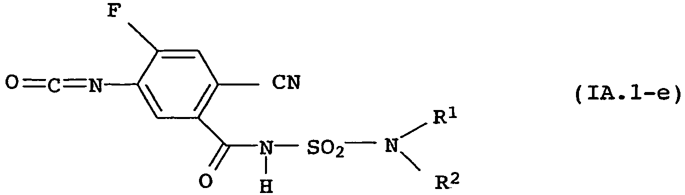

Ganz besonders bevorzugt sind die Isocyanatobenzoylsulfamidsäureamide der Formel IA.1-f (≡ I mit W = Sauerstoff, Ar = Ar-1 mit R^{a} = CN und R^{b} = R^{d} = Wasserstoff und R^{c} = Cl, A = NR¹R²), worin R¹, R² die oben genannten, insbesondere die als bevorzugt genannten Bedeutungen, aufweisen. Beispiele für derartige Verbindungen sind die Verbindungen IA.1-f.1 bis IA.1-f.495, in denen die Variablen R¹, R² gemeinsam die in einer Zeile der Tabelle 1 angegebenen Bedeutungen aufweisen.

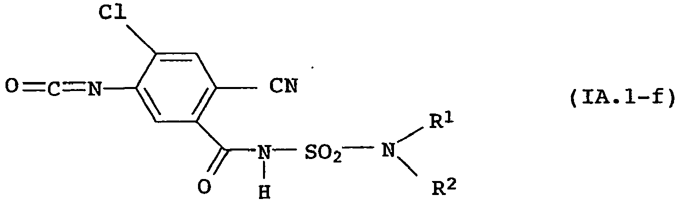

Ganz besonders bevorzugt sind die Isothiocyanatobenzoylsulfamidsäureamide der Formel IA.1-g (- I mit W = Schwefel, Ar = Ar-1 mit R^{a} = Cl und R^{b} = R^{d} = Wasserstoff und R^{c} = F, A = NR¹R²), worin R¹, R² die oben genannten, insbesondere die als bevorzugt genannten Bedeutungen, aufweisen. Beispiele für derartige Verbindungen sind die Verbindungen IA.1-g.1 bis IA.1-g.495, in denen die Variablen R¹, R² gemeinsam die in einer Zeile der Tabelle 1 angegebenen Bedeutungen aufweisen.

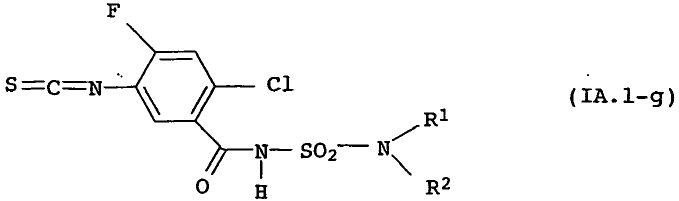

Ganz besonders bevorzugt sind die Isothiocyanatobenzoylsulfamidsäureamide der Formel IA.1-h (≡ I mit W = Schwefel, Ar = Ar-1 mit R^{a} = Cl und R^{b} = R^{d} = Wasserstoff und R^{c} = H, A = NR¹R²), worin R¹, R² die oben genannten, insbesondere die als bevorzugt genannten Bedeutungen, aufweisen. Beispiele für derartige Verbindungen sind die Verbindungen IA.1-h.1 bis IA.1-h.495, in denen die Variablen R¹, R² gemeinsam die in einer Zeile der Tabelle 1 angegebenen Bedeutungen aufweisen.

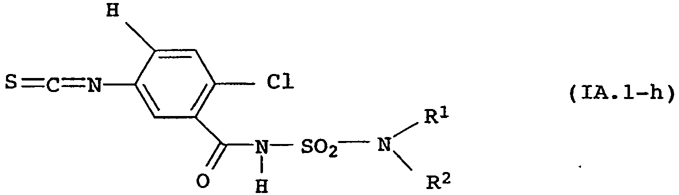

Ganz besonders bevorzugt sind die Isothiocyanatobenzoylsulfamidsäureamide der Formel IA.1-i (≡ I mit W = Schwefel, Ar = Ar-1 mit R^{a} = Cl und R^{b} = R^{d} = Wasserstoff und R^{c} = Cl, A = NR¹R²), worin R¹, R² die oben genannten, insbesondere die als bevorzugt genannten Bedeutungen, aufweisen. Beispiele für derartige Verbindungen sind die Verbindungen IA.1-i.1 bis IA.1-i.495, in denen die Variablen R¹, R² gemeinsam die in einer Zeile der Tabelle 1 angegebenen Bedeutungen aufweisen.

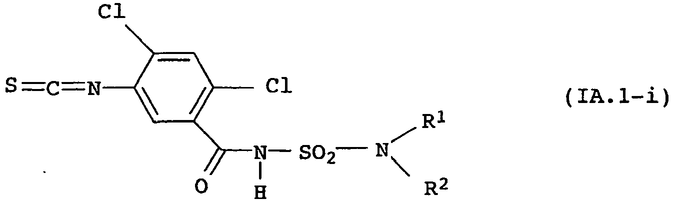

Ganz besonders bevorzugt sind die Isothiocyanatobenzoylsulfamidsäureamide der Formel IA.1-j (≡ I mit W = Schwefel, Ar = Ar-1 mit R^{a} = F und R^{b} = R^{d} = Wasserstoff und R^{c} = F, A = NR¹R²), worin R¹, R² die oben genannten, insbesondere die als bevorzugt genannten Bedeutungen, aufweisen. Beispiele für derartige Verbindungen sind die Verbindungen IA.1-j.1 bis IA.1-j.495, in denen die Variablen R¹, R² gemeinsam die in einer Zeile der Tabelle 1 angegebenen Bedeutungen aufweisen.

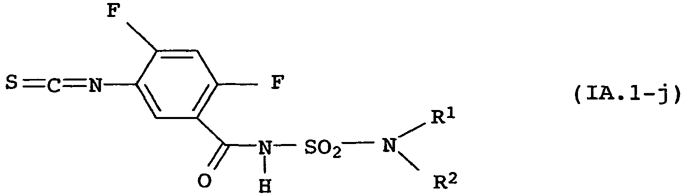

Ganz besonders bevorzugt sind die Isothiocyanatobenzoylsulfamidsäureamide der Formel IA.1-k (≡ I mit W = Schwefel, Ar = Ar-1 mit R^{a} = CN und R^{b} = R^{d} = Wasserstoff und R^{c} = F, A = NR¹R²), worin R¹, R² die oben genannten, insbesondere die als bevorzugt genannten Bedeutungen, aufweisen. Beispiele für derartige Verbindungen sind die Verbindungen IA.1-k.1 bis IA.1-k.495, in denen die Variablen R¹, R² gemeinsam die in einer Zeile der Tabelle 1 angegebenen Bedeutungen aufweisen.

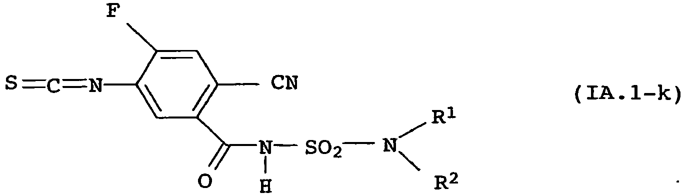

Ganz besonders bevorzugt sind die Isothiocyanatobenzoylsulfamidsäureamide der Formel IA.1-1 (≡ I mit W = Schwefel, Ar = Ar-1 mit R^{a} = CN und R^{b} = R^{d} = Wasserstoff und R^{c} = Cl, A = NR¹R²), worin R¹, R² die oben genannten, insbesondere die als bevorzugt genannten Bedeutungen, aufweisen. Beispiele für derartige Verbindungen sind die Verbindungen IA.1-1.1 bis IA.1-1.495, in denen die Variablen R¹, R² gemeinsam die in einer Zeile der Tabelle 1 angegebenen Bedeutungen aufweisen.

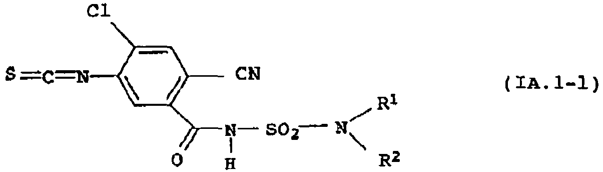

Im erfindungsgemäßen Verfahren werden als Edukt Aminobenzoylsulfamidsäureamide der allgemeinen Formel II eingesetzt. Diese sind ebenfalls neu und stellen wertvolle Zwischenprodukte zur Herstellung der Iso(thio)cyanatobenzoylsulfamidsäureamide I dar. Bezüglich des Herstellungsverfahrens wird auf das zuvor Gesagte verwiesen.

Bevorzugt eingesetzt werden insbesondere Verbindungen der Formel IIA (≡ II mit Ar = Ar-1), 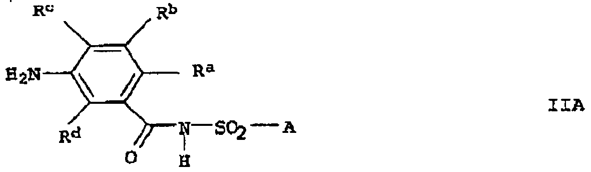 worin R^{a}, R^{b}, R^{c}, R^{d} und A die zuvor genannten Bedeutungen aufweisen- In der Formel IIA stehen R^{a}, R^{b}, R^{c}, R^{d} und A vorzugsweise für diejenigen Reste, die bereits im Zusammenhang mit der Besohreibung der erfindungsgemäßen Verbindung I als bevorzugt für diese variablen genannt wurden.

Besonders bevorzugt sind die Verbindungen der Formel IIA.1, 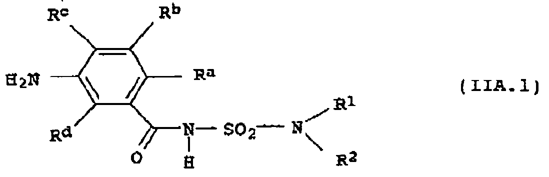 in denen die Variablen R¹, R², R^{a}, R^{b}, R^{c}, R^{d} die zuvor genannten Bedeutungen aufweisen. In Formel IIA.1 weisen die Variablen R¹ R², R^{a}, R^{b}, R^{c}, R^{d} vorzugsweise die Bedeutung auf, die bereits im Zusammenhang mit der Beschreibung der erfindungsgemäßen Verbindungen IA.1 als bevorzugt genannt wurden.

Ganz besonders bevorzugt sind die Aminobenzoylsulfamidsäureamide der Formel IIA.1-a (≡ II mit Ar = Ar-1 mit R^{a} = Cl und R^{b} = R^{d} = Wasserstoff und R^{c} = F, A = NR¹R²), worin R¹, R² die oben genannten, insbesondere die als bevorzugt genannten Bedeutungen, aufweisen. Beispiele für derartige Verbindungen sind die Verbindungen IIA.1-a.1 bis IIA.1-a.495, in denen die Variablen R¹, R² gemeinsam die in einer Zeile der Tabelle 1 angegebenen Bedeutungen aufweisen.

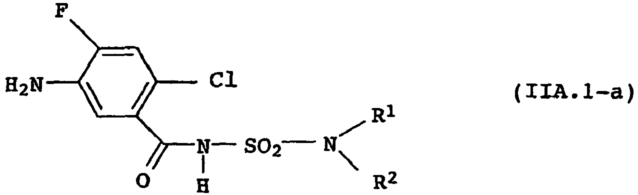

Ganz besonders bevorzugt sind die Aminobenzoylsulfamidsäureamide der Formel IIA.1-b (≡ II mit Ar = Ar-1 mit R^{a} = Cl und R^{b} = R^{d} = Wasserstoff und R^{c} = H, A = NR¹R²), worin R¹, R² die oben genannten, insbesondere die als bevorzugt genannten Bedeutungen, aufweisen. Beispiele für derartige Verbindungen sind die Verbindungen IIA.1-b.1 bis IIA.1-b.495, in denen die Variablen R¹, R² gemeinsam die in einer Zeile der Tabelle 1 angegebenen Bedeutungen aufweisen.

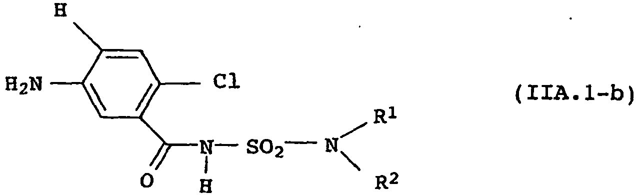

Ganz besonders bevorzugt sind die Aminobenzoylsulfamidsäureamide der Formel IIA.1-c (≡ II mit Ar = Ar-1 mit R^{a} = Cl und R^{b} = R^{d} = Wasserstoff und R^{c} = C1, A = NR¹R²), worin R¹, R² die oben genannten, insbesondere die als bevorzugt genannten Bedeutungen, aufweisen. Beispiele für derartige Verbindungen sind die Verbindungen IIA.1-c.1 bis IIA.1-c.495, in denen die Variablen R¹, R² gemeinsam die in einer Zeile der Tabelle 1 angegebenen Bedeutungen aufweisen.

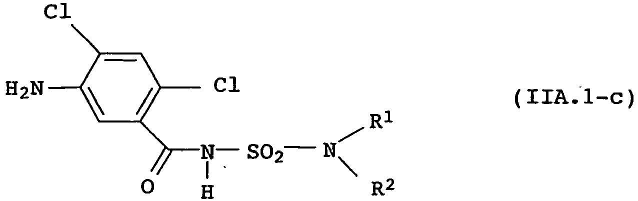

Ganz besonders bevorzugt sind die Aminobenzoylsulfamidsäureamide der Formel IIA.1-d (≡ II mit Ar = Ar-1 mit R^{a} = F und R^{b} = R^{d} = Wasserstoff und R^{c} = F, A = NR¹R²), worin R¹, R² die oben genannten, insbesondere die als bevorzugt genannten Bedeutungen, aufweisen. Beispiele für derartige Verbindungen sind die Verbindungen IIA.1-d.1 bis IIA.1-d.495, in denen die Variablen R¹, R² gemeinsam die in einer Zeile der Tabelle 1 angegebenen Bedeutungen aufweisen.

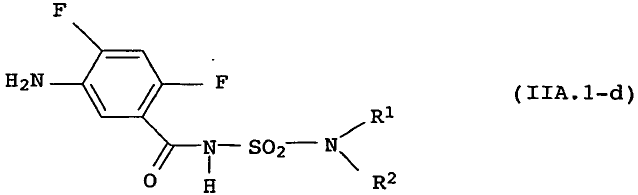

Ganz besonders bevorzugt sind die Aminobenzoylsulfamidsäureamide der Formel IIA.1-e (≡ II mit Ar = Ar-1 mit R^{a} = CN und R^{b} = R^{d} = Wasserstoff und R^{c} = F, A = NR¹R²), worin R¹, R² die oben genannten, insbesondere die als bevorzugt genannten Bedeutungen, aufweisen. Beispiele für derartige Verbindungen sind die Verbindungen IIA.1-e.1 bis IIA.1-e.495, in denen die Variablen R¹, R² gemeinsam die in einer Zeile der Tabelle 1 angegebenen Bedeutungen aufweisen.

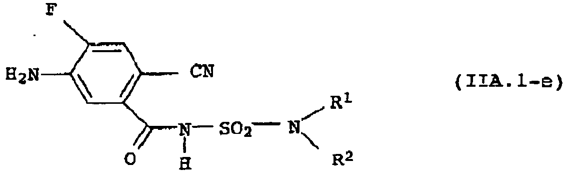

Ganz besonders bevorzugt sind die Aminobenzoylsulfamidsäureamida der Formel IIA.1-f (≡ II mit Ar - Ar-1 mit R^{a} - CN und R^{b} = R^{d} = Wasserstoff und R^{c} = Cl, A = NR¹R²), worin R¹, R² die oben genannten, insbesondere die als bevorzugt genannten Bedeutungen, aufweisen. Beispiele für derartige Verbindungen sind die Verbindungen IIA.1-f.1 bis IIA.1-f.495, in denen die Variablen R¹, R² gemeinsam die in einer zeile der Tabelle 1 angegebenen Bedeutungen aufweisen.

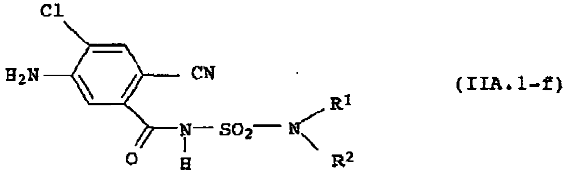

Die Nitrobenzoylsulfamidsäureamide der allgemeinen Formel V sind ebenfalls neu und stellen ebenfalls wertvolle Zwischenprodukte zur Herstellung der Iso(thio)cyanatobenzoylsulfamidsäureamide I dar.

Bevorzugt eingesetzt werden insbesondere Verbindungen der Formel VA (≡ V mit Ar = Ar-1), 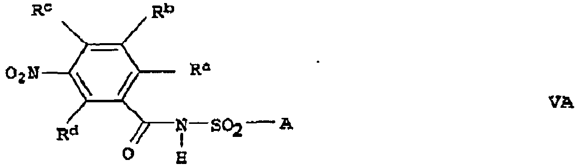 worin R^{a}, R^{b}, R^{c}, R^{d} und A die zuvor genannten Bedeutungen aufweisen. In der Formel VA stehen R^{a}, R^{b}, R^{c}, R^{d} und A vorzugsweise für diejenigen Reste, die bereits im Zusammenhang mit der Beschreibung der erfindungsgemäßen Verbindung I als bevorzugt für diese Variablen genannt wurden.

Ganz besonders bevorzugt sind die Verbindungen der Formel VA.1, 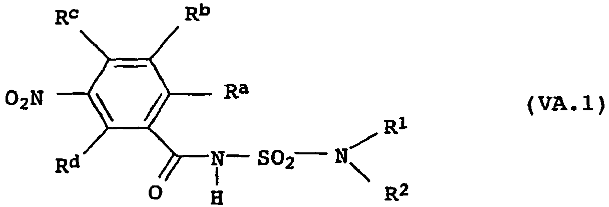 in denen die Variablen R¹, R², R^{a}, R^{b}, R^{c}, R^{d} die zuvor genannten Bedeutungen aufweisen. In Formel VA.1 weisen die Variablen R¹, R², R^{a}, R^{b}, R^{c}, R^{d} vorzugsweise die Bedeutung auf, die bereits im Zusammenhang mit der Beschreibung der erfindungsgemäßen Verbindungen IA.1 als bevorzugt genannt wurden.

Ganz besonders bevorzugt sind die Nitrobenzoylsulfamidsäureamide der Formel VA.1-a (≡ V mit Ar = Ar-1 mit R^{a} = Cl und R^{b} = R^{d} = Wasserstoff und R^{c} = F, A = NR¹R²), worin R¹, R² die oben genannten, insbesondere die als bevorzugt genannten Bedeutungen, aufweisen. Beispiele für derartige Verbindungen sind die Verbindungen VA.1-a.1 bis VA.1-a.495, in denen die Variablen R¹, R² gemeinsam die in einer Zeile der Tabelle 1 angegebenen Bedeutungen aufweisen.

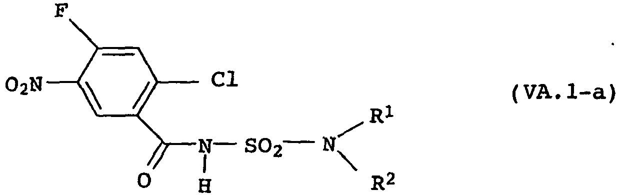

Ganz besonders bevorzugt sind die Nitrobenzoylsulfamidsäureamide der Formel VA.1-b (≡ V mit Ar = Ar-1 mit R^{a} = Cl und R^{b} = R^{d} = Wasserstoff und R^{c} = H, A = NR¹R²), worin R¹, R² die oben genannten, insbesondere die als bevorzugt genannten Bedeutungen, aufweisen. Beispiele für derartige Verbindungen sind die Verbindungen VA.1-b.1 bis VA.1-b.495, in denen die Variablen R¹, R² gemeinsam die in einer Zeile der Tabelle 1 angegebenen Bedeutungen aufweisen.

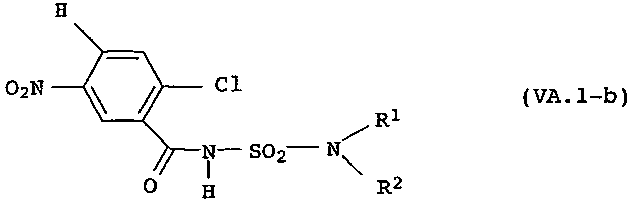

Ganz besonders bevorzugt sind die Nitrobenzoylsulfamidsäureamide der Formel VA.1-c (≡ V mit Ar = Ar-1 mit R^{a} = Cl und R^{b} = R^{d} = Wasserstoff und R^{c} = Cl, A = NR¹R²), worin R¹, R² die oben genannten, insbesondere die als bevorzugt genannten Bedeutungen, aufweisen. Beispiele für derartige Verbindungen sind die Verbindungen VA.1-c.1 bis VA.1-c.495, in denen die Variablen R¹, R² gemeinsam die in einer Zeile der Tabelle 1 angegebenen Bedeutungen aufweisen.

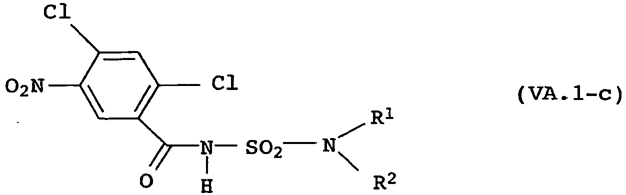

Ganz besonders bevorzugt sind die Nitrobenzoylsulfamidsäureamide der Formel VA.1-d (≡ V mit Ar = Ar-1 mit R^{a} = F und R^{b} = R^{d} = Wasserstoff und R^{c} = F, A = NR¹R²), worin R¹, R² die oben genannten, insbesondere die als bevorzugt genannten Bedeutungen, aufweisen. Beispiele für derartige Verbindungen sind die Verbindungen VA.1-d.1 bis VA.1-d.495, in denen die Variablen R¹, R² gemeinsam die in einer Zeile der Tabelle 1 angegebenen Bedeutungen aufweisen.

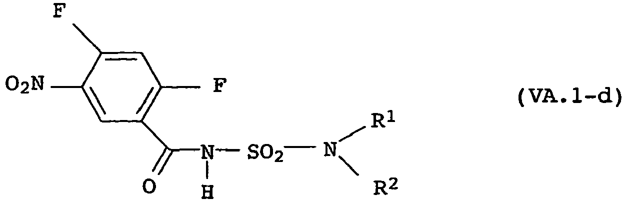

Ganz besonders bevorzugt sind die Nitrobenzoylsulfamidsäureamide der Formel VA.1-e (≡ v mit Ar = Ar-1 mit R^{a} = CN und R^{b} = R^{d} = Wasserstoff und R^{c} = F, A = NR¹R²), worin R¹, R² die oben genannten, insbesondere die als bevorzugt genannten Bedeutungen, aufweisen. Beispiele für derartige Verbindungen sind die Verbindungen VA.1-e.1 bis VA.1-e.495, in denen die Variablen R¹, R² gemeinsam die in einer Zeile der Tabelle 1 angegebenen Bedeutungen aufweisen.

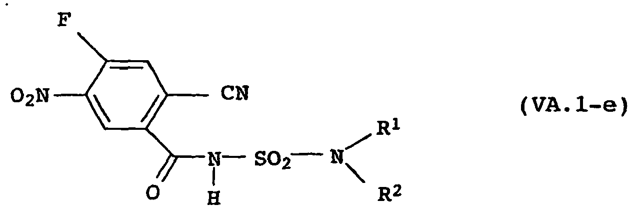

Ganz besonders bevorzugt sind die Nitrobenzoylsulfamidsäureamide der Formel VA.1-f (≡ V mit Ar = Ar-1 mit R^{a} = CN und R^{b} = R^{d} = Wasserstoff und R^{c} = Cl, A = NR¹R²), worin R¹, R² die oben genannten, insbesondere die als bevorzugt genannten Bedeutungen, aufweisen. Beispiele für derartige Verbindungen sind die Verbindungen VA.1-f.1 bis VA.1-f.495, in denen die Variablen R¹, R² gemeinsam die in einer Zeile der Tabelle 1 angegebenen Bedeutungen aufweisen.

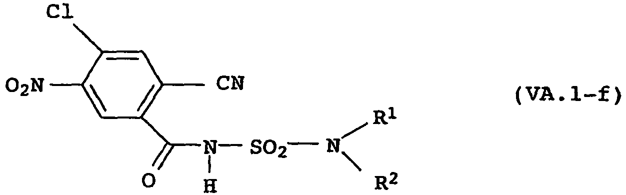

Die erfindungsgemäßen bifunktionellen Phenyliso(thio)cyanate I können als Ausgangsmaterialien für pharmakologisch aktive Verbindungen oder Pflanzenschutzwirkstoffe verwendet werden. Beispielsweise werden in der WO 01/83459 herbizide 3-(Triazolidindion) substituierte Benzoesäuresulfamoylamide der nachfolgenden allgemeinen Formel beschrieben, 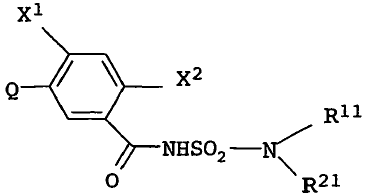 worin X¹ für Wasserstoff, Halogen, C₁-C₄-Alkyl, X² für Wasserstoff, CN, CS-NH₂, Halogen, C₁-C₄-Alkyl, C₁-C₄-Halogenalkyl, R¹¹, R²¹ für die zuvor für R¹, R² angegebenen Bedeutungen und insbesondere für Wasserstoff, gegebenenfalls substituiertes Hydroxy, C₁-C₁₀-Alkyl, C₂-C₁₀-Alkenyl, C₃-C₁₀-Alkinyl, C₃-C₇-Cycloalkyl, Phenyl, Benzyl oder C₅-C₇-Cycloalkenyl stehen oder R¹¹, R²¹ zusammen mit dem Stickstoffatom, an das sie gebunden sind, einen 3- bis 7-gliedrigen heterocyclischen Ring bilden und Q für einen Rest der Formel a 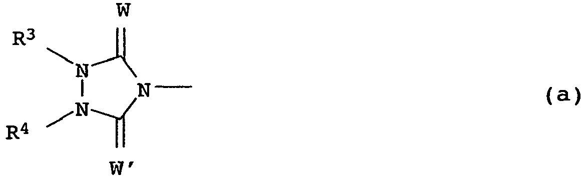 steht, worin W die zuvor genannten Bedeutungen aufweist, W' für O oder S steht und R³ und R⁴ unabhängig voneinander für einen der folgenden Reste stehen: Wasserstoff, Cyano, Amino, C₁-C₆-Alkyl, C₁-C₆-Halogenalkyl, C₁-C₆-Halogenalkoxy, C₃-C₇-Cycloalkyl, C₂-C₆-Alkenyl, C₂-C₆-Halogenalkenyl, C₃-C₆-Alkinyl, Benzyl, OR⁵ (worin R⁵ für Wasserstoff, C₁-C₆-Alkyl, C₁-C₆-Halogenalkyl, C₃-C₇-Cycloalkyl, C₂-C₆-Alkenyl, C₃-C₆-Alkinyl, gegebenenfalls substituiertes Phenyl oder gegegebenenfalls substituiertes Benzyl steht), C₁-C₃-Cyanoalkyl, oder R³ und R⁴ zusammen mit den Stickstoffatomen, an die sie gebunden sind, einen vier- bis siebengliedrigen, gegebenenfalls durch Schwefel, Sauerstoff, eine Gruppe NR⁶ (worin R⁶ die zuvor genannten Bedeutungen aufweist) oder Stickstoff unterbrochenen Heterocyclus bilden, der gegebenenfalls ein- oder mehrfach durch Halogen oder C₁-C₄-Alkyl substituiert ist,
und insbesondere für einen Rest der Formel b steht: 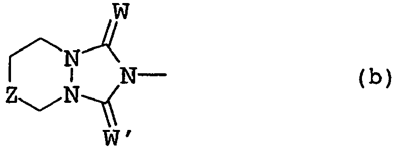 worin W die zuvor genannte Bedeutung und W', Z unabhängig voneinander für Sauerstoff oder Schwefel stehen.

Die in der WO 01/83459 beschriebenen Herbizide sind nicht immer in ausreichender Ausbeute und Reinheit zugänglich. Die dort beschriebenen Verfahren beruhen beispielsweise:
A) auf der Kondensation einer substituierten Benzoesäure mit einem substituierten Sulfamidsäureamid in Gegenwart von N,N-Carbonyldiimidazol (CDI) oder Umwandlung der Carbonsäure in ihr Säurechlorid und anschließende Umsetzung des Säurechlorids mit dem Sulfamidsäureamid. 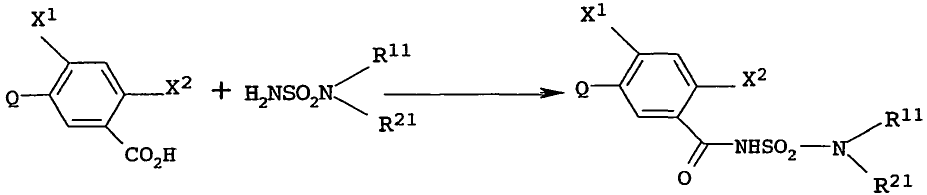 Hierbei können die Variablen R¹¹, R²¹, X¹ und X² die zuvor genannten Bedeutungen aufweisen und Q steht für einen 5- oder 6-gliedrigen Heterocyclus, z. B. für einen Rest a oder b.
   Nachteilig an dem Verfahren ist, dass die eingesetzte Benzoesäure erst durch Spaltung mit Bortribromid bei entsprechendem Salzanfall aus dem vorangehenden Ester erhältlich ist. Zudem liegt die Ausbeute der Kondensation mit Sulfamidsäureamiden nur zwischen 16 und 45 %. Auch der Umweg über ein vorher hergestelltes Säurechlorid führt in nur 26 % Ausbeute zu dem gewünschten Benzoylsulfamidsäureamid, das zudem chromatographisch von seinen Verunreinigungen befreit werden muss.
B) Ersatz eines Halogenrestes durch den heterocyclischen Rest Q: 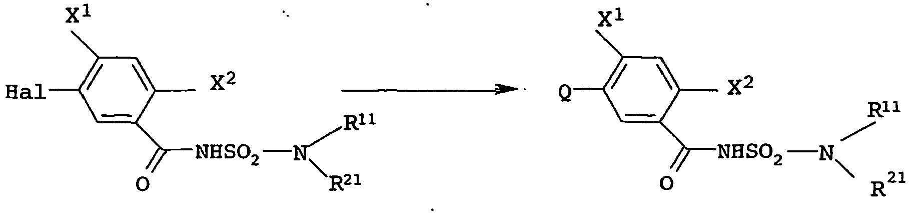 Hierbei können die Variablen R¹¹, R²¹, X¹ und X² die zuvor genannten Bedeutungen aufweisen, Hal steht für Fluor, Chlor oder Brom und Q steht für einen 5- oder 6-gliedrigen Heterocyclus, z. B. für einen Rest a oder b.
   Nachteile dieses Verfahrens sind, dass der eingesetzte Halogenaromat erst umständlich über eine Sandmeyer-Reaktion bereitgestellt werden muss und außerdem die unbefriedigende Selektivität bei der Reaktion der 5-Halogen substituierten Verbindung im Vergleich zu den im gleichen Molekül enthaltenen - aktivierten - 2,4-Dihalogensubstituenten.

Nach dem Stand der Technik sind daher alle bisherigen Verfahren zur Herstellung von 3-(Triazolidindion) substituierten Benzoylsulfamoylamiden und deren Schwefel-Analoga im Hinblick auf einen kurzen Reaktionsverlauf, Einfachheit der Reaktionsführung, Ausbeuten und Reinheit der Endprodukte noch nicht hinreichend zufriedenstellend und daher nicht wirtschaftlich.

Der vorliegenden Erfindung liegt somit auch die Aufgabe zugrunde, ein Verfahren zur Herstellung von Verbindungen der Formel VI, 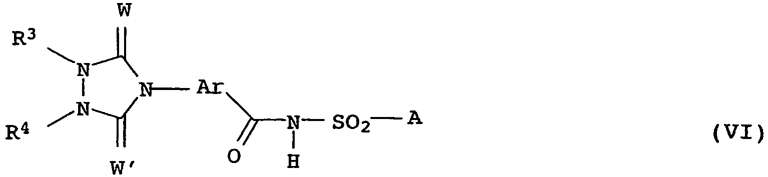 bereitzustellen, worin W, Ar und A die in Anspruch 1 genannten Bedeutungen aufweisen, W' für O oder S steht, und R³ und R⁴ unabhängig voneinander für Wasserstoff, Cyano, Amino, C₁-C₆-Alkyl, C₁-C₆-Halogenalkyl, C₁-C₆-Halogenalkoxy, C₃-C₇-Cycloalkyl, C₂-C₆-Alkenyl, C₂-C₆-Halogenalkenyl, C₃-C₆-Alkinyl, Benzyl, OR⁵ (worin R⁵ für Wasserstoff, C₁-C₆-Alkyl, C₁-C₆-Halogenalkyl, C₃-C₇-Cycloalkyl, C₂-C₆-Alkenyl, C₃-C₆-Alkinyl, gegebenenfalls substituiertes Phenyl oder gegegebenenfalls substituiertes Benzyl steht), C₁-C₃-Cyanoalkyl, stehen, oder R³ und R⁴ zusammen mit den Stickstoffatomen, an die sie gebunden sind, einen vier- bis siebengliedrigen, gegebenenfalls durch Schwefel, Sauerstoff, eine Gruppe NR⁶ (worin R⁶ die zuvor genannten Bedeutungen aufweist) oder Stickstoff unterbrochenen Heterocyclus, der gegebenenfalls ein- oder mehrfach durch Halogen oder C₁-C₄-Alkyl substituiert ist, bilden.

Überraschend wurde nun gefunden, dass man ausgehend von den erfindungsgemäßen Verbindungen der Formel I, insbesondere der Formel IA, die in WO 01/83459 beschriebenen Verbindungen der Formel VI sehr viel einfacher, ohne Nebenreaktionen und in höherer Ausbeute und Reinheit herstellen kann.

Gegenstand der vorliegenden Erfindung ist somit auch ein Verfahren zur Herstellung der Verbindungen der allgemeinen Formel VI 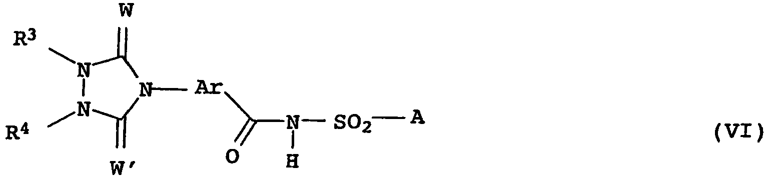 worin R³, R⁴, W, W', Ar, A die zuvor genannten Bedeutungen aufweisen,
umfassend die Schritte
(i) Umsetzung einer Verbindung der Formel I wie zuvor definiert, mit einem Oxadiazincarbonsäureester der Formel VII, 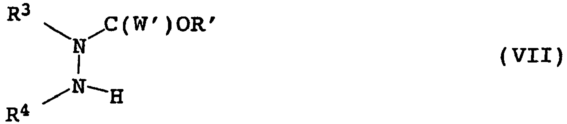 worin W' die zuvor genannte Bedeutung aufweist und R' für C₁-C₄-Alkyl steht, wobei man ein Harnstoffderivat der Formel VIII erhält, 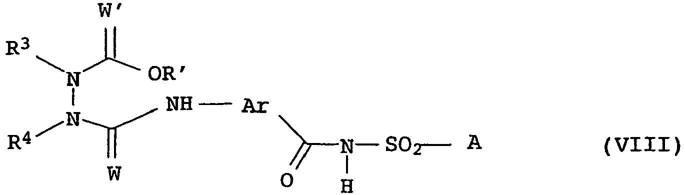 worin die Variablen R³, R⁴, R', W, W', Ar und A die zuvor genannten Bedeutungen aufweisen, und
(ii) Cyclisierung des erhaltenen Zwischenproduktes VIII, wobei man eine Verbindung der Formel VI erhält.

Die Durchführung des Schritts (i) erfolgt in an sich bekannter Weise, z. B. wie in der WO 02/20531 beschrieben. In der Regel gibt man das erfindungsgemäße Iso(thio)cyanat der Formel I zu einer Verbindung der Formel VII, vorzugsweise in einem Lösungsmittel, zu. Als Lösungsmittel kommen Kohlenwasserstoffe wie Pentan, Hexan, Cyclopentan, Cyclohexan, Toluol, Xylol, chlorierte Kohlenwasserstoffe wie Methylenchlorid, Chloroform, 1,2-Dichlorethan, 1,1,2,2-Tetrachlorethan, Chlorbenzol, 1,2-, 1,3- oder 1,4-Dichlorbenzol, Ether wie 1,4-Dioxan, Anisol, Glykolether wie Dimethylglykolether, Diethylglykolether, Diethylenglykoldimethylether, Ester wie Ethylacetat, Propylacetat, Methylisobutyrat, Isobutylacetat, Carbonsäureamide wie N,N-Dimethylformamid, N-Methylpyrrolidon, Nitrokohlenwasserstoffe wie Nitrobenzol, Nitrile wie Acetonitril, Propionitril, Butyronitril oder Isobutyronitril oder auch Gemische einzelner Lösungsmittel in Betracht. Die Zugabe erfolgt in der Regel innerhalb 5 bis 30 Minuten. Üblicherweise beträgt die Temperatur während der Zugabe 10 bis 25 ⁰C. Zur Vervollständigung der Reaktion rührt man noch 0,5 bis 24 Stunden bei 20 bis 80 °C nach. Selbstverständlich kann man auch das Iso(thio)cyanat I in einem der vorgenannten Lösungsmittel vorlegen und die Verbindung VII zugeben und dann die Reaktion wie oben beschrieben zu Ende führen. Üblicherweise setzt man 0,9 bis 1,4 mol, vorzugsweise 0,95 bis 1,1 mol und besonders bevorzugt 0,98 bis 1,15 mol der Verbindung VII pro mol der Verbindung I ein. Die in Schritt (i) eingesetzte Verbindung der allgemeinen Formel VII ist bekannt oder lässt sich in Anlehnung an das in der WO 02/20531 beschriebene Verfahren herstellen.

Schritt (ii) erfolgt wiederum in an sich bekannter Weise, z. B. wie in der WO 02/20531 beschrieben, indem man die Verbindung der Formel VIII mit einer Base behandelt.

Als Base kommen grundsätzlich alle Verbindungen in Betracht, die das azide Proton der NH-Gruppe der Harnstofffunktion in den Verbindungen der Formel VIII abstrahieren können. Hierzu zählen Oxobasen, Stickstoffbasen und Hydridbasen.

Zu den Oxobasen zählen beispielsweise anorganische Basen wie Alkali- oder Erdalkalihydroxide, Alkali- und Erdalkalihydrogencarbonate sowie Alkali- und Erdalkalicarbonate, beispielsweise Lithium-, Natrium-, Kalium-, Calcium- oder Magnesiumhydroxid, -hydrogencarbonat oder -carbonat. Ebenfalls geeigente Oxobasen sind Alkalimetallalkoholate, insbesondere des Lithiums, Natriums oder Kaliums, wobei man in der Regel Alkoholate von C₁-C₆- vorzugsweise C₁-C₄-Alkanolen wie Natrium- oder Kaliummethylat, -ethylat, -n-butylat oder tert.-butylat einsetzt. Zu den Stickstoffbasen zählen primäre, sekundäre oder vorzugsweise tertiäre Amine z. B. Trialkylamine wie Triethylamin, Tri-n-propylamin, N-Ethyl-diisopropylamin, cycloaliphatische Amine wie N, N-Dimethylcyclohexylamin, cyclische Amine wie Azabicyclo[2.2.2]octan (= Triethylendiamin), N-Methylpyrrolidin, N-Ethylpiperidin, Dialkylaniline wie Dimethylaminoanilin, p-Dimethylaminopyridin, weiterhin aromatische Stickstoffheterocyclen wie Pyridin, α-, β- oder γ-Picolin, 2,4-und 2,6-Lutidin, Chinolin, Chinazolin, Chinoxalin, Pyrimidin, sowie tertäre Amide, z. B. Dimethylformamid, Ameisensäure-N-methylamidid, N-Methylpyrrolidon oder Tetramethylharnstoff.

Hydridbasen sind beispielsweise Alkalimetallhydride wie Natriumhydrid oder Kaliumhydrid. Bevorzugte Basen sind tertiäre Amine, insbesondere Trialkylamine.

Vorzugsweise setzt man 0,9 bis 1,4 mol, insbesondere 0,95 bis 1,2 mol und besonders bevorzugt 0,98 bis 1,15 mol der Verbindung VIII pro mol Base ein.

Zur Umsetzung von Verbindung VIII mit der Base legt man vorzugsweise die Verbindung VIII in einem der vorgenannten Lösungsmittel oder einen Lösungsmittelgemisch vor, gibt unter Durchmischen, z. B. unter Rühren, die Base in den Reaktionsansatz. Vorzugsweise erfolgt die Basenzugabe bei einer Temperatur im Bereich von 0 bis 50 °C und insbesondere bei 10 bis 30 °C.

In der Regel wird man dann zur Vervollständigung der Reaktion die Komponenten noch 10 Minuten bis 48 Stunden bei 20 bis 150 °C, vorzugsweise 20 bis 100 °C und insbesondere 20 bis 60 °C nachreagieren lassen. Die Reaktion ist bei Thioharnstoffen der Formel VIII (W = S) im allgemeinen nach 0,5 bis 10 Stunden, bei Harnstoffen der Formel VIII (W = O) nach 4 bis 48 Stunden und insbesondere nach 8 bis 24 Stunden weitgehend vollständig (Umsatz > 90%). Man kann jedoch auch die Base, vorzugsweise in einem der vorgenannten Lösungsmittel vorlegen, und dann die Verbindung VIII zugeben und wie oben die Reaktion zu Ende führen.

Die Konzentration der Edukte im Lösungsmittel liegt im allgemeinen im Bereich von 0,5 bis 5 mol/l, bevorzugt im Bereich von 0,2 bis 2 mol/l.

Die Aufarbeitung der Reaktion erfolgt in üblicher Weise, beispielsweise wässrig extraktiv, durch Dialyse und/oder chromatographisch.

Insbesondere betrifft das vorliegende Verfahren die Herstellung der Verbindungen VIA 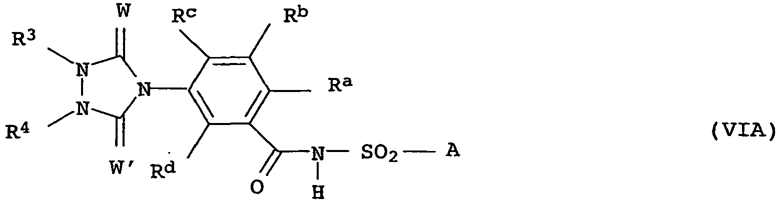 worin R³ und R⁴ die zuvor genannten Bedeutungen, die Variablen W, W', R^{a}, R^{b}, R^{c}, R^{d}, A die zuvor genannten Bedeutungen, und insbesondere die bereits im Zusammenhang mit der Beschreibung der Verbindung IA als bevorzugt für diese Variablen genannten Bedeutungen aufweisen. Die im erfindungsgemäßen Verfahren zur Herstellung der Verbindung VIA eingesetzte Verbindung ist dann eine Verbindungen der Formel IA, vorzugsweise eine Verbindung der Formel IA.1.

Eine bevorzugte Verbindung der Formel VII ist beispielsweise eine Verbindung der Formel (VII') 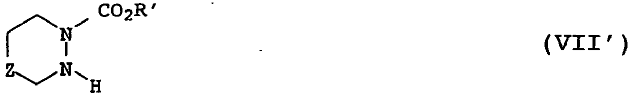 worin Z für 0 oder S und R' für C₁-C₄-Alkyl steht. Diese Verbindung ist aus der WO 02/20531 bekannt.

Insbesondere gelingt es auf diesem Weg, ausgehend von den Verbindungen der Formel IA, gemäß dem folgenden Schema 3, Verbindungen der Formel IX (= Verbindung VIA mit R^{b} = R^{d} = H, A = NP¹R², W = W' = O und R³, R⁴ stehen für CH₂CH₂OCH₂) herzustellen. 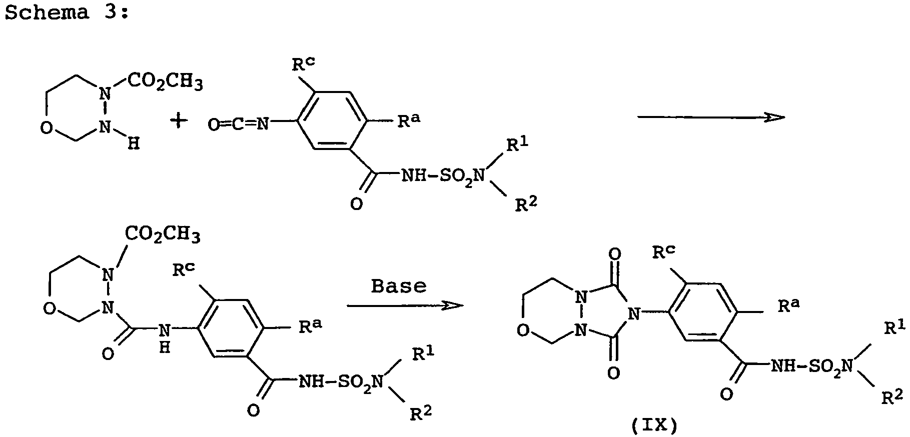 Die Variablen R^{a}, R^{c}, R¹ und R² weisen hierin die zuvor genannten Bedeutungen auf.

Das erfindungsgemäße Verfahren ist dem in der WO 01/83459 beschriebenen Verfahren hinsichtliche Ausbeute und Reinheit überlegen. Außerdem ist es sehr viel einfacher durchzuführen. Bezüglich der Nachteile des aus der WO 01/83459 bekannten Verfahrens sei auf das zuvor Gesagte verwiesen.

Die folgenden Beispiele dienen der Erläuterung der Erfindung

### I Herstellung der Nitrobenzoylsulfamidsäureamide (Vorstufe der allgemeinen Formel VA.1; Vorprodukte VA.1-1 bis VA.1-24):

### Beispiel 1: N-(2-Chlor-4-fluor-5-nitro-benzoyl)-N'-n-propyl-N'-allylsulfamid (VA.1-a.241)

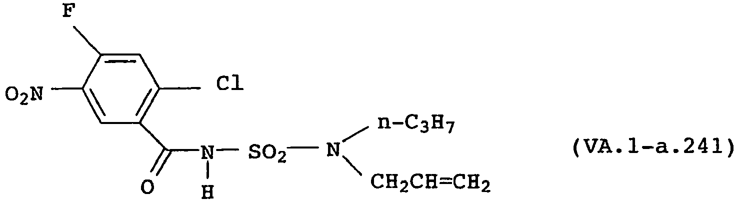

Bei -5 °C bis 0 °C gab man zu einer Mischung von 8,50 g (0,048 mol) N'-Propyl-N'-allylsulfamid, 10,38 g (0,103 mol) Triethylamin und 0,09 g (0,736 mmol) 4-N,N-Dimethylaminopyridin in 90 ml Methylenchlorid unter Rühren innerhalb 30 Minuten 11,62 g (0,0474 mol) 2-Chlor-4-fluor-5-nitrobenzoylchlorid in 50 ml Methylenchlorid. Man spülte mit 10 ml des Lösungsmittels nach. Man rührte zunächst 1 Stunde bei 0 °C und anschließend 2 Stunden bei 22 °C nach. Anschließend gab man 50 ml 1N Salzsäure zu, rührte und trennte die Phasen. Man wusch die organische Phase noch zweimal mit 1N Salzsäure und extrahierte die wässrige Phase mit Methylenchlorid. Nach dem Trocknen der organischen Phase über Magnesiumsulfat filtrierte man und engte die Lösung ein. Den Rückstand verrührte man mit Diethylether/Pentan, saugte ab und trocknete, wobei man 18,41 g (91,9% der Theorie) der Titelverbindung mit einem Schmelzpunkt (Schmp.) von 110-112 °C erhielt.

In analoger Weise wurden die in Tabelle 2 angegebenen Vorstufen VA.1 (Verbindungen der Formel VI mit Ar = Ar-1 mit R^{b}, R^{d} = H mit den in Tabelle 1 angegebenen Bedeutungen für R¹ und R²) der Beispiele 2 bis 24 erhalten.

**Tabelle 2:**

| | | | | | |
|---|---|---|---|---|---|
| 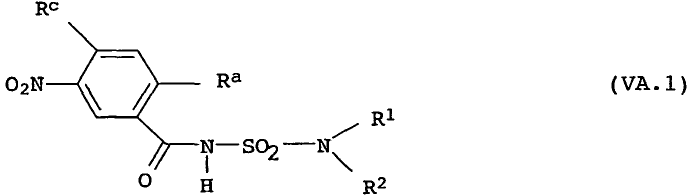 | | | | | |

| Beispiel / Nr. 1) | R^{c} | R^{a} | R¹ | R² | Schmp. [°C]/ ¹H-NMR (400 MHz, CDCl₃) δ (ppm) |
|---|---|---|---|---|---|
| 1 VA.1-a.241 | F | Cl | n-C₃H₇ | CH₂=CH-CH₂ | 110-112 |
| 2 VA.1-a.490 | F | Cl | CH₂-CH₂-CH(CH₃)-CH₂-CH₂ | | 137 - 138 |
| 3 VA.1-a.387 | F | Cl | i-C₃H₇ | HC≡C-CH₂ | 160 - 161 |
| 4 VA.1-b.492 | H | Cl | CH₂-CH₂-CH(CH₃)-CH₂-CH₂ | | 151 - 152 |
| 5 VA.1-b.241 | H | Cl | n-C₃H₇ | CH₂=CH-CH₂ | 132 - 134 |
| 6 VA.1-b.387 | H | Cl | i-C₃H₇ | HC≡C-CH₂ | 138 - 140 |
| 7 VA.1-a.86 | F | Cl | CH3 | i-C₃H₇ | 121 - 122 |
| 8 VA.1-a.76 | F | Cl | CH3 | CH₃ | |
| 9 VA.1-a. 77 | F | Cl | CH₃ | C₂H₅ | |
| 10 VA.1-a.84 | F | Cl | CH3 | n-C₃H₇ | |
| 11 VA.1-a.98 | F | Cl | CH3 | C-C₃H₅ | |
| 12 VA.1-a.85 | F | Cl | CH3 | n-C₄H₉ | |
| 13 VA.1-a.88 | F | Cl | CH3 | i-C₄H₉ | |
| 14 VA.1-a.87 | F | Cl | CH3 | sek.-C₄H₉ | |
| 15 VA.1-a.89 | F | Cl | CH₃ | tert.-C₄H₉ | |
| 16 VA.1-a.93 | F | Cl | CH3 | CH₂=CH-CH₂ | |
| 17 VA.1-a.96 | F | Cl | CH3 | HC≡C-CH₂ | |
| 18 VA.1-a.107 | F | Cl | CH3 | C₆H₅ | |
| 19 VA.1-a.445 | F | Cl | CH3 | Cyclohexyl | |
| 20 VA.1-a.181 | F | Cl | C₂H₅ | C₆H₅ | |
| 21 VA.1-a.446 | F | Cl | C₂H₅ | Cyclohexyl | |
| 22 VA.1-a.160 | F | Cl | C₂H₅ | i-C₃H₇ | |
| 23 VA.1-a.167 | F | Cl | C₂H₅ | CH₂=CH-CH₂ | |
| 24 VA.1.-b.87 | H | Cl | CH3 | sek.-C₄H₉ | 8,4 (d, 1H), 8,2 (m, 1H), 7,6 (d, 1H), 4,0 (sept., 1H), 2,9 (s, 3H), 1,5 (m, 2H), 1,2 (d, 6H), 0,9 (t, 3H). |

### 1) Verbindungsnummer gemäß Tabelle 1

### II Herstellung der Aminobenzoylsulfamidsäureamide der allgemeinen Formel IIA (Vorprodukte IIA.1):

### IIa Reduktion der Nitrogruppe mit Eisenpulver in Essigsäure

### Beispiel 25: N-(5-Amino-2-chlor-4-fluor-benzoyl)-N'-allyl-N'-n-propylsulfamid (IIA.1-a.241)

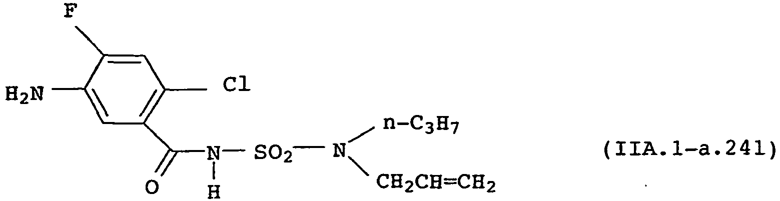

Zu einer Suspension von 7,54 g (135,072 mmol) Eisenpulver in 60 ml Essigsäure gab man unter Rühren innerhalb 25 Minuten eine Lösung von 17,1 g (45,02 mmol) der Verbindung VA.1-a.241 aus Beispiel 1 in einer Mischung von 5 ml Tetrahydrofuran und 40 ml Essigsäure bei 70 bis 75 °C. Man rührte noch 1 Stunde bei 70 bis 75 °C nach, ließ abkühlen und engte im Vakuum ein. Man rührte den Rückstand mit Essigester, filtrierte und wusch den Niederschlag mit Essigester. Man rührte das Filtrat mit Aktivkohle und Magnesiumsulfat, filtrierte, wusch und engte ein. Nach dem Anteigen des Rückstands mit Essigester, Verrühren mit Pentan, Absaugen und Trocknen erhielt man 12,1 g (75,3% der Theorie) der Titelverbindung mit einem Schmelzpunkt von 104 bis 106 °C.

### IIb Katalytische Hydrierung der Nitrogruppe

### Beispiel 31: N-(5-Amino-2-chlor-4-fluor-benzoyl)-N'-methyl-N'-isopropylsulfamid (IIA.1-a.86)

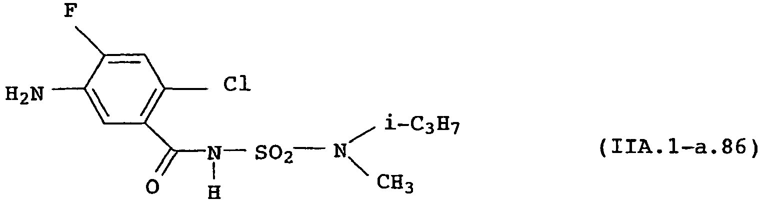

Man legte 112,0 g (0,317 mol) der Verbindung VA.1-a.86 aus Beispiel 7 und 100 g Raney-Nickel in 1200 ml Methanol in einer Hydrierapparatur vor. Unter Rühren spülte man mit 10 1 Stickstoff und mit 10 1 Wasserstoff. Unter Rühren hydrierte man bei 22 - 23 °C mit 0,1 bar Wasserstoff. Insgesamt wurden 21,3 1 Wasserstoff aufgenommen. Nach dem Abblasen des Überdrucks spülte man erneut mit 10 1 Stickstoff. Man saugte das Reaktionsgemisch über Kieselgel ab und engte das Filtrat im Vakuum ein. Man erhielt 100,5 g

(97 % der Theorie) der Titelverbindung mit einem Schmelzpunkt von 160 - 162 °C (HPLC-Reinheit: 99,1 %).

In analoger Weise wurden ausgehend von den in Tabelle 2 angegebenen Nitrobenzyolsulfamidsäureamiden VA.1 die in Tabelle 3 angegebenen Vorstufen IIA (Verbindungen der Formel II mit Ar = Ar-1 mit R^{b}, R^{d} = H mit den in Tabelle 1 angegebenen Bedeutungen für R¹ und R²) der Beispiele 26 bis Beispiel 48 erhalten.

**Tabelle 3:**

| | | | | | |
|---|---|---|---|---|---|
| 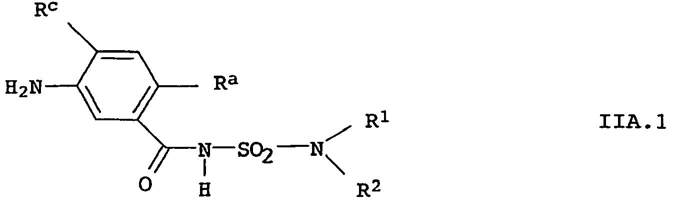 | | | | | |

| Beispiel / Nr. ¹⁾ | R^{c} | R^{a} | R¹ | R² | Schmp. [°C]/ ¹H-NMR (400 MHz, CDCl₃) δ (ppm) |
|---|---|---|---|---|---|
| 25 IIA.1-a.241 | F | Cl | n-C₃H₇ | CH₂=CH-CH₂ | 104 - 106 |
| 26 F Cl IIA.1-a.492 | F | Cl | CH₂-CH₂-CH(CH₃)-CH₂-CH₂ | | 144 - 145 |
| 27 IIA.1-a.387 | F | Cl | i-C₃H₇ | HC≡C-CH₂ | 153 - 154 |
| 28 IIA.1-b.492 | H | Cl | CH₂-CH₂-CH(CH₃)-CH₂-CH₂ | | 139 |
| 29 IIA.1-b.241 | H | Cl | n-C₃H₇ | CH₂=CH-CH₂ | 138 |
| 30 IIA.1-b.387 | H | Cl | i-C₃H₇ | HC≡C-CH₂ | 139 - 140 |
| 31 IIA.1-a.86 | F | Cl | CH₃ | i-C₃H₇ | 160 - 162 |
| 32 IIA.1-a.76 | F | Cl | CH₃ | CH₃ | |
| 33 IIA.1-a.77 | F | Cl | CH₃ | C₂H₅ | |
| 34 IIA.1-a.84 | F | Cl | CH₃ | n-C₃H₇ | |
| 35 IIA.1-a.98 | F | Cl | CH3 | C-C₃H₅ | |
| 36 IIA.1-a.85 | F | Cl | CH₃ | n-C₄H₉ | |
| 37 IIIA.1-a.88 | F | Cl | CH₃ | i-C₄H₉ | |
| 38 87 IIIA.1-a.87 | F | Cl | CH₃ | sek.-C₄H₉ | |
| 39 IIA.1-a.89 | F | Cl | CH3 | tert.-C₄H₉ | |
| 40 IIA.1-a.93 | F | Cl | CH3 | CH₂=CH-CH₂ | |
| 41 IIA.1-a.96 | F | Cl | CH₃ | HC≡C-CH₂ | |
| 42 IIA.1-a.107 | F | Cl | CH₃ | C₆H₅ | |
| 43 IIA.1-a.445 | F | Cl | CH3 | Cyclohexyl | |
| 44 IIA.1-a.181 | F | Cl | C₂H₅ | C₆H₅ | |
| 45 IIA.1-a.446 | F | Cl | C₂H₅ | Cyclohexyl | |
| 46 IIA.1-a.160 | F | Cl | C₂H₅ | i-C₃H₇ | |
| 47 IIA.1-a.167 | F | Cl | C₂H₅ | CH₂=CH-CH₂ | |
| 48 IIA.1-b.87 | H | Cl | CH3 | sek.-C₄H₉ | 8,8 (br. s), 7,2 (d, 1H), 7,1 (m, 1H), 6,8 (d, 1H), 4,0 (m, 1H), 3,8 (br. s, 2H), 2,9 (s, 3H), 1,6-1,4(m, 2H), 1,2 (d, 3H), 0,9 (t, 3H) |

### 1) Verbindungsnummer gemäß Tabelle 1

### III Herstellung der Phenyliso(thio)cyanate I

### Beispiel 109: N-(2-Chlor-4-fluor-5-isocyanato-benzoyl)-N'-allyl-N'-n-propylsulfamid (IA.1-a.241)

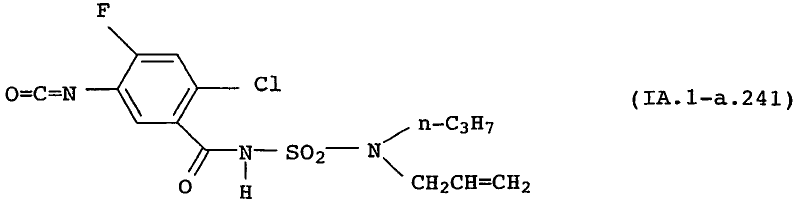

Zu 6,0 g (17,2 mmol) der Verbindung IIA.1-a.241 aus Beispiel 25 in 50 ml Dioxan gab man unter Rühren bei 15 bis 25 °C 4,7 ml einer 4 M HCl-Lösung in Dioxan (entspricht 18,9 mmol Chlorwasserstoff). Man rührte noch 1 Stunde bei 22 °C nach. Anschließend leitete man unter Rühren und langsamer Erhöhung der Temperatur auf 95 °C 3,4 g (34,3 mmol) Phosgen innerhalb 1 h ein. Nicht umgesetztes Phosgen wurde mit Stickstoff ausgetragen. Man engte danach die Reaktionsmischung im Vakuum ein, verrührte den Rückstand mit Pentan, dekantierte den Überstand ab und engte den Überstand im Vakuum ein. Man erhielt 6,5 g (95,8% d. Th., Reinheit laut ¹H-NMR : 95 %) der Titelverbindung mit einem Schmelzpunkt von 85 - 95°C (Zers.).
IR (KBr): N=C=O 2265 cm⁻¹; C=O 1724 cm⁻¹.

### Beispiel 94: N-(2-Chlor-4-fluor-5-isocyanato-benzoyl)-N'-methyl-N'-isopropyl-sulfamid (IA.1-a.86)

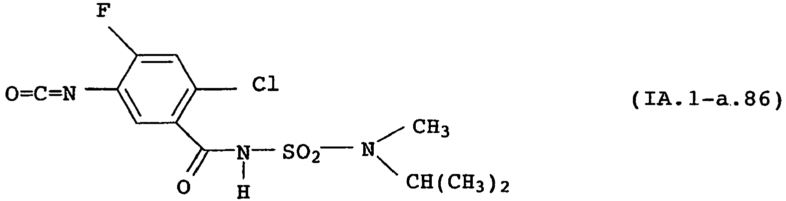

### A) durch Umsetzung mit Phosgen

In eine Lösung von 5,0 g (15,4 mmol) der Verbindung IIA.1-a.86 aus Beispiel 31 in 50 ml Dioxan leitete man bei 22 °C unter Rühren Phosgen ein. Innerhalb 20 Minuten erhöhte man die Temperatur bis zum Rückfluss des Lösungmittels. Man leitete noch 1 Stunde Phosgen ein, ließ auf Raumtemperatur abkühlen und spülte mit Stickstoff. Man engte das Reaktionsgemisch im Vakuum zunächst bei 22 °C und anschließend bei 70 °C ein. Den Rückstand verrührte man mit n-Hexan, dekantierte und trocknete den Rückstand bei 70 °C, wobei man 5,5 g (99,8 % der Theorie mit einer ¹H-NMR-Reinheit von 98 %) der Titelverbindung mit einem Schmelzpunkt von 146 - 149 °C erhielt.

### B) durch Umsetzung mit Diphosgen

Zu einer Lösung von 5,0 g (15,4 mmol) der Verbindung IIA.1-a.86 in 50 ml Dioxan tropfte man unter Rühren bei 10 °C 6,11 g (30,9 mmol) Diphosgen. Man ließ das Reaktionsgemisch auf 22 °C erwärmen und rührte noch 1,5 Stunden. Laut dünnschichtchromatographischer Untersuchungen war die Umsetzung dann vollständig. Nach Rühren über Nacht spülte man mit Stickstoff und arbeitete wie zuvor in Beispiel 94A beschrieben auf. Man erhielt 5,5 g (99,8 % der Theorie, mit einer ¹R-NMR-Reinheit von 98 %) der Titelverbindung mit einem Schmelzpunkt von 148 - 150 °C.

### Beispiel 118 N-(2-Chlor-4-fluor-5-isocyanato-benzoyl)-N-(4-methyl-piperidin-sulfonsäureamid) (IA.1-a.492)

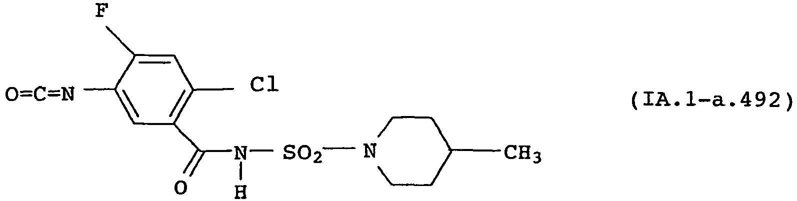

Zu 1,8 g (5,1 mmol) der Verbindung IIA.1-a.492 aus Beispiel 26 in 50 ml Dioxan gab man unter Rühren bei 20 bis 25°C 2,6 ml einer 4 M HCl-Lösung (entsprechend 0,38 g (10,3 mmol) Chlorwasserstoff) in Dioxan. Man rührte noch 1 Stunde bei 22 °C nach. Anschließend gab man weitere 1,12 g (5,66 mmol) Diphosgen unter Rühren zu, rührte 30 min bei 22 °C, erhitzte langsam auf 95°C und rührte 1 Stunde nach. Nach dem Abkühlen auf Raumtemperatur engte man im Vakuum ein, verrührte den Rückstand mit Pentan, dekantierte die überstehende Lösung ab und engte die Lösung erneut im Vakuum ein. Man erhielt 2,0 g (98,3% der Theorie, mit 95% ¹H-NMR-Reinheit) der Titelverbindung mit einem Schmelzpunkt von 122-124 °C (Zers.), 135°C. klar.
IR (KBr): N=C=O 2246 cm⁻¹ ; C=O 1697 cm⁻¹.

### Beispiel 193: N-(2-Chlor-4-fluor-5-isothiocyanato-benzoyl)-N'-allyl-N'-n-propyl-sulfamid (IA.1-g.241)

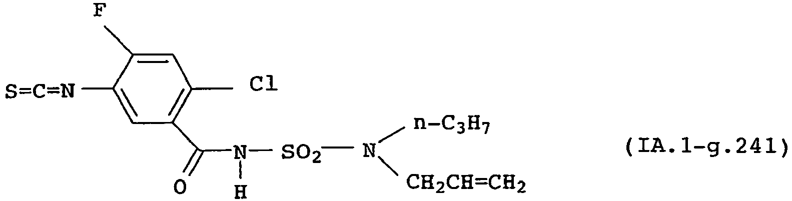

Zu 3,0 g (8,6 mmol) der Verbindung IIA.1-a.241 aus Beispiel 25 in 50 ml Essigester gab man unter Rühren bei 22°C 1,1 g (9,4 mmol) Thiophosgen, rührte anschließend 1 Stunde nach, erwärmte dann auf 75 °C und rührte eine weitere Stunde nach. Nach dem Einengen im Vakuum, Verrühren des Rückstandes mit Pentan, Absaugen und Trocknen erhielt man 3,4 g (96,1% der Theorie, 95% ¹H-NMR-Reinheit) der Titelverbindung mit einem Schmelzpunkt von 83-85 °C.
IR (KBr): N=C=S 2030 cm⁻¹, C=O 1725 cm⁻¹.

In analoger Weise wurden ausgehend von den in Tabelle 3 angegebenen Aminobenzoylsulfamidsäureamiden IIA.1 die in Tabelle 4 angegebenen Titelverbindungen IA.1 (Verbindungen der Formel I mit Ar = Ar-1 mit R^{b}, R^{d} = H mit den in Tabelle 1 angegebenen Bedeutungen für R¹ und R²) der Beispiele 49 bis Beispiele 216 erhalten.

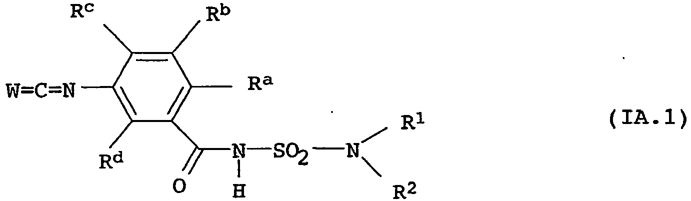

**Tabelle 4:**

| Bsp. | W | R^{c} | R^{a} | R¹ | R² | Schmp. [°C] |
|---|---|---|---|---|---|---|
| 49 | O | H | Cl | CH₃ | CH₃ | |
| 50 | O | H | Cl | CH₃ | C₂H₅ | |
| 51 | O | H | Cl | CH₃ | n-C₃H₇ | |
| 52 | O | H | Cl | CH₃ | i-C₃H₇ | |
| 53 | O | H | Cl | CH3 | C-C₃H₅ | |
| 54 | O | H | Cl | CH₃ | n-C₄H₉ | |
| 55 | O | H | Cl | CH₃ | i-C₄H₉ | |
| 56 | O | H | Cl | CH₃ | sek.- C₄H₉ | |
| 57 | O | H | Cl | CH₃ | tert.-C₄H₉ | |
| 58 | O | H | Cl | C₂H₅ | C₂H₅ | |
| 59 | O | H | Cl | C₂H₅ | n-C₃H₇ | |
| 60 | O | H | Cl | C₂H₅ | i-C₃H₇ | |
| 61 | O | H | Cl | C₂H₅ | c-C₃H₅ | |
| 62 | O | H | Cl | C₂H₅ | n-C₄H₉ | |
| 63 | O | H | Cl | C₂H₅ | i-C₄H₉ | |
| 64 | O | H | Cl | C₂H₅ | sek.- C₄H₉ | |
| 65 | O | H | Cl | CH₂=CH-CH₂ | CH3 | |
| 66 | O | H | Cl | CH₂=CH-CH₂ | C₂H₅ | |
| 67 | O | H | Cl | CH₂=CH-CH₂ | n-C₃H₇ | 102 - 104 (Zers.) |
| 68 | O | H | Cl | CH₂=CH-CH₂ | i-C₃H₇ | |
| 69 | O | H | Cl | CH₂=CH-CH₂ | n-C₄H₉ | |
| 70 | O | H | Cl | CH₂=CH-CH₂ | sek.- C₄H₉ | |
| 71 | O | H | Cl | HC≡C-CH₂ | CH3 | |
| 72 | O | H | Cl | HC≡C-CH₂ | C₂H₅ | |
| 73 | O | H | Cl | HC=C-CH₂ | n-C₃H₇ | |
| Bsp. | W | R^{c} | R^{a} | R¹ | R² | Schmp. [ °C] |
| 74 | O | H | Cl | HC≡C-CH₂ | i-C₃H₇ | 133 - 141 (Zers.) |
| 75 | O | H | Cl | HC≡C-CH₂ | n-C₄H₉ | |
| 76 | O | H | Cl | CH₂-CH₂-CH(CH₃)-CH₂-CH₂ | | 110 - 115 (Zers.) |
| 77 | O | H | Cl | CH₃ | Cyclohexyl | |
| 78 | O | H | Cl | CH₃ | C₆H5 | |
| 79 | O | H | Cl | C₂H₅ | Cyclohexyl | |
| 80 | O | H | Cl | C₂H₅ | C₆H₅ | |
| 81 | O | H | Cl | [CH₂]₄ | | |
| 82 | O | H | Cl | [CH₂]₅ | | |
| 83 | O | H | CN | CH₃ | CH₃ | |
| 84 | O | H | CN | CH₃ | C₂H₅ | |
| 85 | O | H | CN | CH₃ | i-C₃H₇ | |
| 86 | O | H | CN | CH₃ | n-C₃H₇ | |
| 87 | O | H | CN | CH₃ | i-C₄H₉ | |
| 88 | O | H | CN | CH₃ | sek.-C₄H₉ | |
| 89 | O | H | CN | CH₃ | Cyclohexyl | |
| 90 | O | H | CN | CH₃ | C₆H₅ | |
| 91 | O | F | Cl | CH₃ | CH₃ | |
| 92 | O | F | Cl | CH₃ | C₂H₅ | |
| 93 | O | F | Cl | CH₃ | n-C₃H₇ | |
| 94 | O | F | Cl | CH₃ | i-C₃H₇ | 144 - 148 |
| 95 | O | F | Cl | CH₃ | C-C₃H₅ | |
| 96 | O | F | Cl | CH₃ | n-C₄H₉ | |
| 97 | O | F | Cl | CH₃ | i-C₄H₉ | |
| 98 | O | F | Cl | CH₃ | sek.- C₄H₉ | |
| 99 | O | F | Cl | CH₃ | tert.-C₄H₉ | |
| 100 | O | F | Cl | C₂H₅ | C₂H₅ | |
| 101 | O | F | Cl | C₂H₅ | n-C₃H₇ | |
| 102 | O | F | Cl | C₂H₅ | i-C₃H₇ | |
| 103 | O | F | Cl | C₂H₅ | C-C₃H₅ | |
| 104 | O | F | Cl | C₂H₅ | n-C₄H₉ | |
| 105 | O | F | Cl | C₂H₅ | i-C₄H₉ | |
| 106 | O | F | Cl | C₂H₅ | sek.- C₄H₉ | |
| 107 | O | F | Cl | CH₂=CH-CH₂ | CH₃ | |
| 108 | O | F | Cl | CH₂=CH-CH₂ | C₂H₅ | |
| 109 | O | F | Cl | CH₂=CH-CH₂ | n-C₃H₇ | 85 - 95 (Zers.) |
| 110 | O | F | Cl | CH₂=CH-CH₂ | i-C₃H₇ | |
| 111 | O | F | Cl | CH₂=CH-CH₂ | n-C₄H₉ | |
| 112 | O | F | Cl | CH₂=CH-CH₂ | sek.- C₄H₉ | |
| 113 | O | F | Cl | HC≡C-CH_{Z} | CH₃ | |
| 114 | O | F | Cl | HC≡C-CH₂ | C₂H₅ | |
| 115 | O | F | Cl | HC≡C-CH₂ | n-C₃H₇ | |
| 116 | O | F | Cl | HC≡C-CH₂ | i-C₃H₇ | 124 -126 (Zers.) |
| 117 | O | F | Cl | HC≡C-CH₂ | n-C₄H₉ | |
| 118 | O | F | Cl | CH₂-CH₂-CH(CH₃)-CH₂-CH₂ | | 122 - 124 (Zers.) |

| Bsp. | W | R^{c} | R^{a} | R¹ | R² | Schmp. [ °C] |
|---|---|---|---|---|---|---|
| 119 | O | F | Cl | CH₃ | Cyclohexyl | |
| 120 | O | F | Cl | CH₃ | C₆H₅ | |
| 121 | O | F | Cl | C₂H₅ | Cyclohexyl | |
| 122 | O | F | Cl | C₂H₅ | C₆H₅ | |
| 123 | O | F | Cl | [CH₂]₄ | | |
| 124 | O | F | Cl | [CH₂]₅ | | |
| 125 | O | F | CN | CH₃ | CH₃ | |
| 126 | O | F | CN | CH₃ | C₂H₅ | |
| 127 | O | F | CN | CH₃ | i-C₃H₇ | |
| 128 | O | F | CN | CH₃ | n-C₃H₇ | |
| 129 | O | F | CN | CH₃ | i-C₄H₉ | |
| 130 | O | F | CN | CH₃ | sek.-C₄H₉ | |
| 131 | O | F | CN | CH₃ | Cyclohexyl | |
| 132 | O | F | CN | CH₃ | C₆H₅ | |
| 133 | S | H | Cl | CH₃ | CH₃ | |
| 134 | S | H | Cl | CH₃ | C₂H₅ | |
| 135 | S | H | Cl | CH₃ | n-C₃H₇ | |
| 136 | S | H | Cl | CH₃ | i-C₃H₇ | |
| 137 | S | H | Cl | CH₃ | C-C₃H₅ | |
| 138 | S | H | Cl | CH₃ | n-C₄H₉ | |
| 139 | S | H | Cl | CH₃ | i-C₄H₉ | |
| 140 | S | H | Cl | CH₃ | sek.- C₄H₉ | |
| 141 | S | H | Cl | CH₃ | tert.-C₄H₉ | |
| 142 | S | H | Cl | C₂H₅ | C₂H₅ | |
| 143 | S | H | Cl | C₂H₅ | n-C₃H₇ | |
| 144 | S | H | Cl | C₂H₅ | i-C₃H₇ | |
| 145 | S | H | Cl | C₂H₅ | C-C₃H₅ | |
| 146 | S | H | Cl | C₂H₅ | n-C₄H₉ | |
| 147 | S | H | Cl | C₂H₅ | i-C₄H₉ | |
| 148 | S | H | Cl | C₂H₅ | sek₋- C₄H₉ | |
| 149 | S | H | Cl | CH₂=CH-CH₂ | CH3 | |
| 150 | S | H | Cl | CH₂=CH-CH₂ | C₂H₅ | |
| 151 | S | H | Cl | CH₂=CH-CH₂ | n-C₃H₇ | 99 - 100 |
| 152 | S | H | Cl | CH₂=CH-CH₂ | i-C₃H₇ | |
| 153 | S | H | Cl | CH₂=CH-CH₂ | n-C₄H₉ | |
| 154 | S | H | Cl | CH₂=CH-CH₂ | sek.- C₄H₉ | |
| 155 | S | H | Cl | HC≡C-CH₂ | CH₃ | |
| 156 | S | H | Cl | HC≡C-CH₂ | C₂H₅ | |
| 157 | S | H | Cl | HC≡C-CH₂ | n-C₃H₇ | |
| 158 | S | H | Cl | HC≡C-CH₂ | i-C₃H₇ | 163 - 164 |
| 159 | S | H | Cl | HC≡C-CH₂ | n-C₄H₉ | |
| 160 | S | H | Cl | CH₂-CH₂-CH(CH₃)-CH₂-CH₂ | | 143 - 144 |
| 161 | S | H | Cl | CH₃ | Cyclohexyl | |
| 162 | S | H | Cl | CH₃ | C₆H₅ | |
| 163 | S | H | Cl | C₂H₅ | Cyclohexyl | |
| 164 | S | H | Cl | C₂H₅ | C₆H₅ | |
| 165 | S | H | Cl | [CH₂]₄ | | |
| 166 | S | H | Cl | [CH₂]₅ | | |
| 167 | S | H | CN | CH₃ | CH₃ | |
| 168 | S | H | CN | CH₃ | C₂H₅ | |
| 169 | S | H | CN | CH3 | i-C₃H₇ | |
| 170 | S | H | CN | CH3 | n-C₃H₇ | |
| 171 | S | H | CN | CH₃ | i-C₄H₉ | |
| 172 | S | H | CN | CH₃ | sek.-C₄H₉ | |
| 173 | S | H | CN | CH₃ | Cyclohexyl | |
| 174 | S | H | CN | CH₃ | C₆H₅ | |
| 175 | S | F | Cl | CH₃ | CH₃ | |
| 176 | S | F | Cl | CH₃ | C₂H₅ | |
| 177 | S | F | Cl | CH₃ | n-C₃H₇ | |
| 178 | S | F | Cl | CH₃ | i-C₃H₇ | |
| 179 | S | F | Cl | CH3 | C-C₃H₅ | |
| 180 | S | F | Cl | CH₃ | n-C₄H₉ | |
| 181 | S | F | Cl | CH₃ | i-C₄H₉ | |
| 182 | S | F | Cl | CH₃ | sek.- C₄H₉ | |
| 183 | S | F | Cl | CH₃ | tert.-C₄H₉ | |
| 184 | S | F | Cl | C₂H₅ | C₂H₅ | |
| 185 | S | F | Cl | C₂H₅ | n-C₃H₇ | |
| 186 | S | F | Cl | C₂H₅ | i-C₃H₇ | |
| 187 | S | F | Cl | C₂H₅ | c-C₃H₅ | |
| 188 | S | F | Cl | C₂H₅ | n-C₄H₉ | |
| 189 | S | F | Cl | C₂H₅ | i-C₄H₉ | |
| 190 | S | F | Cl | C₂H₅ | sek.- C₄H₉ | |
| 191 | S | F | Cl | CH₂=CH-CH₂ | CH₃ | |
| 192 | S | F | Cl | CH₂=CH-CH₂ | C₂H₅ | |
| 193 | S | F | Cl | CH₂=CH-CH₂ | n-C₃H₇ | 83 - 85 |
| 194 | S | F | Cl | CH₂=CH-CH₂ | i-C₃H₇ | |
| 195 | S | F | Cl | CH₂=CH-CH₂ | n-C₄H₉ | |
| 196 | S | F | Cl | CH₂=CH-CH₂ | sek.- C₄H₉ | |
| 197 | S | F | Cl | HC≡C-CH₂ | CH₃ | |
| 198 | S | F | Cl | HC≡C-CH₂ | C₂H₅ | |
| 199 | S | F | Cl | HC≡C-CH₂ | n-C₃H₇ | |
| 200 | S | F | Cl | HC≡C-CH₂ | i-C₃H₇ | 155 - 156 |
| 201 | S | F | Cl | HC≡C-CH₂ | n-C₄H₉ | |
| 202 | S | F | Cl | CH₂-CH₂-CH(CH₃)-CH₂-CH₂ | | 152 - 153 |
| 203 | S | F | Cl | CH₃ | Cyclohexyl | |
| 204 | S | F | Cl | CH₃ | C₆H₅ | |
| 205 | S | F | Cl | C₂H₅ | Cyclohexyl | |
| 206 | S | F | Cl | C₂H₅ | C₆H₅ | |
| 207 | S | F | Cl | [CH₂]₄ | | |
| 208 | S | F | Cl | [CH₂]₅ | | |
| 209 | S | F | CN | CH₃ | CH₃ | |
| 210 | S | F | CN | CH₃ | C₂H₅ | |
| 211 | S | F | CN | CH₃ | i-C₃H₇ | |
| 212 | S | F | CN | CH₃ | n-C₃H₇ | |
| 213 | S | F | CN | CH₃ | i-C₄H₉ | |
| 214 | S | F | CN | CH₃ | sek.-C₄H₉ | |
| 215 | S | F | CN | CH₃ | Cyclohexyl | |
| 216 | S | F | CN | CH₃ | C₆H₅ | |

### Beispiel 217: 8-(5'-N-Isopropyl-N-methylsulfamoyl-carboxamido-4'-chlor-2'-fluorphenyl)-4-oxo-7,9-dioxo-1,2,8-triaza[4.3.0]nonan (Beispiel 146 der WO 01/83459)

### 217.1: Tetrahydro-N-(4-chlor-2-fluor-5-N-isopropyl-N-methyl-sulfamoyl-carboxamido-phenyl)-4H-1,3,4-oxadiazin-3-carboxamid-4-carbonsäuremethylester

Zu einer Mischung von 3,5 g (10,1 mmol) N-(2-Chlor-4-fluor-5-isocyanato-benzoyl)-N'-isopropyl-N'-methyl-sulfamid IA-a.86 aus Beispiel 94 in 100 ml 1,2-Dichlorethan gab man innerhalb 5 Minuten bei 22 °C unter Rühren 9,8 g (10,1 mmol) Tetrahydro-4H-1,3,4-oxadiazin-4-carbonsäuremethylester als 15%ige Lösung in 1,2-Dichlorethan und rührte 18 Stunden nach. Anschließend reinigte man das Reaktionsgemisch durch Flash-Chromatographie an Kieselgel, wobei man mit 200 ml Portionen eines Gemisch aus Methylenchlorid/Diethylether = 1:6 eluierte. Nach dem Entfernen des Lösungsmittels im Vakuum erhielt man 4,3 g (82,3% der Theorie) Tetrahydro-N-(4-chlor-2-fluor-5-N-isopropyl-N-methyl-sulfamoylcarboxamido-phenyl)-4H-1,3,4-oxadiazin-3-carboxamid-4-carbonsäuremethylester mit Schmelzpunkt 69 °C (Zersetzung).

### 217.2: 8-(5'-N-Isopropyl-N-methylsulfamoyl-carboxamido-4'-chlor--2'-fluorphenyl)-4-oxo-7,9-dioxo-1,2,8-triaza[4.3.0]nonan

In einem Reaktionsgefäß mit Rührer und Wasserabscheider legte man 0,85 g (1,7 mmol) der Verbindung aus Beispiel 217.1 in 80 ml Toluol vor. Hierzu gab man unter Rühren bei 22 °C 0,18 g (1,8 mmol) 97%iges Natrium-tert.-butylat und erhitzte dann unter Rühren zum Rückfluss. Von Zeit zu Zeit erneuerte man das Toluol. Insgesamt erhitzte man 7 Stunden zum Rückfluss, bis die Reaktionsmischung dünnflüssiger wurde und Feststoffe fast ganz gelöst waren. Nach dem Abkühlen säuerte man das Reaktionsgemisch mit einer 1M HCl-Lösung in 10 ml Diethylether an und engte im Vakuum ein. Man löste den Rückstand in Methylenchlorid, extrahierte mit 1N Salzsäure und Wasser, trocknete und engte im Vakuum ein. Man erhielt 0,67 g (76% der Theorie) der Titelverbindung mit einem Schmelzpunkt von 112-118 °C. Nach dem Verrühren mit Diethylether betrug der Schmelzpunkt 115-120 °C.

## Patentansprüche

1. verfahren zur Herstellung von phenyliso(thio)cyanaten der allgemeinen Formel I, 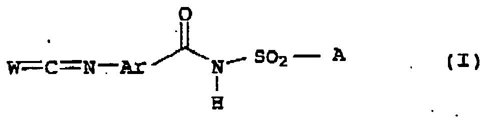 worin die Variablen folgende Bedeutung haben:
W Sauerstoff oder Schwefel,
Ar Phenyl, das durch folgende Gruppen ein- oder mehrfach substituiert sein kann: Wasserstoff, Halogen, C₁-C₄-Halogenalkyl oder Cyano,
A ein von einem primären oder sekundären Amin abgeleiteter Rest oder NH₂,
**dadurch gekennzeichnet, dass** man eine Verbindung der allgemeinen Formel **II,** 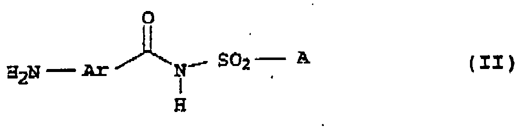 worin die Variablen Ar und A die zuvor genannten Bedeutungen aufweisen, oder deren BCl-Addukt mit Phosgen, Thiophosgen oder Diphosgen umsetzt.

2. Verfahren nach Anspruch 1, **dadurch gekennzeichnet, dass** man das HCl-Addukt der Verbindung II einsetzt.

3. Verfahren nach Anspruch 1 oder 2, **dadurch gekennzeichnet, dass** man 0,9 bis 2 Moläquivalente Phosgen, Thiophosgen oder Diphosgen bezogen auf die Verbindung **II** einsetzt.

4. Verfahren nach einem der vorherigen Ansprüche, **dadurch gekennzeichnet, dass** man die Umsetzung des chlorwasserstoffadduktes der Verbindung II in Gegenwart von Aktivkohle durchführt.

5. Verfahren nach einem der vorherigen Ansprüche, **dadurch gekennzeichnet, dass** man eine Verbindung der Formel IIA, 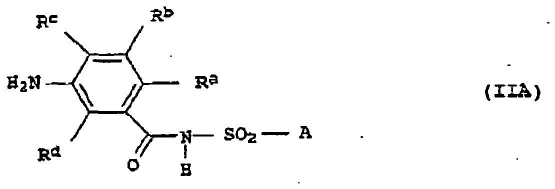 worin
R^{a}, R^{b}, R^{c} und R^{d} unabhängig voneinander für Wasserstoff, Ealogen, C₁-C₄-Halogenalkyl oder cyano stehen und
A die zuvor genannte Bedeutung aufweist,
oder deren HCl-Addukt mit Phosgen, Thiophosgen oder Diphosgen umsetzt, wobei man eine verbindung der Formel IA, 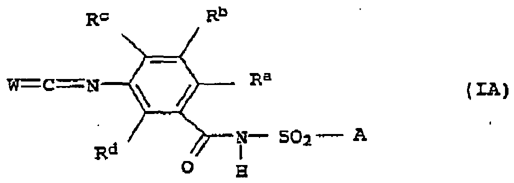 worin die Variablen R^{a}, R^{b}, R^{c}, R^{d}, A und W die zuvorgenannten Bedeutungen aufweisen, erhält.

6. verfahren nach einem der vorhergehenden Ansprüche, **dadurch gekennzeichnet, dass** der Rest A in Formel I für -NR¹R² steht, worin die Variablen R¹ und R² die folgenden Bedeutungen aufweisen:
R¹ und R² stehen unabhängig voneinander für Wasserstoff, C₁-C₁₀-Akkyl, C₂-C₁₀-Alkenyl oder C₂-C₁₀-Alkinyl, die unsubstituiert oder durch einen der folgenden Reste substituiert sein können: C₁-C₄-Alkoxy, C₁-C₄-Alkylthio, CN, NO₂, Formyl, C₁-C₄-Alkylcarbonyl, C₁-C₄-Alkoxycarbonyl, C₁-C₄-Alkylaminocarbonyl, C₁-C₄-Dialkylaminocarbornyl, C₁-C₄-Alkylsulfinyl, C₁-C₄-Alkylsulfonyl, C₃-C₁₀-Cycloalkyl, 3- bis 8-gliedriges Heterocyclyl mit ein, zwei oder drei unter O, S, N und einer Gruppe NR⁶ (worin R⁶ für wasserstoff, C₁-C₆-Alkyl, C₃-C₆-Alkenyl oder C₃-C₆-Alkinyl steht) ausgewählten Heteroatomen, Phenyl, das seinerseits 1, 2, 3 oder 4 Substituenten, ausgewählt unter Halogen, C₁-C₄-Alkyl, C₁-C₄-Alkoxy, C₁-C₄-Fluoralkyl, C₁-C₄-Alkyloxycarbonyl, Trifluormethylsulfonyl, C₁-C₃-Alkylamino, C₁-C₃-Dialkylamino, Formyl, Nitro oder cyano, aufweisen kann, c₁-c₁₀ₗ-Halogenalkyl, C₂-C₁₀-Halogenalkenyl, C₂-C₁₀-Halogen-alkinol, C₃-C₈-Cycloalkyl, C₃-C₁₀-cycloalkenyl, 3- bis 8-gliedriges Heterocyclyl mit ein bis drei Heteroatomen, ausgewählt unter O, S, N und einer Gruppe NR⁶ (worin R⁶ für Wasserstoff, C₁-C₆-Alkyl, C₃-C₆-Alkenyl oder C₃-C₆-Alkinyl steht), phenyl oder Naphthyl, wobei C₃-C₈-Cycloalkyl, C₃-C₁₀-Cycloalkenyl, 3- bis 8-gliedriges Heterocyclyl, Phenyl oder Naphthyl, ihrerseits 1, 2, 3 oder 4 Substituenten, ausgewählt unter Halogen, C₁-C₄-Alkyl, C₁-C₄-Alkoxy, C₁-C₄-Fluoralkyl, C₁-C₄-Alkyloxycarbonyl, Trifluormethylsulfonyl, Formyl, C₁-C₃-Alkylamino, C₁-C₃-Dialkylamino, Phenoxy, Nitro oder Cyano, aufweisen können, oder
R¹ und R² bilden gemeinsam einen gesättigten oder teilweise ungesättigten 5- bis 8-gliedrigen stickstoffheterocyclus, der seinerseits durch C₁-C₄-Alkyl, C₁-C₄-Alkoxy und/oder C₁-C₄-Halogenalkyl, substituiert sein kann, ein oder zwei carbonylgruppen, Thiocarbonylgruppen und/oder ein oder zwei weitere Heteroatome, ausgewählt unter O, S, N und einer Gruppe NR⁶ (worin R⁶ die zuvor genannten Bedeutungen aufweist), als Ringglieder aufweisen kann.

7. Verfahren nach Anspruch 1, dadurch gekenntzeichnet, dass das Verfahren zusätzlich die folgenden Schritte umfasst:
i) Umsetzung einer Aroylverbindung der allgemeinen Formel III, 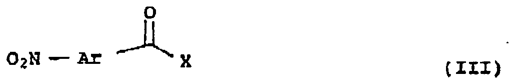 worin die variable Ar die zuvorgenannten Bedeutungen aufweist und X für Halogen, OH oder C₁-C₄-Alkoxy steht, mit einem Sulfamidsäureamid der Formel IV
H₂N-SO₂-A (IV),
worin A die zuvor genannten Bedeutungen aufweist und
ii) Reduktion des in Schritt i) erhaltenen N-Aroylsulfamidsäureamid der allgemeinen Formel V, 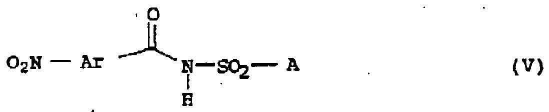 worin Ar und A die zuvor genannten Bedeutungen aufweisen, wobei man eine Verbindung der Formel II erhalt.

8. verfahren nach Anspruch 7, **dadurch gekennzeichnet dass** in Schritt (ii) die Reduktion mit Wasserstoff in Gegenwart katalytischer Mengen an Übergangsmetallen oder Übergangsmetallverbindungen erfolgt.

9. verfahren nach Anspruch 7, **dadurch gekennzeichnet, dass** in Schritt (ii) die Reduktion in Gegenwart von Eisen und wenigstens einer C₁-C₄-Carbonsäure erfolgt.

10. Verfahren nach Anspruch 7, **dadurch gekennzeichnet, dass** in Schritte (ii) die Reduktion in Gegenwart von Raney-Nickel und Wasserstoff erfolgt.

11. Phenyliso(thio)cyanate der allgemeinen Formel I wie in Anspruch 1 definiert.

12. Phenyliso(thio)cyanate der allgemeinen Formel IA wie in Anspruch 5 definiert, **dadurch gekennzeichnet, dass** R^{a} für Fluor, Chlor oder Cyano steht, R^{c} für Wasserstoff, Fluor oder Chlor steht und R^{b} und R^{d} jeweils für Wasserstoff stehen.

13. Phenyliso(thio)cyanate der allgemeinen Formel IA wie in Anspruch 5 definiert, **dadurch gekennzeichnet, dass** A für einen Rest der Formel NR¹R² steht, worin R¹ und R² die in Anspruch 6 angegebenen Bedeutungen aufweisen.

14. Phenyliso(thio)cyanate der allgemeinen Formel IA nach Anspruch 12, **dadurch gekennzeichnet, dass** R¹ und R² unabhängig voneinander für Wasserstoff, C₁-C₆-Alkyl, das gegebenenfalls durch einen Substituenten ausgewählt unter Halogen, Cyano, C₁-C₄-Alkoxy, C₁-C₄-Alkoxycarbonyl, C₁-C₄-Alkylthio, C₃-C₈-Cycloalkyl, Furyl, Thienyl, 1,3-Dioxolenyl, Phenyl, das seinerseits gegebenenfalls durch Halogen oder C₁-C₄-Alkoxy substituiert ist, substituiert ist,
C₂-C₆-Alkonyl C₂-C₆-Alkinyl, C₃-C₈-Cycloalkyl oder Phenyl, das gegebenenfalls durch 1 oder 2 Substituenten, ausgewählt unter Halogen, C₁-C₆-Alkyl, C₁-C₄-Fluoralkyl, C₁-C₄-Alkoxy, C₁-C₄-Alkoxycarbonyl, Nitro oder C₁-C₃-Dialkylamino substituiert ist, Naphthtyl oder Pyridyl stehen oder
R¹ und R² zusammen einen fünf-, sechs- oder siebengliedrigen gesättigen oder ungesättigten Sticketoffheterocyclus, der gegebenenfalls ein weiteres Heteroatom, ausgewählt unter N, einer Gruppe NR⁶ (worin R⁶ die zuvor genannten Bedeutungen aufweist) und O, als Ringglied enthalten kann, bilden und/oder durch ein, zwei oder drei Substituenten, ausgewählt unter C₁-C₄-Alkyl und C₁-C₄-Halogenalkyl, substituiert sein kann.

15. Verfahren zur Herstellung von Verbindungen der Formel VI, 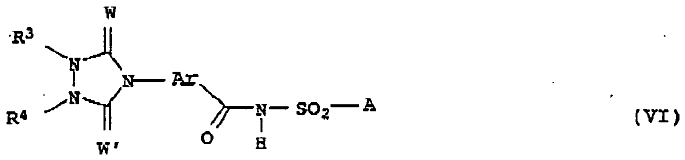 worin W, Ar und A die in Anspruch 1 genannten Bedeutungen aufweisen, W' für O oder S steht und R³ und R⁴ unabhängig voneinander für Wasserstoff, Cyano, Amino, C₁-C₆-Alkyl, C₁-C₆-Halogenalkyl, C₁-C₆-Halogenalkoxy, C₃-C₇-Cycloalkyl, C₂-C₅-Alkenyl, C₂-C₆-Halogenalkenyl, C₃-C₆-Alkinyl, Benzyl, OR⁵ (worin R⁵ für Wasserstoff, C₁-C₆-Alkyl, C₁-C₆-Halogenalkyl, C₃-C₇-Cycloalkyl, C₂-C₅-Alkenyl, C₃-C₆-Alkinyl, gegebenenfalls substituiertes Phenyl oder gegegebenenfalls substituiertes Benzyl steht), C₁-C₃-Cyanoalkyl, stehen, oder R³ und R⁴ zusammen mit den stickstoffatomen, an die sie gebunden sind, einen vierbis siebengliedrigen, gegebenenfalls durch Schwefel, Sauerstoff, eine Gruppe NR⁶ (worin R⁶ die zuvor genannten Bedeutungen aufweist) oder Stickstoff unterbrochenen Heterocyclus, der gegebenenfalls ein- oder mehrfach durch Halogen oder C₁-C₄-Alkyl substituiert ist, bilden,
**dadurch gekennzeichnet, dass** man
(i) eine Verbindung der Formel I wie in Anspruch 1 definiert, mit einem Oxadiazincarbonsäureester der Formel VII, 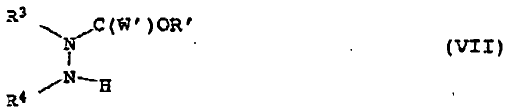 worin W' die zuvor genannte Bedeutung aufweist und R' für C₁-C₄-Alkyl steht, umsetzt, wobei man ein Harnstoffderivat der Formel VIII erhält, 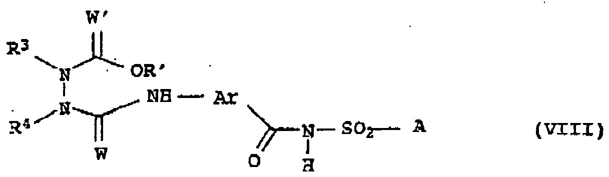 worin die Variablen R³, R⁴, R', W, W', Ar und A die zuvor genannten Bedeutungen aufweisen, erhält und
(ii) das erhaltene Zwischenprodukt VIII cyclisiert, wobei man eine Verbindung der Formel VI erhält.

16. Verfahren nach Anspruch 15, **dadurch gekennzeichnet, dass** die in Schritt (i) eingesetzte Verbindung der Formel I für eine Verbindung der Formel IA 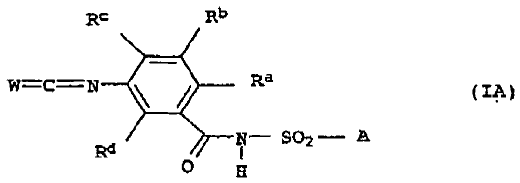 worin die Variablen Ra, R^{b}, R^{c}, R^{d}, A und w die zuvorgenannte Bedeutungen aufweisen, steht.

17. Verfahren nach Anspruch 15, **dadurch gekennzeichnet, dass** die in Schritt (i) eingesetzte Verbindung VII für eine Verbindung der Formel VII' steht, 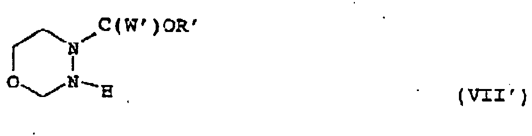 worin W' für O oder S und R' für C1-C4-Alkyl stehen.

## Claims

1. A process for preparing phenyl iso(thio)cyanates of the formula I 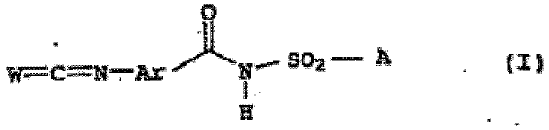 where the variables are as defined below:
W is oxygen or sulfur,
Ar is phenyl which may be mono- or polysubstituted by the following groups: hydrogen, halogen, C₁-C₄-haloalkyl or cyano,
A is a radical derived from a primary or secondary amine or is NH₂,
which comprises reacting a compound of the formula II 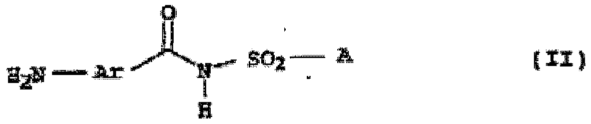 where the variables Ar and A are as defined above or its HCl adduct with phosgene, thiophosgene or diphosgene.

2. A process as claimed in claim 1, wherein the HCl adduct of the compound II is used.

3. A process as claimed in claim 1 or 2, wherein from 0.9 to 2 molar equivalents of phosgene, thiophosgene or diphosgene are used, based on the compound II.

4. A process as claimed in any of the preceding claims, wherein the reaction of the hydrogen chloride adduct of the compound II is carried out in the presence of activated carbon.

5. A process as claimed in any of the preceding claims, wherein a compound of the formula IIA 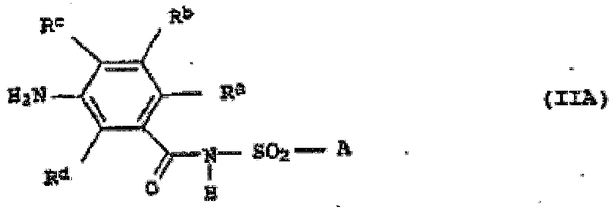 where
R^{a}, R^{b}, R^{c} and R^{d} independently of one another are hydrogen, halogen, C₁-C₄-haloalkyl or cyano and
A is as defined above
or its HCl adduct is reacted with phosgene, thiophosgene or diphosgene, giving a compound of the formula IA 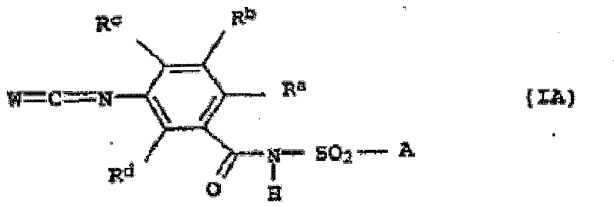 where the variables R^{a}, R^{b}, R^{c}, R^{d}, A and W are as defined above.

6. A process as claimed in any of the preceding claims, wherein the radical A in formula I is -N¹R², where the variables R¹ and R² are as defined below:
R¹ and R² independently of one another represent hydrogen, C₁-C₁₀-alkyl, C₂-C₁₀-alkenyl or C₂-C₁₀-alkynyl which may be unsubstituted or substituted by one of the following radicals: C₁-C₄-alkoxy, C₁-C₄-alkylthio, CN, NO₂, formyl, C₁-C₄-alkylcarbonyl, C₁-C₄-alkoxycarbonyl, C₁-C₄-alkylaminocarbonyl, C₁-C₄-dialkylaminocarbonyl, C₁-C₄-alkylsulfinyl, C₁-C₄-alkylsulfonyl, C₃-C₁₀-cycloalkyl, 3- to 8-membered heterocyclyl having one, two or three heteroatoms selected from the group consisting of O, S, N and a group NR⁶ (where R⁶ is hydrogen, C₁-C₆-alkyl, C₃-C₆-alkenyl or C₃-C₆-alkynyl), phenyl, which for its part may have 1, 2, 3 or 4 substituents selected from the group consisting of halogen, C₁-C₄-alkyl, C₁-C₄-alkoxy, C₁-C₄-fluoroalkyl, C₁-C₄-alkyloxycarbonyl, trifluoromethylsulfonyl, C₁-C₃-alkylamino, C₁-C₃-dialkylamino, formyl, nitro and cyano, C₁-C₁₀-haloalkyl, C₂-C₁₀-haloalkenyl, C₂-C₁₀-haloalkynyl, C₃-C₈-cycloalkyl, C₃-C₁₀-cycloalkenyl, 3- to 8-membered heterocyclyl having one to three heteroatoms selected from the group consisting of O, S, N and a group NR⁶ (where R⁶ is hydrogen, C₁-C₆-alkyl, C₃-C₆-alkenyl or C₃-C₆-alkynyl), phenyl or naphthyl, where C₃-C₈-cycloalkyl, C₃-C₁₀-cycloalkenyl, 3- to 8-membered heterocyclyl, phenyl and naphthyl may for their part have 1, 2, 3 or 4 substituents selected from the group consisting of halogen, C₁-C₄-alkyl, C₁-C₄-alkoxy, C₁-C₄-fluorcalkyl, C₁-C₄-alkyloxycarbonyl, trifluoromethylsulfonyl, formyl, C₁-C₃-alkylamino, C₁-C₃-dialkylamino, phenoxy, nitro and cyano, or
R¹ and R² together form a saturated or partially unsaturated 5- to 8-membered nitrogen heterocycle which for its part may be substituted by C₁-C₄-alkyl, C₁-C₄-alkoxy and/or C₁-C₄-haloalkyl and may have one or two carbonyl groups, thiocarbonyl groups and/or one or two further heteroatoms selected from the group consisting of O, S, N and a group NR⁶ (where R⁶ is as defined above) as ring members.

7. A process as claimed in claim 1, wherein the process additionally comprises the following steps:
i) reaction of an aroyl compound of the formula III 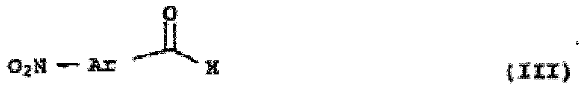 in which the variable Ar is as defined above and X is halogen, OH or C₁-C₄-alkoxy with a sulfamic acid amide of the formula IV
H₂N-SO₂-A (IV),
where A is as defined above and
ii) reduction of the N-aroylsulfamic acid amide, obtained in step i), of the formula V 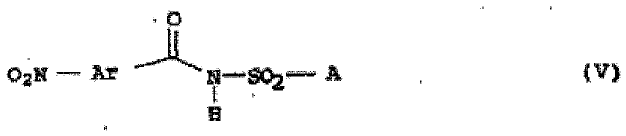 where Ar and A are as defined above, giving a compound of the formula II.

8. A process as claimed in claim 7, wherein in step (ii) the reduction is carried out with hydrogen in the presence of catalytic amounts of transition metals or transition metal compounds.

9. A process as claimed in claim 7, wherein in step (ii) the reduction is carried out in the presence of iron and at least one C₁-C₄-carboxylic acid.

10. A process as claimed in claim 7, wherein in step (ii) the reduction is carried out in the presence of Raney nickel and hydrogen.

11. A phenyl iso(thio)cyanate of the formula I as defined in claim 1.

12. A phenyl iso(thio)cyanate of the formula IA as defined in claim 5, wherein R^{a} is fluorine, chlorine or cyano, R^{c} is hydrogen, fluorine or chlorine and R^{b} and R^{d} are each hydrogen.

13. A phenyl iso(thio)cyanate of the formula IA as defined in claim 5, wherein A is a radical of the formula NR¹R² where R¹ and R² are as defined in claim 6.

14. A phenyl iso(thio)cyanate of the formula IA as claimed in claim 12, wherein R¹ and R² independently of one another are hydrogen, C₁-C₆-alkyl which is optionally substituted by a substituent selected from the group consisting of halogen, cyano, C₁-C₄-alkoxy, C₁-C₄-alkoxycarbonyl, C₁-C₄-alkylthio, C₃-C₈-cycloalkyl, furyl, thienyl, 1,3-dioxolanyl, phenyl which for its part is optionally substituted by halogen or C₁-C₄-alkoxy,
C₂-C₆-alkenyl, C₂-C₆-alkynyl, C₃-C₈-cycloalkyl or phenyl which is optionally substituted by 1 or 2 substituents selected from the group consisting of halogen, C₁-C₄-alkyl, C₁-C₄-fluoroalkyl, C₁-C₄-alkoxy, C₁-C₄-alkoxycarbonyl, nitro and C₁-C₃-dialkylamino, naphthyl or pyridyl or
R¹ and R² together form a five-, six- or seven-membered saturated or unsaturated nitrogen heterocycle which may optionally comprise a further heteroatom selected from the group consisting of N, a group NR⁶ (where R⁶ is as defined above) and 0 as ring member and/or which may be substituted by one, two or three substituents selected from the group consisting of C₁-C₄-alkyl and C₁-C₄-haloalkyl.

15. A process for preparing compounds of the formula VI 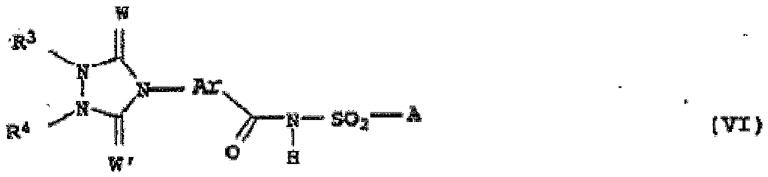 where W, Ar and A are as defined in claim 1, W' is O or S and R³ and R⁴ independently of one another are hydrogen, cyano, amino, C₁-C₆-alkyl, C₁-C₆-haloalkyl, C₁-C₆-haloalkoxy, C₃-C₇-cycloalkyl, C₂-C₆-alkenyl, C₂-C₆-haloalkenyl, C₃-C₆-alkynyl, benzyl, OR⁵ (where R⁵ is hydrogen, C₁-C₆-alkyl, C₁-C₆-haloalkyl, C₃-C₇-cycloalkyl, C₂-C₆-alkenyl, C₃-C₆-alkynyl, unsubstituted or substituted phenyl or unsubstituted or substituted benzyl), C₁-C₃-cyanoalkyl, or R³ and R⁴ together with the nitrogen atoms to which they are attached form a four- to seven-membered heterocycle which is optionally interrupted by sulfur, oxygen, a group NR⁶ (where R⁶ is as defined above) or nitrogen and which is unsubstituted or mono- or polysubstituted by halogen or C₁-C₄-alkyl,
which comprises
(i) reacting a compound of the formula I as defined in claim 1 with an oxadiazinecarboxylic acid ester of the formula VII 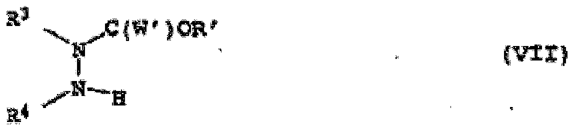 where W' is as defined above and R' is C₁-C₄-alkyl, giving a urea derivative of the formula VIII 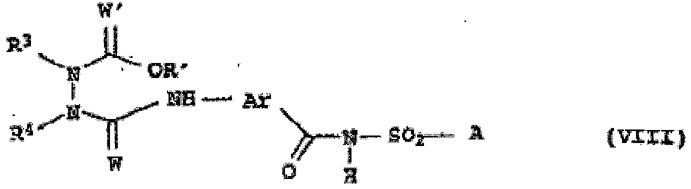 where the variables R³, R⁴, R', W, W', Ar and A are as defined above and
(ii) cyclizing the resulting intermediate VIII, giving a compound of the formula VI.

16. A process as claimed in claim 15, wherein the compound of the formula I used in step (i) is a compound of the formula IA 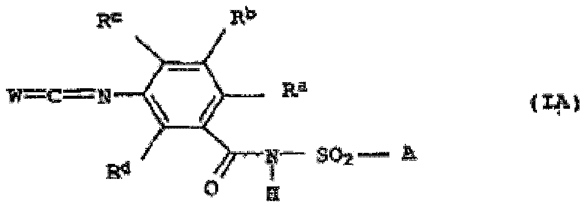 where the variables R^{a}, R^{b}, R^{c}, R^{d}, A and W are as defined above.

17. A process as claimed in claim 15, wherein the compound VII used in step (i) is a compound of the formula VII' 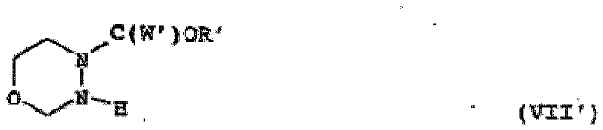 where W' is 0 or S and R' is C₁-C₄-alkyl.

## Revendications

1. Procédé de fabrication d''iso(thio)cyanates de phényle de formule générale I 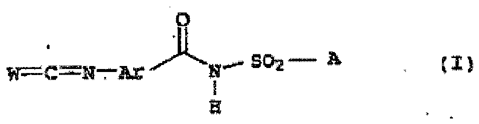 dans laquelle les variables ont la signification suivante :
W oxygène ou soufre,
Ar phényle, qui peut être substitué une ou plusieurs fois par les groupes suivants : hydrogène, halogène, halogénoalkyle en C₁-C₄ ou cyano,
A un radical dérivé d'une amine primaire ou secondaire ou NH₂,
**caractérisé en ce qu'**un composé de formule générale II 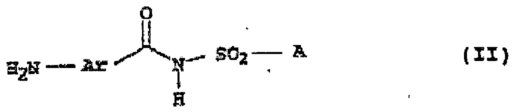 dans laquelle les variables Ar et A ont les significations mentionnées précédemment, ou son adduit HCl, est mis en réaction avec du phosgène, du thiophosgène ou du diphosgène.

2. Procédé selon la revendication 1, **caractérisé en ce que** l'adduit HCl du composé II est utilisé.

3. Procédé selon la revendication 1 ou 2, **caractérisé en ce que** 0,9 à 2 équivalents molaires de phosgène, thiophosgène ou diphosgène sont utilisés par rapport au composé II.

4. Procédé selon l'une quelconque des revendications précédentes, **caractérisé en ce que** la réaction de l'adduit chlorure d'hydrogène du composé II est réalisée en présence de charbon actif.

5. Procédé selon l'une quelconque des revendications précédentes, **caractérisé en ce qu'**un composé de formule IIA 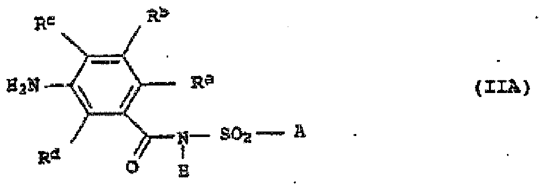 dans laquelle
R^{a}, R^{b}, R^{c} et R^{d} représentent indépendamment les uns des autres hydrogène, halogène, halogénoalkyle en C₁-C₄ ou cyano, et
A a la signification mentionnée précédemment,
ou son adduit HCl est mis en réaction avec du phosgène, du thiophosgène ou du diphosgène, un composé de formule IA 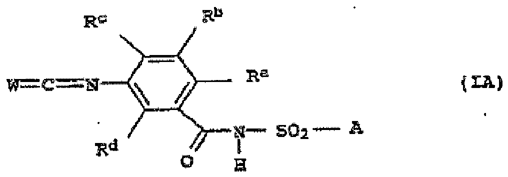 dans laquelle les variables R^{a}, R^{b}, R^{c}, R^{d}, A et W ont les significations susmentionnées, étant obtenu.

6. Procédé selon l'une quelconque des revendications précédentes, **caractérisé en ce que** le radical A dans la formule I représente -NR¹R², les variables R¹ et R² ayant les significations suivantes :
R¹ et R² représentent indépendamment l'un de l'autre hydrogène, alkyle en C₁-C₁₀, alcényle en C₂-C₁₀ ou alcynyle en C₂-C₁₀, qui peuvent être non substitués ou substitués par un des radicaux suivants : alcoxy en C₁-C₄, alkylthio en C₁-C₄, CN, NO₂, formyle, alkylcarbonyle en en C₁-C₉, alcoxycarbonyle en C₁-C₄, alkylaminocarbonyle en C₁-C₄, dialkylaminocarbonyle en C₁-C_{4;} alkylsulfinyle en C₁-C₄, alkylsulfonyle en C₁-C₄, cycloalkyle en C₃-C₁₀, hétérocyclyle de 3 à 8 éléments comprenant un, deux ou trois hétéroatomes choisis parmi O, S, N et un groupe NR⁶ (R⁶ représentant hydrogène, alkyle en C₁-C₆, alcényle en C₃-C₆ ou alcynyle en C₃-C₆), phényle, qui peut comprendre de son côté 1, 2, 3 ou 4 substituants choisis parmi halogène, alkyle en C₁-C₄, alcoxy en C₁-C₄, fluoroalkyle en C₁-C₄, alkyloxycarbonyle en C₃-C₄, trifluorométhylsulfonyle, alkylamino en C₁-C₃, dialkylamino en C₁-C₃, formyle, nitro ou cyano, halogénoalkyle en C₁-C₁₀, halogénoalcényle en C₂-C₁₀, halogénoalcynyle en C₂-C₁₀, cycloalkyle en C₃-C₈, cycloalcényle en C₃-C₁₀, hétérocyclyle de 3 à 8 éléments comprenant un à trois hétéroatomes choisis parmi 0, S, N et un groupe NR⁶ (R⁶ représentant hydrogène, alkyle en C₁-C₆, alcényle en C₃-C₆ ou alcynyle en C₃-C₆), phényle ou naphtyle, cycloalkyle en C₃-C₈, cycloalcényle en C₃-C₁₀, hétérocyclyle de 3 à 8 éléments, phényle ou naphtyle pouvant de leur côté comprendre 1, 2, 3 ou 4 substituants choisis parmi halogène, alkyle en C₁-C₄, alcoxy en C₁-C₄, fluoroalkyle en C₁-C₄, alkyloxycarbonyle en C₁-C₄, trifluorométhylsulfonyle, formyle, alkylamino en C₁-C₃, dialkylamino en C₁-C₃, phénoxy, nitro ou cyano, ou
R¹ et R² forment ensemble un hétérocycle azoté de 5 à 8 éléments saturé ou partiellement insaturé, qui peut de son côté être substitué par alkyle en C₁-C₄, alcoxy en C₁-C₄ et/ou halogénoalkyle en C₁-C₄, qui peut comprendre en tant qu'éléments de cycle un ou deux groupes carbonyle, groupes thiocarbonyle et/ou un ou deux hétéroatomes supplémentaires, choisis parmi O, S, N et un groupe NR⁶ (R⁶ ayant les significations mentionnées précédemment).

7. Procédé selon la revendication 1, **caractérisé en ce que** le procédé comprend également les étapes suivantes :
i) la mise en réaction d'un composé d'aroyle de formule générale III 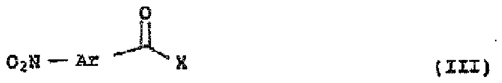 dans laquelle la variable Ar a les significations susmentionnées et X représente halogène, OH ou alcoxy en C₁-C₄, avec un amide d'acide sulfamidique de formule IV
H₂N-SO₂-A (IV)
dans laquelle A a les significations mentionnées précédemment, et
ii) la réduction de l'amide d'acide N-aroylsulfamidique de formule générale V obtenu à l'étape i) 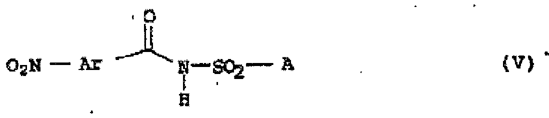 dans laquelle Ar et A ont les significations mentionnées précédemment, un composé de formule II étant obtenu.

8. Procédé selon la revendication 7, **caractérisé en ce qu'**à l'étape (ii), la réduction a lieu avec de l'hydrogène en présence de quantités catalytiques de métaux de transition ou de composés de métaux de transition.

9. Procédé selon la revendication 7, **caractérisé en ce qu'**à l'étape (ii), la réduction a lieu en présence de fer et d'au moins un acide carboxylique en C₁-C₄.

10. Procédé selon la revendication 7, **caractérisé en ce qu'**à l'étape (ii), la réduction a lieu en présence de nickel de Raney et d'hydrogène.

11. Iso(thio)cyanates de phényle de formule générale I tels que définis dans la revendication 1.

12. Iso(thio)cyanates de phényle de formule générale IA tels que définis dans la revendication 5, **caractérisés en ce que** R^{a} représente fluor, chlore ou cyano, R^{c} représente hydrogène, fluor ou chlore, et R^{b} et R^{d} représentent chacun hydrogène.

13. Iso (thio) cyanates de phényle de formule générale IA tels que définis dans la revendication 5, **caractérisés en ce que** A représente un radical de formule NR¹R², R¹ et R² ayant les significations données dans la revendication 6.

14. Iso(thio)cyanates de phényle de formule générale IA selon la revendication 12, **caractérisés en ce que** R¹ et R² représentent indépendamment l'un de l'autre hydrogène, alkyle en C₁-C₆, qui peut éventuellement être substitué par un substituant choisi parmi halogène, cyano, alcoxy en C₁-C₄, alcoxycarbonyle en C₁-C₄, alkylthio en C₁-C₄, cycloalkyle en C₃-C₈, furyle, thiényle, 1,3-dioxolanyle, phényle, qui est de son côté éventuellement substitué par halogène ou alcoxy en C₁-C₄,
alcényle en C₂-C₆, alcynyle en C₂-C₆, cycloalkyle en C₃-C₈ ou phényle, qui est éventuellement substitué par 1 ou 2 substituants choisis parmi halogène, alkyle en C₁-C₄, fluoroalkyle en C₁-C₄, alcoxy en C₁-C₄, alcoxycarbonyle en C₁-C₄, nitro ou dialkylamino en C₁-C₃, naphtyle ou pyridyle, ou
R¹ et R² forment ensemble un hétérocycle azoté saturé ou insaturé de cinq, six ou sept éléments, qui peut éventuellement contenir en tant qu'élément de cycle un hétéroatome supplémentaire choisi parmi N, un groupe NR⁶ (R⁶ ayant les significations susmentionnées) et O, et/ou qui peut être substitué par un, deux ou trois substituants choisis parmi alkyle en C₁-C₄ et halogénoalkyle en C₁-C₄.

15. Procédé de fabrication de composés de formule VI 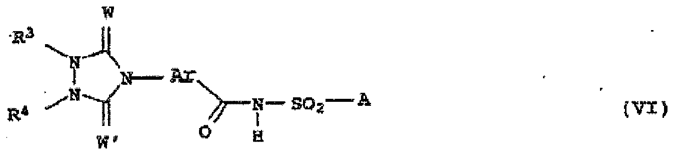 dans laquelle W, Ar et A ont les significations mentionnées dans la revendication 1, W' représente 0 ou S, et R³ et R⁴ représentent indépendamment l'un de l'autre hydrogène, cyano, amino, alkyle en C₁-C₆, halogénoalkyle en C₁-C₆, halogénoalcoxy en C₁-C₆, cycloalkyle en C₃-C₇, alcényle en C₂-C₆, halogénoalcényle en C₂-C₆, alcynyle en C₃-C₆, benzyle, OR⁵ (R⁵ représentant hydrogène, alkyle en C₁-C₆, halogénoalkyle en C₁-C₆, cycloalkyle en C₃-C₇, alcényle en C₂-C₆, alcynyle en C₃-C₆, phényle éventuellement substitué ou benzyle éventuellement substitué), cyanoalkyle en C₁-C₃, ou R³ et R⁴ forment ensemble avec les atomes d'azote auxquels ils sont reliés un hétérocycle de quatre à sept éléments, éventuellement interrompu par soufre, oxygène, un groupe NR⁶ (R⁶ ayant les significations mentionnées précédemment) ou azote, qui est éventuellement substitué une ou plusieurs fois par halogène ou alkyle en C₁-C₄,
**caractérisé en ce que**
(i) un composé de formule I tel que défini dans la revendication 1 est mis en réaction avec un ester
d'acide oxadiazine-carboxylique de formule VII 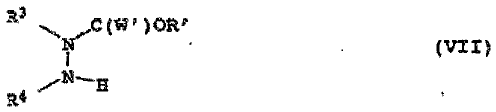 dans laquelle W' a la signification mentionnée précédemment et R' représente alkyle en C₁-C₄, un dérivé d'urée de formule VIII étant obtenu 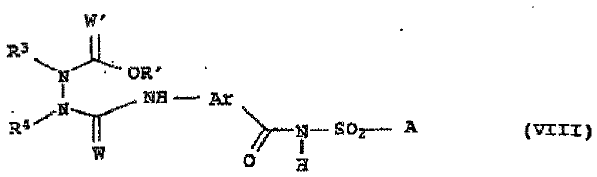 dans laquelle les variables R³, R⁴, R', W, W', Ar et A ont les significations mentionnées précédemment, et
(ii) le produit intermédiaire VIII obtenu est cyclisé, un composé de formule VI étant obtenu.

16. Procédé selon la revendication 15, **caractérisé en ce que** le composé de formule I utilisé à l'étape (i) représente un composé de formule IA 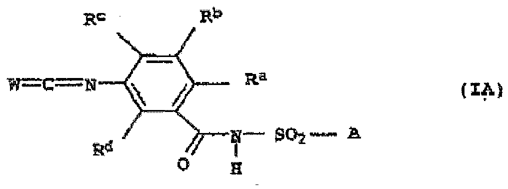 dans laquelle les variables R^{a}, R^{b}, R^{c}, R^{a}, A et W ont les significations susmentionnées.

17. Procédé selon la revendication 15, **caractérisé en ce que** le composé VII utilisé à l'étape (i) représente un composé de formule VII' 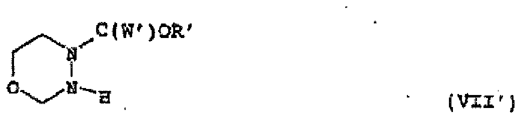 dans laquelle W' représente 0 ou S et R' représente alkyle en C₁-C₄.
